# EUROPEAN PATENT SPECIFICATION

(11) **EP 4 121 429 B1**
(45) Date of publication and mention of the grant of the patent: **13.08.2025**
(21) Application number: 21711275.4
(22) Date of filing: 16.03.2021
(51) Int. Cl.: C07D 471/04, A61P 35/00, A61K 31/427

(54) **CONDENSED PYRIDINE DERIVATIVES SUBSTITUED BY AMIDE FUNCTIONS AS ACSS2 INHIBITORS**
DURCH AMIDFUNKTIONEN SUBSTITUIERTE KONDENSIERTE PYRIDINDERIVATE ALS ACSS2-INHIBITOREN
DÉRIVÉS DE PYRIDINE CONDENSÉS SUBSTITUÉS PAR DES FONCTIONS AMIDE UTILISÉS EN TANT QU'INHIBITEURS D'ACSS2

(30) Priority: 19.03.2020 EP 20164207
(43) Date of publication of application: 25.01.2023
(73) Proprietor: Merck Patent GmbH, 64293 Darmstadt (DE); Ryvu Therapeutics S.A., 30-394 Krakow (PL)
(72) Inventor: KOETZNER, Lisa, 64293 Darmstadt (DE); FUCHSS, Thomas, 64293 Darmstadt (DE); SCHINDLER, Christina, 64293 Darmstadt (DE); KUHN, Daniel, 64293 Darmstadt (DE)
(74) Representative: Merck Serono S.A. Intellectual Property
(86) International application number: PCT/EP2021/056585
(87) International publication number: WO 2021/185789

(56) References cited:
- WO-A1-2015/175845
- WO-A1-2020/252407

## Description

### Field of the invention

The present invention relates to substituted amide derivatives. These compounds are useful for inhibiting acetyl-CoA synthetase 2 (ACSS2) and for the prevention and/or treatment of several medical conditions including hyperproliferative disorders and diseases that are affected by ACSS2 activity.

### Background of the invention

It is well established that the rapid and uncontrolled growth of tumors and proliferation of cancer cells require increased energy (ATP) and biomass (lipids) production when compared to normal, healthy cells.

In recent years the role of acetate metabolism for cancer cell proliferation has become of growing interest in cancer research and the development of cancer therapy. It has been shown that some tumors primarily utilize acetate for energy production, while others mainly use it for lipid (i.e. biomass) synthesis or regulation of histone acetylation and thus gene transcription (Z.T. Schug, et al., Nature Reviews Cancer 16, 707-717 (2016)). In all these processes acetate is converted into acetyl-CoA by means of acetyl-CoA synthetase, ACSS, etiher by the mitochondria-localized ACSS1 or the nucleocytosol-localized ACSS2. Thus, acetyl-CoA is an important metabolite of cancer cells not only with regard to energy production in the mitochondrion but also with regard to lipid and fatty acid synthesis in the cytosol of cancer cells as well as the histone acetylation in the nucleus of the cell.

Studies have shown that in particular ACSS2 is highly expressed in many cancer tissues. These findings and the fact that it is upregulated by hypoxia and low nutrient availability make ACSS2 an attractive target for cancer treatment (Z.T. Schug, et al., Cancer Cell (2015) 27, 57-71; Z.T. Schug, et al., Nature Reviews Cancer 16, 707-717 (2016)).

### Prior Art

WO 2015/175845 A1 discloses certain benzimidazole derivatives as inhibitors of ACSS2.

WO 2020/252407 A1 discloses certain benzimidazole derivatives as inhibitors of ACSS2.

### Description of the invention

It is an object of the present invention to provide compounds that are useful for the prevention and/or treatment of medical conditions, disorders and/or diseases, in particular of hyperproliferative disorders/diseases, which compounds are inhibitors of ACSS2.

The object has surprisingly been solved by the compounds of the present invention. This invention provides an amide derivative of formula I-a, I-b or I-c 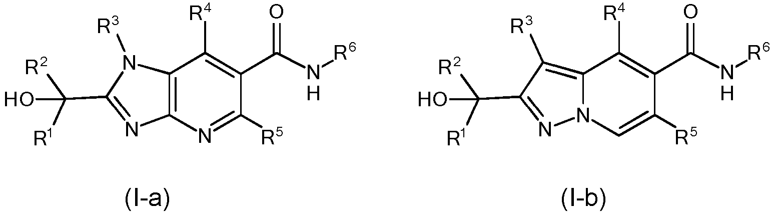 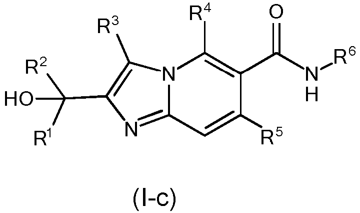
wherein independently from each other
R¹ denotes Ar^{A} or Hetar^{A};
R² denotes Ar^{B} or Hetar^{B};
R³ denotes C₁₋₆-aliphatic or -O-C₁₋₆-aliphatic;
R⁴ denotes H, D, C₁₋₆-aliphatic or -O-C₁₋₆-aliphatic;
R⁵ denotes H, D, C₁₋₆-aliphatic, -O-C₁₋₆-aliphatic or halogen;
R⁶ denotes Hetar^{C} or -CH₂-Hetar^{C};
Ar^{A} is a mono- or biaryl with 5, 6, 7, 8, 9, 10, 11 ring carbon atoms, wherein that aryl may be unsubstituted or substituted with substituents R^{A1}, R^{A2}, R^{A3}, R^{A4} and/or R^{A5} which may be the same or different;
Ar^{B} is a mono- or biaryl with 5, 6, 7, 8, 9, 10, 11 ring carbon atoms, wherein that aryl may be unsubstituted or substituted with substituents R^{B1}, R^{B2}, R^{B3}, R^{B4} and/or R^{B5} which may be the same or different;
Hetar^{A} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{A1}, R^{A2}, R^{A3}, R^{A4} and/or R^{A5} which may be the same or different;
Hetar^{B} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{B1}, R^{B2}, R^{B3}, R^{B4} and/or R^{B5} which may be the same or different;
Hetar^{C} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{C1}, R^{C2}, R^{C3}, R^{C4} and/or R^{C5} which may be the same or different;
R^{A1}, R^{A2}, R^{A3}, R^{A4}, R^{A5}, R^{B1}, R^{B2}, R^{B3}, R^{B4}, R^{B5}, R^{C1}, R^{C2}, R^{C3}, R^{C4}, R^{C5} are independently from each other H, D, halogen, C₁₋₆-aliphatic, -O-C₁₋₆-aliphatic;
halogen denotes F, Cl, Br or I;
or any N-oxide, solvate, tautomer or stereoisomer thereof and/or any pharmaceutically acceptable salt of each of the foregoing, including mixtures thereof in all ratios.

In general, all residues, radicals, substituents, groups, moieties, etc. which occur more than once may be identical or different, i.e. are independent of one another. Above and below, the residues and parameters have the meanings indicated for formula I-a, I-b or I-c, unless expressly indicated otherwise. Accordingly, the invention relates, in particular, to the compounds of formula I-a, I-b or I-c in which at least one of the said residues, radicals, substituents has one of the preferred meanings indicated below.

Any of those particular or even preferred embodiments of the present invention as specified below and in the claims do not only refer to the specified compounds of formula I-a, I-b or I-c but to N-oxides, solvates, tautomers or stereoisomers thereof as well as the pharmaceutically acceptable salts of each of the foregoing, including mixtures thereof in all ratios, too, unless indicated otherwise.

In a particular embodiment, PE1, the compound of the present invention is an amide of formula I-a, I-b or I-c, or any N-oxide, solvate tautomer or stereoisomer thereof and/or any pharmaceutically acceptable salt of each of the foregoing, including mixtures thereof in all ratios, in which R¹ and R² have the same meaning. This means that, for instance, if R¹ denotes Ar^{A}, then R² denotes Ar^{B} which in turn denotes the same Ar^{A} as for R¹, i.e. it is the same substituent; likewise, if R¹ denotes Hetar^{A}, then R² means Hetar^{B} which in turn means the same Hetar^{A} as for R¹ (i.e. it is the same substituent). A further particular embodiment of the present invention, designated as PE2,

is an amide of formula I-a, I-b or I-c, or any N-oxide, solvate, tautomer or stereoisomer thereof and/or any pharmaceutically acceptable salt of each of the foregoing, including mixtures thereof in all ratios, in which R¹ and R² have a different meaning. In this embodiment the substituents R¹ and R² are chosen to be non-identical. For instance, if R¹ denotes 2-methylphenyl (i.e., Ar^{A} is a monocyclic 6-membered aromatic ring and one of the substituents R^{A1}, R^{A2}, R^{A3}, R^{A4} and/or R^{A5} being methyl in 2-position relative to the point of connection of Ar^{A} with the hydroxy-substituted carbon atom to which it is attached while the others are hydrogen), then R² may be Hetar^{B} as defined herein or Ar^{B} except for 2-methylphenyl.

It has to be recognized that any amide derivative of PE2 has a chiral center at the carbon atom that bears the different substituents R¹ and R² as this carbon atom will then be substituted with four different substituents.

In still another particular embodiment of the invention, designated as PE3, the amide derivative of the present invention is an amide of formula I-a, I-b or I-c, or any N-oxide, solvate, tautomer or stereoisomer thereof and/or any pharmaceutically acceptable salt of each of the foregoing, including mixtures thereof in all ratios, in which both substituents R¹ and R² are independently from each other monocyclic rings, i.e.
- Ar^{A}: is phenyl which may be unsubstituted or mono-, di-, tri-, tetra- or pentasubstituted with R^{A1}, R^{A2}, R^{A3}, R^{A4}, R^{A5};
- Ar^{B}: is phenyl which may be unsubstituted or mono-, di-, tri-, tetra- or pentasubstituted with R^{B1}, R^{B2}, R^{B3}, R^{B4}, R^{B5};
- Hetar^{A}: is a monocyclic heteroaryl with 5 or 6 ring atoms or a bicyclic heteroaryl with 10 ring atoms wherein 1 or 2 of said ring atoms is/are a hetero atom(s) selected from N, O and/or S and the remaining are carbon atoms, wherein that heteroaryl may be unsubstituted or mono-, di-, tri-, tetra- or pentasubstituted with R^{A1}, R^{A2}, R^{A3}, R^{A4}, R^{A5};
- Hetar^{B}: is a monocyclic heteroaryl with 5 or 6 ring atoms wherein 1 or 2 of said ring atoms is/are a hetero atom(s) selected from N, O and/or S and the remaining are carbon atoms, wherein that heteroaryl may be unsubstituted or mono-, di-, tri-, tetra- or pentasubstituted with R^{B1}, R^{B2}, R^{B3}, R^{B4}, R^{B5};
wherein R^{A1}, R^{A2}, R^{A3}, R^{A4}, R^{A5} may be the same or different; and R^{B1}, R^{B2}, R^{B3}, R^{B4}, R^{B5} may be the same or different.

If R¹ and R² are identical, i.e. have the same meaning, then this PE3 is also within the scope of PE1. Likewise, if R¹ and R² do not have the same meaning, this PE3 is within the scope of PE2.

In a preferred particular embodiment, PE3a, of PE3
- Ar^{A}: is phenyl which may be unsubstituted or mono- or di-substituted with R^{A1} and/or R^{A2} wherein R^{A1} and/or R^{A2} are as defined in any preceding claim; or trideuterophenyl, tetradeuterophenyl or pentadeuterophenyl;
- Ar^{B}: is phenyl which may be unsubstituted or mono- or di-substituted with R^{B1} and/or R^{B2} wherein R^{B1} and/or R^{B2} are as defined in any preceding claim; or trideuterophenyl, tetradeuterophenyl or pentadeuterophenyl;
- Hetar^{A}: is a monocyclic heteroaryl with 5 or 6 ring atoms or a bicyclic heteroaryl with 10 ring atoms wherein 1 or 2 of said ring atoms is/are a hetero atom(s) selected from N, O and/or S and the remaining are carbon atoms, wherein that heteroaryl may be unsubstituted or substituted with substituents R^{A1} and/or R^{A2} which may be the same or different;
- Hetar^{B}: is a monocyclic heteroaryl with 5 or 6 ring atoms, wherein 1 or 2 of said ring atoms is/are a hetero atom(s) selected from N, O and/or S and the remaining are carbon atoms, wherein that heteroaryl may be unsubstituted or substituted with substituents R^{B1} and/or R^{B2} which may be the same or different.

In still another preferred particular embodiment, PE3b, of PE3a
R^{A1}, R^{A2}, R^{B1}, R^{B2} are independently from each other H, D, F, Cl, unsubstituted or substituted C₁₋₄-aliphatic, unsubstituted or substituted - O-C₁₋₄-aliphatic.

If in PE3a or PE3b substituent Ar^{A} being phenyl is mono-substituted with substituent R^{A1} or R^{A2}, then this substituent R^{A1} or R^{A2} is preferably located in 2-position or 4-position of the phenyl ring. If that phenyl ring Ar^{A} is di-substituted with R^{A1} and R^{A2}, then these two substituents, which may be the same or different, are preferably in 2- and 4-position of that phenyl ring. Likewise, if in PE3a or PE3b substituent Ar^{B} being phenyl is mono-substituted with substituent R^{B1} or R^{B2}, then this substituent R^{B1} or R^{B2} is preferably located in 2-position or 4-position of the phenyl ring. If that phenyl ring Ar^{B} is di-substituted with R^{B1} and R^{B2}, then these two substituents, which may be the same or different, are preferably in 2- and 4-position of that phenyl ring.

In still another particular embodiment, PE4, the amide derivative of the present invention is an amide of formula I-a, I-b or I-c, or any N-oxide, solvate, tautomer or stereoisomer thereof and/or any pharmaceutically acceptable salt of each of the foregoing, including mixtures thereof in all ratios, in which independently from each other
- R³: denotes C₁₋₄-alkyl, preferably C₁₋₂-alkyl which is unsubstituted or mono-substituted with -OH, -OCH₃ or -N(CH₃)₂;
- R⁴: denotes H;
- R⁵: denotes H, C₁₋₄-alkyl, -O-C₁₋₄-alkyl, F or Cl; preferably H, C₁₋₂-alkyl, -O-C₁₋₂-alkyl, F or Cl;
and wherein the remaining radicals, residues, groups or substituents are as defined for the formulas I-a, I-b and I-c above in general or for any other particular embodiments mentioned above, i.e. PE1, PE2, PE3, PE3a, PE3b.

In yet another particular embodiment, PE5, the amide derivative of the present invention is an amide of formula I-a, I-b or I-c, or any N-oxide solvate, tautomer or stereoisomer thereof and/or any pharmaceutically acceptable salt of each of the foregoing, including mixtures thereof in all ratios, in which
- R⁶: denotes -CH₂-Hetar^{C} or
- Hetar^{C}: is a monocyclic heteroaryl with 5 or 6 ring atoms wherein 1, 2 or 3 of said ring atoms is/are a hetero atom(s) selected from N, O and/or S and the remaining are carbon atoms, wherein that heteroaryl may be unsubstituted or substituted with substituents R^{C1} and/or R^{C2} which may be the same or different;
- R^{C1}, R^{C2}: denote H or substituted or unsubstituted C₁₋₄-alkyl wherein at least one of R^{C1} and R^{C2} is not H.

In a preferred particular embodiment, PE5a, of PE5
- R⁶: denotes Hetar^{C};
- Hetar^{C}: is a monocyclic heteroaryl with 5 ring atoms wherein 2 or 3 of said ring atoms is/are a hetero atom(s) selected from N and/or O and the remaining are carbon atoms, wherein that heteroaryl may be unsubstituted or substituted with one substituent R^{C1};
- R^{C1}: denotes ethyl, 2-aminoethyl, 2-hydroxyethyl, 2-methoxyethyl.

In a specific particular embodiment, PE5b, of PE5a substituent R^{C1} is in 3-position of the monocyclic heteroaryl Hetar^{C} relative to the point of attachement of that heteroaryl moiety Hetar^{C} to the nitrogen atom of the adjacent amido group.

In another specific particular embodiment, PE5c, of PE5 or PE5a or PE5b R¹ and R² are the same. In another specific particular embodiment, PE5d, of PE5 or PE5a or PE5b R¹ and R² are different.

In still another particular embodiment, PE6, the amide derivative of the present invention is an amide of formula I-a, I-b or I-c, or any N-oxide solvate, tautomer or stereoisomer thereof and/or any pharmaceutically acceptable salt of each of the foregoing, including mixtures thereof in all ratios, in which independently from each other
- R¹: denotes Ar^{A} or Hetar^{A};
- R²: denotes Ar^{B} or Hetar^{B};
- R³: denotes methyl, ethyl, 2-dimethylaminoethyl, 2-hydroxyethyl, 2-methoxyethyl;
- R⁴: denotes H;
- R⁵: denotes H, methyl, ethyl, methoxy, F or Cl;
- R⁶: denotes Hetar^{C};
- Ar^{A}: phenyl; mono-, di-, tri-, tetra- or pentadeuterophenyl; preferably pentadeuterophenyl; fluorophenyl, preferably 2-fluorophenyl; methylphenyl (tolyl), preferably 2-methylphenyl; methoxyphenyl, preferably 2-methoxyphenyl; difluoromethoxy, preferably 4-difluoromethoxy; 4-difluoromethoxy-2-fluorophenyl;
- Ar^{B}: phenyl; mono-, di-, tri-, tetra- or pentadeuterophenyl; preferably pentadeuterophenyl; fluorophenyl, preferably 2-fluorophenyl; methylphenyl (tolyl), preferably 2-methylphenyl; methoxyphenyl, preferably 2-methoxyphenyl; difluoromethoxy, preferably 4-difluoromethoxy; 4-difluoromethoxy-2-fluorophenyl;
- Hetar^{A} yl;: methylpyrazolyl, preferably 1-methlypyrazol-3-yl, 1-methylpyrazol-4-yl; thien-2-yl, thien-3-yl; methylthienyl, preferably 5-methylthien-2-yl; thiazolyl, preferably 1,3-thiazol-2-yl; pyridinyl, preferably pyridin-2-yl; pyrimidinyl, preferably pyrimidin-4-yl; pyridazinyl, preferably pyridazin-3-quinolinyl, preferably quinolin-2-yl;
- Hetar^{B}: thien-2-yl, thien-3-yl; methylthienyl, preferably 5-methylthien-2-yl; pyridinyl, preferably pyridin-2-yl;
- Hetar^{C}: ethylpyrazolyl, preferably 1-ethylpyrazol-3-yl, 1-ethylpyrazol-4-yl; hydroxyethylpyrazolyl, preferably 1-(2-hydroxyethyl)pyrazol-4-yl; methoxyethylpyrazolyl, preferably 1-(2-methyoxyethyl)pyrazol-4-yl; ethylimidazolyl, preferably 1-ethyl-1H-imidazol-4-yl; ethyloxazolyl, preferably 4-ethyl-1,3-oxazol-2-yl, 5-ethyl-1,3-oxazol-2-yl; ethyltriazolyl, preferably 1-ethyl-1H-1,2 ,2,4-triazol-3-yl, 2-ethyl-2H-1,2,3-triazol-4-yl; aminoethyltriazolyl, preferably 1-(2-aminoethyl)-1H-1,2,4-triazol-3-yl; hydroxyethyltriazolyl, preferably 1-(2hydroxyethyl)-1H-1,2,4-triazol-3-yl; methoxyethyltriazolyl, preferably 1-(2-methoxyethyl)-1H-1,2,4-triazol-3-yl; ethyloxadiazolyl, preferably 5-ethyl-1,3,4-oxadiazol-2-yl, 5-ethyl-1,2,4-oxadiazol-3-yl.

If R¹ and R² are identical, i.e. have the same meaning, then this is another particular embodiment PE6a of PE6. Likewise, if R¹ and R² do not have the same meaning, this is yet another particular embodiment PE6b of PE6.

In yet another particular embodiment, PE7, the amide derivative of the present invention is selected from a compound of formula I-a (i.e. 1H-imidazo[4,5-b]pyridine derivatives), or any N-oxide, solvate, tautomer or stereoisomer thereof and/or any pharmaceutically acceptable salt of each of the foregoing, including mixtures thereof in all ratios, in which R¹, R², R³, R⁴, R⁵ and R⁶ are as defined in claim 1 or for any of the particular embodiments PE1, PE2, PE3, PE3a, PE3b, PE4, PE5, PE5a, PE5b, PE5c, PE5d, PE6, PE6a, PE6b. In a specific particular embodiment, PE7a, of PE7
- R¹: denotes phenyl; mono-, di-, tri-, tetra- or pentadeuterophenyl, preferably pentadeuterophenyl; fluorophenyl, preferably 2-fluorophenyl; methylphenyl (tolyl), preferably 2-methylphenyl; methoxyphenyl, preferably 2-methoxyphenyl; difluoromethoxy, preferably 4-difluoromethoxy; 4-difluoromethoxy-2-fluorophenyl; methylpyrazolyl, preferably 1-methlypyrazol-3-yl, 1-methylpyrazol-4-yl; thien-2-yl, thien-3-yl; methylthienyl, preferably 5-methylthien-2-yl; thiazolyl, preferably 1,3-thiazol-2-yl; pyridinyl, preferably pyridin-2-yl; pyrimidinyl, preferably pyrimidin-4-yl; pyridazinyl, preferably pyridazine-3-yl; quinolinyl, preferably quinoline-2-yl;
- R²: denotes phenyl; mono-, di-, tri-, tetra- or pentadeuterophenyl, preferably pentadeuterophenyl; fluorophenyl, preferably 2-fluorophenyl; methylphenyl (tolyl), preferably 2-methylphenyl; difluoromethoxy, preferably 4- difluoromethoxy; thien-2-yl, thien-3-yl; methylthienyl, preferably 5-methylthien-2-yl; pyridinyl, preferably pyridin-2-yl;
- R³: denotes methyl, ethyl, 2-dimethylaminoethyl, 2-hydroxyethyl, 2-methoxyethyl; preferably methyl or ethyl;
- R⁴: is H;
- R⁵: denotes H, methyl, methoxy, F, Cl;
- R⁶: denotes ethylpyrazolyl, preferably 1-ethylpyrazol-3-yl, 1-ethylpyrazol-4-yl; hydroxyethylpyrazolyl, preferably 1-(2-hydroxyethyl)pyrazol-4-yl; methoxyethylpyrazolyl, preferably 1-(2-methyoxyethyl)pyrazol-4-yl; ethylimidazolyl, preferably 1-ethyl-1H-imidazol-4-yl; ethyloxazolyl, preferably 4-ethyl-1,3-oxazol-2-yl, 5-ethyl-1,3-oxazol-2-yl; ethyltriazolyl, preferably 1-ethyl-1H-1,2,4-triazol-3-yl, 2-ethyl-2H-1,2,3-triazol-4-yl; aminoethyltriazolyl, preferably 1-(2-aminoethyl)-1H-1,2,4-triazol-3-yl; hydroxyethyltriazolyl, preferably 1-(2hydroxyethyl)-1H-1,2,4-triazol-3-yl; methoxyethyltriazolyl, preferably 1-(2-methoxyethyl)-1H-1,2,4-triazol-3-yl; ethyloxadiazolyl, preferably 5-ethyl-1,3,4-oxadiazol-2-yl, 5-ethyl-1,2,4-oxadiazol-3-yl.

In another specific particular embodiment, PE7b, of PE7 or PE7a R¹ and R² are the same. In another specific particular embodiment, PE7c, of PE7 or PE7a R¹ and R² are different.

In yet another particular embodiment, PE8, the amide derivative of the present invention is selected from a compound of formula I-b (i.e. pyrazolo[1,5-a]pyridine derivatives), or any N-oxide, solvate, tautomer or stereoisomer thereof and/or any pharmaceutically acceptable salt of each of the foregoing, including mixtures thereof in all ratios, in which R¹, R², R³, R⁴, R⁵ and R⁶ are as defined in claim 1 or for any of the particular embodiments PE1, PE2, PE3, PE3a, PE3b, PE4, PE5, PE5a, PE5b, PE5c, PE5d, PE6, PE6a, PE6b. In a specific particular embodiment, PE8a, of PE8
- R¹: denotes phenyl, 2-fluorophenyl;
- R²: denotes phenyl, 2-fluorophenyl;
- R³: denotes ethyl;
- R⁴: is H;
- R⁵: denotes H, methyl, methoxy, Cl;
- R⁶: denotes ethylpyrazolyl, ethyltriazolyl; preferably 1-ethylpyrazol-4-yl, 1-ethyl-1H-1,2,4-triazol-3-yl.

In another specific particular embodiment, PE8b, of PE8 or PE8a R¹ and R² are the same. In another specific particular embodiment, PE8c, of PE8 or PE8a R¹ and R² are different.

In yet another particular embodiment, PE 9, the amide derivative of the present invention is selected from a compound of formula I-c (i.e. imidazo[1,2-a]pyridine derivatives), or any N-oxide, solvate, tautomer or stereoisomer thereof and/or any pharmaceutically acceptable salt of each of the foregoing, including mixtures thereof in all ratios, in which R¹, R², R³, R⁴, R⁵ and R⁶ are as defined in claim 1 or for any of the particular embodiments PE1, PE2, PE3, PE3a, PE3b, PE4, PE5, PE5a, PE5b, PE5c, PE5d, PE6, PE6a, PE6b. In a specific particular embodiment, PE9a, of PE9
- R¹: denotes phenyl, 2-fluorophenyl, 1-methlypyrazol-3-yl, 5-methylthien-2-yl, 1,3-thiazol-2-yl, pyridin-2-yl;
- R²: denotes phenyl;
- R³: denotes ethyl;
- R⁴: H;
- R⁵: H, ethyl, methoxy, F, Cl;
- R⁶: denotes ethylpyrazolyl, ethyltriazolyl; preferably 1-ethylpyrazol-4-yl, 1-ethyl-1H-1,2,4-triazol-3-yl.

In another specific particular embodiment, PE9b, of PE9 or PE9a R¹ and R² are the same. In another specific particular embodiment, PE9c, of PE9 or PE9a R¹ and R² are different.

In still another particular embodiment, PE10, the amide derivative of formula I-a, I-b or I-c, is selected from the group consisting of the compounds shown in Table 1 which is divided in Tables 1a, 1b, 1c and 1d as well as any any N-oxide, solvate, tautomer or stereoisomer thereof and/or any pharmaceutically acceptable salt of each of the foregoing, including mixtures thereof in all ratios.

As used herein, the following definitions shall apply unless otherwise indicated or defined specifically elsewhere in the description and/or the claims for specific substituents, radicals, residues, groups or moieties.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon or tricyclic hydrocarbon that is completely saturated or that contains one or more units of unsaturation, such as one or more C=C double bond(s) and/or C≡C triple bond(s), but which is not aromatic (also referred to herein as "carbocycle", "cycloaliphatic" or "cycloalkyl"), that has - in general and if not defined otherwise in this specification or the accompanied claims - a single point of attachment to the rest of the molecule. Unless otherwise specified, aliphatic groups contain 1-8 or 1-6 aliphatic carbon atoms. In some embodiments, aliphatic groups contain 1-5 aliphatic carbon atoms. In other embodiments, aliphatic groups contain 1-4 aliphatic carbon atoms. In still other embodiments, aliphatic groups contain 1-3 aliphatic carbon atoms, and in yet other embodiments, aliphatic groups contain 1-2 aliphatic carbon atoms. In some embodiments, "cycloaliphatic" ("cycloalkyl") refers to a monocyclic C₃-C₇ hydrocarbon that is completely saturated or that contains one or more units of unsaturation, but which is not aromatic, that has a single point of attachment to the rest of the molecule. In another embodiment the term "carbocycle" refers to a monocyclic or bicyclic cycloaliphatic ring system which is fused to an aromatic, heteroaromatic or heterocyclic ring or ring system via 2 adjacent ring atoms of that aromatic, heteroaromatic or heterocyclic ring or ring system; in other words, such carbocycle shares two ring atoms with the ring or ring system to which it is fused thereby having two points of attachement to the rest of the molecule. Suitable aliphatic groups include, but are not limited to, linear or branched, substituted or unsubstituted alkyl, alkenyl, alkynyl groups and hybrids thereof as (cycloalkyl)alkyl, (cycloalkenyl)alkyl or (cycloalkyl)alkenyl.

The term "alkyl" usually refers to a saturated aliphatic and acyclic moiety, while the term "alkenyl" usually refers to an unsaturated alphatic and acyclic moiety with one or more C=C double bonds and the term "alkynyl" usually refers to an aliphatic and acyclic moiety with one or more C≡C triple bonds. Exemplary aliphatic groups are linear or branched, substituted or unsubstituted C₁₋₈-alkyl, C₁₋₆-alkyl, C₁₋₄-alkyl, C₂₋₈-alkenyl, C₂₋₆-alkenyl, C₂₋₈-alkynyl, C₂₋₆-alkynyl groups and hybrids thereof such as (cycloalkyl)alkyl, (cycloalkenyl)alkyl or (cycloalkyl)alkenyl.

In particular, the term "C₁₋₃-alkyl" refers to alkyl groups, i.e. saturated acyclic aliphatic groups, having 1, 2 or 3 carbon atoms. Exemplary C₁₋₃-alkyl groups are methyl, ethyl, propyl and isopropyl. The term "C₁₋₄-alkyl" refers to alkyl groups having 1, 2, 3 or 4 carbon atoms. Exemplary C₁₋₄-alkyl groups are methyl, ethyl, propyl, isopropyl, butyl, isobutyl, and tert-butyl. The term "C₁₋₆-alkyl" refers to alkyl groups having 1, 2, 3, 4, 5 or 6 carbon atoms. Exemplary C₁₋₆-alkyl groups are methyl, ethyl, propyl, isopropyl, butyl, isobutyl, tert-butyl, n-pentyl, 2-pentyl, n-hexyl, and 2-hexyl. The term "C₁₋₈-alkyl" refers to alkyl groups having 1, 2, 3, 4, 5, 6, 7, or 8 carbon atoms. Exemplary C₁₋₈-alkyl groups are methyl, ethyl, propyl, isopropyl, butyl, isobutyl, tert-butyl, n-pentyl, 2-pentyl, n-hexyl, 2-hexyl n-heptyl, 2-heptyl, n-octyl, 2-octyl, and 2,2,4-trimethylpentyl. Each of these alkyl groups may be straight-chain or - except for C₁-alkyl and C₂-alkyl - branched and may be unsubstituted or substituted with 1, 2 or 3 substituents that may be the same or different and are, if not specified differently elsewhere in this specification, selected from the group comprising halogen, hydroxy, alkoxy, unsubstituted amino or amino which is mono- or disubstituted with alkyl or aralkyl.

In some instances the C₁₋₃-alkyl, C₁₋₄-alkyl, C₁₋₆-alkyl, C₁₋₈-alkyl groups may also comprise those residues in which 1 or 2 of non-terminal and non-adjacent -CH₂- (methylene) groups are replaced by -O-, -S- and/or 1 or 2 non-terminal and non-adjacent -CH₂- or -CH- groups are replaced by -NH- or -N-. These replacements yield, for instance, (modified) alkyl groups like -CH₂-CH₂-O-CH₃, -CH₂-CH₂-CH₂-S-CH₃, CH₂-CH₂-NH-CH₂-CH₃, CH₂-CH₂-O-CH₂-CH₂-O-CH₃, CH₂-CH₂-N(CH₃)-CH₂-CH₃, and the like. Further and/or different replacements of -CH- and -CH₂- groups may be defined for specific alkyl substituents or radicals elsewhere in the description and/or the claims.

The term "C₃₋₇-cycloalkyl" refers to a cycloaliphatic hydrocarbon, as defined above, with 3, 4, 5, 6 or 7 ring carbon atoms. C₃₋₇-cycloalkyl groups may be unsubstituted or substituted with - unless specified differently elsewhere in this specification - 1, 2 or 3 substituents that may be the same of different and are - unless specified differently elsewhere in this specification - selected from the group comprising C₁₋₆-alkyl, O-C₁₋₆-alkyl (alkoxy), halogen, hydroxy, unsubstituted or mono- or di-substituted amino. Exemplary C₃₋₇-cycloalkyl groups are cyclopropyl, 2-methyl-cyclopropyl, cyclopropenyl, cyclobutyl, cyclobutenyl, cyclopentyl, cyclopentenyl, cyclohexyl, cyclohexenyl, cycloheptyl, cycloheptenyl.

The term "aliphatoxy" refers to saturated or unsaturated aliphatic groups or substituents as defined above that are connected to another structural moiety via an oxygen atom (-O-). The term "alkoxy" refers to a particular subgroup of saturated aliphatoxy, i.e. to alkyl substituents and residues that are connected to another structural moiety via an oxygen atom (-O-). Sometimes, it is also referred to as "O-alkyl" and more specifically as "O-C₁₋₄-alkyl", "O-C₁₋₆-alkyl", "O-C₁₋₈-alkyl". Like the similar alkyl groups, it may be straight-chain or - except for -O-C₁-alkyl and -O-C₂-alkyl - branched and may be unsubstituted or substituted with 1, 2 or 3 substituents that may be the same or different and are, if not specified differently elsewhere in this specification, selected from the group comprising halogen, unsubstituted or mono- or di-substituted amino. Exemplary alkoxy groups are methoxy, fluoromethoxy, difluoromethoxy, trifluoromethoxy, ethoxy, 2,2,2-trifluoroethoxy, n-propoxy, iso-propoxy, n-butoxy, sec-butoxy, tert-butoxy, n-pentoxy.

The term "alkylene" refers to a divalent aliphatic group and in particular a divalent alkyl group. An "alkylene chain" is a polymethylene group, i.e., - (CH₂)ₓ-, wherein x is a positive integer, preferably 1, 2, 3, 4, 5 or 6. In the context of the present invention "C₁₋₃-alkylene" refers to an alkylene moiety with 1, 2 and 3, respectively, -CH₂- groups; the term "alkylene", however, not only comprises linear alkylene groups, i.e. "alkylene chains", but branched alkylene groups as well. The term "C₁₋₆-alkylene" refers to an alkylene moiety that is either linear, i.e. an alkylene chain, or branched and has 1, 2, 3, 4, 5 or 6 carbon atoms. A substituted alkylene chain is a polymethylene group in which one or more methylene hydrogen atoms are replaced by (or with) a substituent. Suitable substituents include those described herein for a substituted alkyl group. In some instances 1 or 2 methylene groups of the alkylene chain may be replaced by, for instance, O, S and/or NH or N-C₁₋₄-alkyl. Exemplary alkylene groups are -CH₂-, -CH₂-CH₂-, -CH₂-CH₂-CH₂-CH₂-, -O-CH₂-O-, -O-CH₂-CH₂-O-, -O-CH₂-CH₂-CH₂-O-,-CH₂-NH-CH₂-CH₂-, -CH₂-N(CH₃)-CH₂-CH₂-.

The term "alkenylene" refers to a bivalent alkenyl group. A substituted alkenylene chain is a polymethylene group containing at least one double bond in which one or more hydrogen atoms are replaced with a substituent. Suitable substituents include those described herein for a substituted aliphatic group.

The term "alkynylene" refers to a bivalent alkynyl group. A substituted alkynylene chain is a polymethylene group containing at least one triple bond in which one or more hydrogen atoms are replaced with a substituent. Suitable substituents include those described herein for a substituted aliphatic group.

The term "halogen" means F, Cl, Br, or I.

The term "heteroatom" means one or more of oxygen (O), sulfur (S), or nitrogen (N), including, any oxidized form of nitrogen or sulfur, e.g. N-oxides, sulfoxides and sulfones; the quaternized form of any basic nitrogen or a substitutable nitrogen of a heterocyclic or heteroaromatic ring, for example N (as in 3,4-dihydro-2*H*-pyrrolyl), NH (as in pyrrolidinyl) or N-SUB with SUB being a suitable substituent (as in N-substituted pyrrolidinyl).

The term "aryl" used alone or as part of a larger moiety as in "aralkyl", "aralkoxy", or "aryloxyalkyl", refers to monocyclic, bicyclic and tricyclic ring systems having a total of five to fourteen ring members, that ring members being carbon atoms, wherein at least one ring in the system is aromatic, i.e., it has (4n+2) π (pi) electrons (with n being an integer selected from 0, 1, 2, 3), which electrons are delocalized over the system, and wherein each ring in the system contains three to seven ring members. Preferably, all rings in the aryl system or the entire ring system are aromatic. The term "aryl" is used interchangeably with the term "aryl ring". In certain embodiments of the present invention, "aryl" refers to an "aromatic ring system". More specifically, those aromatic ring systems may be mono-, bi- or tricyclic with 5, 6, 7, 8, 9, 10, 11, 12, 13, 14 ring carbon atoms. Even more specifically, those aromatic ring systems may be mono- or bicyclic with 6, 7, 8, 9, 10 ring carbon atoms. The term "monoaryl" refers to a monocyclic aryl. The term "biaryl" refers to a bicyclic aryl. Exemplary aryl groups are phenyl, biphenyl, naphthyl, anthracyl and the like, which may be unsubstituted or substituted with one or more identical or different substituents. Also included within the scope of the terms "aryl" or "aromatic ring system", as they are used herein, is a group in which an aromatic ring is fused to one or more non-aromatic rings, such as indanyl, phthalimidyl, naphthimidyl, phenanthridinyl, or tetrahydronaphthyl, and the like. In the latter case the "aryl" group or substituent is attached to its pendant group via the aromatic part of the ring system.

The term "benzo" refers to a six-membered aromatic ring (with carbon ring atoms) that is fused via two adjacent carbon atoms to another ring, being it a cycloaliphatic, aromatic, heteroaromatic or heterocyclic (heteroaliphatic) ring; as a result a ring sytem with at least two rings is formed in which the benzo ring shares two common carbon atoms with the other ring to which it is fused. For example, if a benzo ring is fused to a phenyl ring, a napthaline ring system is formed, while fusing a benzo ring to a pyridine provides for either a quinoline or an isoquinoline.

The terms "heteroaryl" and "heteroar-", used alone or as part of a larger moiety, e.g., "heteroaralkyl", or "heteroaralkoxy", refer to groups having 3, 4, 5, 6, 7, 8, 9, 10, 11, 12, 13, 14 ring atoms (which atoms are carbon and hetero atoms), preferably 5, 6, 9 or 10 ring atoms; having 6, 10, or 14 π (pi) electrons shared in a cyclic array; and having, in addition to carbon atoms, 1, 2, 3, 4 or 5 heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, furazanyl, pyridyl (pyridinyl), pyridazinyl, pyrimidinyl, pyrazinyl, indolizinyl, purinyl, naphthyridinyl, pteridinyl, and pyrrolopyridinyl, in particular pyrrolo[2,3-b]pyridinyl. The terms "heteroaryl" and "heteroar-", as used herein, also include groups in which a heteroaromatic ring is fused to one or more aryl, cycloaliphatic, or heterocyclyl rings, where the radical or point of attachment is preferably on the heteroaromatic or, if present, the aryl ring. Nonlimiting examples include indolyl, isoindolyl, benzothienyl (benzothiophenyl), benzofuranyl, dibenzofuranyl, indazolyl, benzimidazolyl, benzothiazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, 9H-carbazolyl, dibenzofuranyl and pyrido[2,3-b]-1,4-oxazin-3(4H)-one. For example, an indolyl ring may be attached via one of the ring atoms of the six-membered aryl ring or via one of the ring atoms of the five-membered heteroaryl ring. A heteroaryl group is optionally mono-, bi- or tricyclic. The term "heteroaryl" is used interchangeably with the terms "heteroaryl ring", "heteroaryl group", or "heteroaromatic", any of which terms include rings that are unsubstituted or substituted with one or more identical or different substituents. The term "heteroaralkyl" refers to an alkyl group substituted by a heteroaryl, wherein the alkyl and heteroaryl portions independently are optionally substituted.

A heteroaryl ring can be attached to its pendant group at any of its hetero or carbon ring atoms which attachment results in a stable structure or molecule: any of the ring atoms may be unsubstituted or substituted.

The structures of typical examples of "heteroaryl" substituents as used in the present invention are depicted below:

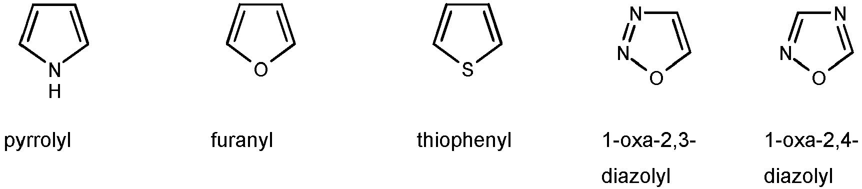

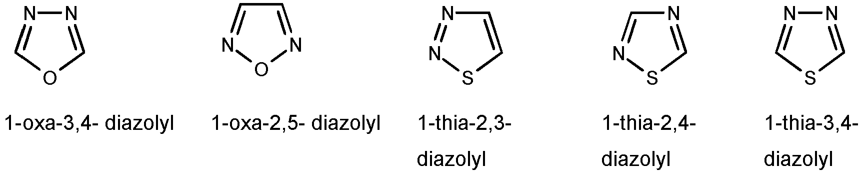

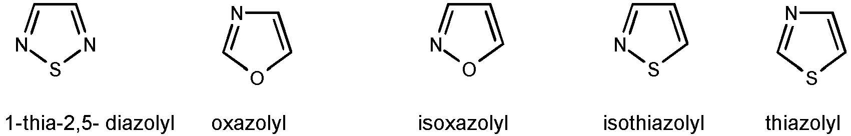

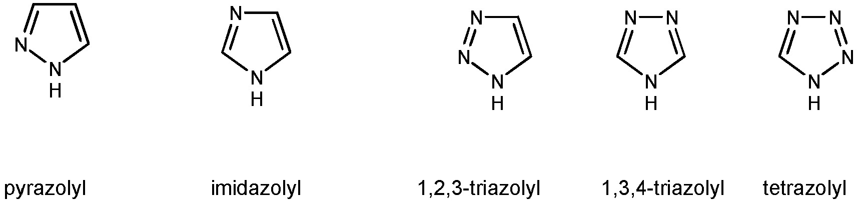

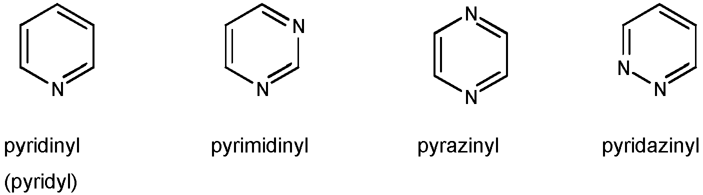

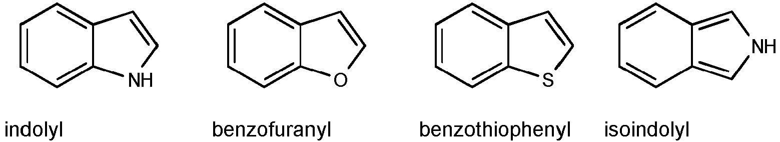

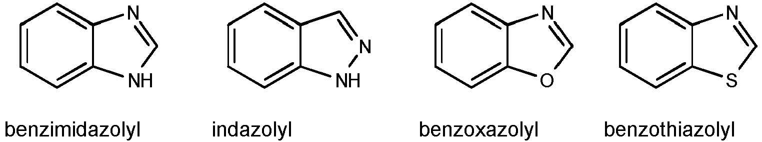

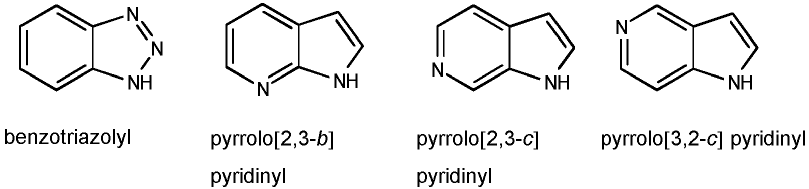

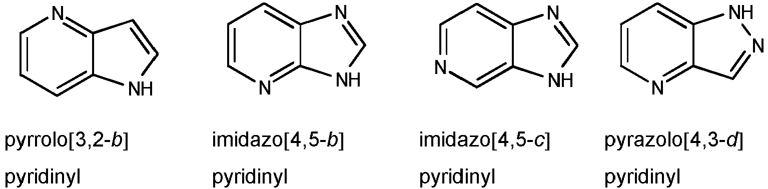

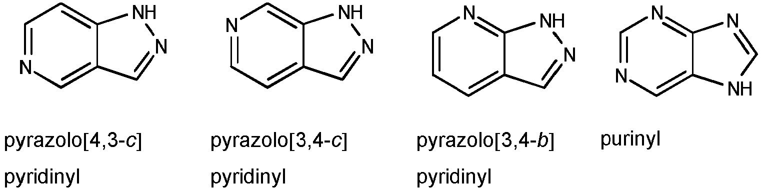

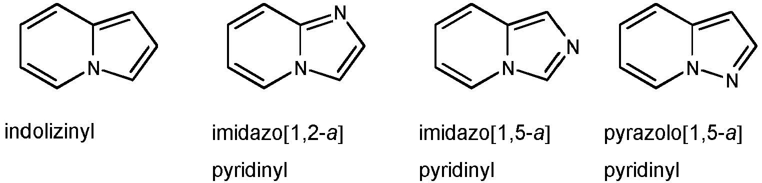

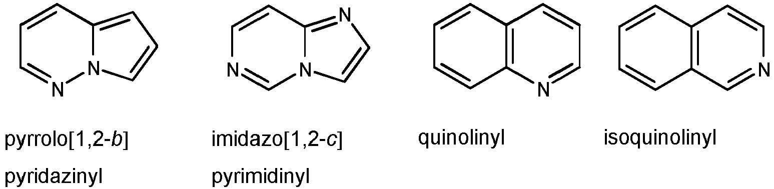

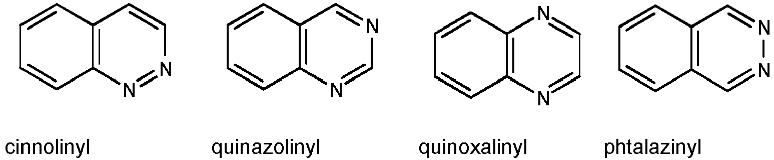

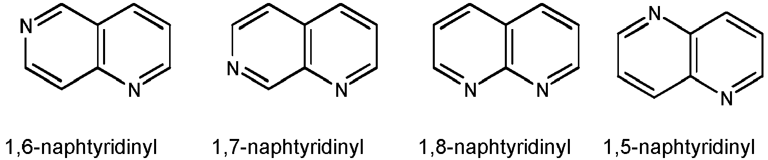

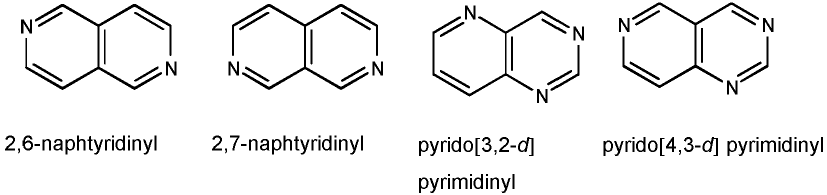

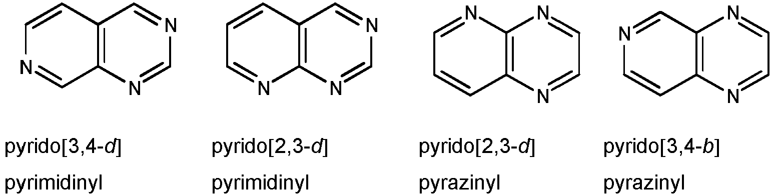

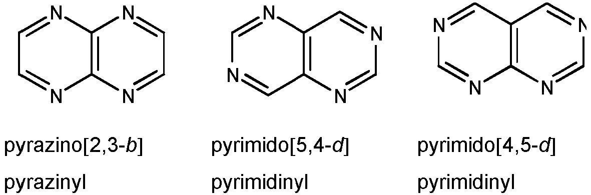

Those heteroaryl substituents can be attached to any pendant group via any of its ring atoms suitable for such an attachment.

When used in reference to a ring atom of a heterocycle, the term "nitrogen" includes a substituted nitrogen. As an example, in a saturated or partially unsaturated ring having 1-3 heteroatoms selected from oxygen, sulfur or nitrogen, the nitrogen is N (as in 3,4-dihydro-2H-pyrrolyl), NH (as in pyrrolidinyl), or N-SUB with SUB being a suitable substituent (as in N-substituted pyrrolidinyl).

The term "unsaturated", as used herein, means that a moiety or group or substituent has one or more units of unsaturation.

As used herein, the term "bicyclic", "bicyclic ring" or "bicyclic ring system" refers to any bicyclic ring system, i.e. carbocyclic or heterocyclic, saturated or having one or more units of unsaturation, i.e. being partially unsaturated or aromatic, having one or more atoms in common between the two rings of the ring system. Thus, the term includes any permissible ring fusion, such as *ortho-*fused or spirocyclic. As used herein, the term "heterobicyclic" is a subset of "bicyclic" that requires that one or more heteroatoms are present in one or both rings of the bicycle. Such heteroatoms may be present at ring junctions and are optionally substituted, and may be selected from nitrogen (including N-oxides), oxygen, sulfur (including oxidized forms such as sulfones and sulfonates), phosphorus (including oxidized forms such as phosphates), boron, etc. In some embodiments, a bicyclic group has 7-12 ring members and 0-4 heteroatoms independently selected from nitrogen, oxygen, or sulfur. Likewise, the term "tricyclic", "tricyclic ring" or "tricyclic ring system" refers to any tricyclic ring system, i.e. carbocyclic or heterocyclic, saturated or having one or more units of unsaturation, i.e. being partially unsaturated or aromatic, in which a bicyclic ring system (as defined above) is fused with another, third ring. Thus, the term includes any permissible ring fusion. As used herein, the term "heterotricyclic" is a subset of "tricyclic" that requires that one or more heteroatoms are present in one or both rings of the tricycle. Such heteroatoms may be present at ring junctions and are optionally substituted, and may be selected from nitrogen (including N-oxides), oxygen, sulfur (including oxidized forms such as sulfones and sulfonates), phosphorus (including oxidized forms such as phosphates), boron, etc. In some embodiments, a tricyclic group has 10-14 ring members and 0-5 heteroatoms independently selected from nitrogen, oxygen, or sulfur.

As described herein, certain compounds of the invention contain "substituted" or "optionally substituted" moieties. In general, the term "substituted", whether preceded by the term "optionally" or not, means that one or more hydrogens of the designated moiety are replaced with a suitable substituent. "Substituted" applies to one or more hydrogens that are either explicit or implicit from the structure. Unless otherwise indicated, a "substituted" or "optionally substituted" group has a suitable substituent at each substitutable position of the group, and when more than one position in any given structure is substituted with more than one substituent selected from a specified group, the substituent is either the same or different at every position. If a certain group, substituent, moiety or radical is "mono-substituted", it bears one (1) substituent. If it is "di-substituted", it bears two (2) substituents, being either the same or different; if it is "tri-substituted", it bears three (3) substituents, wherein all three are the same or two are the same and the third is different or all three are different from each other. Combinations of substituents envisioned by this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

If not specified otherwise elsewhere in the specification or the accompanying claims it is understood that each optional substituent on a substitutable carbon is a monovalent substituent independently selected from halogen; -(CH₂)₀₋₄R°; -(CH₂)₀₋₄OR°; -O(CH₂)₀₋₄R°, -O-(CH₂)₀₋₄C(O)OR°; -(CH₂)₀₋₄CH(OR°)₂; -(CH₂)₀₋₄SR°; -(CH₂)₀₋₄Ph, which may be substituted with one or more R°; - (CH₂)₀₋₄O(CH₂)₀₋₁Ph which may be substituted with one or more R°; - CH=CHPh, which may be substituted with one or more R°; -(CH₂)₀₋₄O(CH₂)₀₋₁-pyridyl which may be substituted with one or more R°; -NO₂; -CN; - N₃; -(CH₂)₀₋₄N(R°)₂; -(CH₂)₀₋₄N(R°)C(O)R°; -N(R°)C(S)R°; -(CH₂)₀₋₄N(R°)C(O)NR°₂; -N(R°)C(S)NR°₂; -(CH₂)₀₋₄N(R°)C(O)OR°; - N(R°)N(R°)C(O)R°; -N(R°)N(R°)C(O)NR°₂; -N(R°)N(R°)C(O)OR°; -(CH₂)₀₋₄C(O)R°; -C(S)R°; -(CH₂)₀₋₄C(O)OR°; -(CH₂)₀₋₄C(O)SR°; -(CH₂)₀₋₄C(O)OSiR°₃; -(CH₂)₀₋₄OC(O)R°; -OC(O)(CH₂)₀₋₄SR-, SC(S)SR°; -(CH₂)₀₋₄SC(O)R°; -(CH₂)₀₋₄C(O)NR°₂; -C(S)NR°₂; -C(S)SR°; -SC(S)SR°, -(CH₂)₀₋₄OC(O)NR°₂; -C(O)N(OR°)R°; -C(O)C(O)R°; -C(O)CH₂C(O)R°; - C(NOR°)R°; -(CH₂)₀₋₄SSR°; -(CH₂)₀₋₄S(O)₂R°; -(CH₂)₀₋₄S(O)₂OR°; -(CH₂)₀₋₄OS(O)₂R°; -S(O)₂NR°₂; -S(O)(NR°)R°; -S(O)₂N=C(NR°₂)₂; -(CH₂)₀₋₄S(O)R°; -N(R°)S(O)₂NR°₂; -N(R°)S(O)₂R°; -N(OR°)R°; -C(NH)NR°₂; - P(O)₂R°; -P(O)R°₂; -OP(O)R°₂; -OP(O)(OR°)₂; SiR°₃; -(C₁₋₄ straight or branched alkylene)O-N(R°)₂; or -(C₁₋₄ straight or branched alkylene)C(O)ON(R°)₂. It is understood that "Ph" means phenyl; and that "-(CH₂)₀₋₄" means that there is either no alkylene group if the subscript is "0" (zero) or an alkylene group with 1, 2, 3 or 4 CH₂ units.

Each R° is independently hydrogen, halogen, C₁₋₆ aliphatic, -CH₂Ph, - O(CH₂)₀₋₁Ph, -CH₂-(5-6 membered heteroaryl ring), or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted by a divalent substituent on a saturated carbon atom of R° selected from =O and =S; or each R° is optionally substituted with a monovalent substituent independently selected from halogen, -(CH₂)₀₋₂R^{•}, -(haloR^{•}), - (CH₂)₀₋₂OH, -(CH₂)₀₋₂OR^{•}, -(CH₂)₀₋₂CH(OR^{•})₂; -O(haloR^{•}), -CN, -N₃, - (CH₂)₀₋₂C(O)R^{•}, -(CH₂)₀₋₂C(O)OH, -(CH₂)₀₋₂C(O)OR^{•}, -(CH₂)₀₋₂SR^{•}, - (CH₂)₀₋₂SH, -(CH₂)₀₋₂NH₂, -(CH₂)₀₋₂NHR^{•}, -(CH₂)₀₋₂NR^{•}₂, -NO₂, -SiR^{•}₃, - OSiR^{•}₃, -C(O)SR^{•}, -(C₁₋₄ straight or branched alkylene)C(O)OR^{•}, or -SSR^{•}. It is understood that "Ph" means phenyl; "halo" means halogen; and "-(CH₂)₀₋₂" means that there is either no alkylene group if the subscript is "0" (zero) or an alkylene group with 1 or 2 CH₂ units.

Each R^{•} is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens; or wherein an optional substituent on a saturated carbon is a divalent substituent independently selected from =O, =S, =NNR*₂, =NNHC(O)R*, =NNHC(O)OR*, =NNHS(O)₂R*, =NR*, =NOR*, -O(C(R*₂))₂₋₃O-, or -S(C(R*₂))₂₋₃S-, or a divalent substituent bound to vicinal substitutable carbons of an "optionally substituted" group is -O(CR*₂)₂₋₃O-, wherein each independent occurrence of R* is selected from hydrogen, C₁₋₆ aliphatic or an unsubstituted 5-6-membered saturated, partially unsaturated, or aryl ring having 0-4 heteroatoms independently selected from nitrogen, oxygen, or sulfur.

When R* is C₁₋₆ aliphatic, R* is optionally substituted with halogen, - R^{•}, -(haloR^{•}), -OH, -OR^{•}, -O(haloR^{•}), -CN, -C(O)OH, -C(O)OR^{•}, -NH₂, - NHR^{•}, -NR^{•}₂, or -NO₂, wherein each R^{•} is independently selected from C₁-₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens.

An optional substituent on a substitutable nitrogen is independently -R^{†}, - NR^{†}₂, -C(O)R^{†}, -C(O)OR^{†}, -C(O)C(O)R^{†}, - C(O)CH₂C(O)R^{†}, -S(O)₂R^{†}, -S(O)₂NR^{†}₂, -C(S)NR^{†}₂, -C(NH)NR^{†}₂, or - N(R^{†})S(O)₂R^{†}; wherein each R^{†} is independently hydrogen, C₁₋₆ aliphatic, unsubstituted -OPh, or an unsubstituted 5-6-membered saturated, partially unsaturated, or aryl ring having 0-4 heteroatoms independently selected from nitrogen, oxygen, or sulfur, or, two independent occurrences of R^{†}, taken together with their intervening atom(s) form an unsubstituted 3-12-membered saturated, partially unsaturated, or aryl mono- or bicyclic ring having 0-4 heteroatoms independently selected from nitrogen, oxygen, or sulfur; wherein when R^{†} is C₁₋₆ aliphatic, R^{†} is optionally substituted with halogen, - R^{•}, -(haloR^{•}), -OH, -OR^{•}, -O(haloR^{•}), -CN, -C(O)OH, -C(O)OR^{•}, -NH₂, - NHR^{•}, -NR^{•}₂, or -NO₂, wherein each R^{•} is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens. It is understood that "Ph" means phenyl; and "halo" means halogen.

In the context of the present invention the term "derivative" means any nontoxic salt, ester, salt of an ester or other derivative of a compound of this invention that, upon administration to a recipient, is capable of providing, either directly or indirectly, a compound of this invention or an inhibitorily active metabolite or residue thereof.

The compounds of the present invention can be in the form of a prodrug compound. "Prodrugs" and "prodrug compound" mean a derivative that is converted into a biologically active compound according to the present invention under physiological conditions in the living body, e.g., by oxidation, reduction, hydrolysis or the like, each of which is carried out enzymatically, or without enzyme involvement. Examples of prodrugs are compounds, in which the amino group in a compound of the present invention is acylated, alkylated or phosphorylated, e.g., eicosanoylamino, alanylamino, pivaloyl-oxymethylamino or in which the hydroxyl group is acylated, alkylated, phosphorylated or converted into the borate, e.g. acetyloxy, palmitoyloxy, pivaloyloxy, succinyloxy, fumaryloxy, alanyloxy or in which the carboxyl group is esterified or amidated, or in which a sulfhydryl group forms a disulfide bridge with a carrier molecule, e.g. a peptide, that delivers the drug selectively to a target and/or to the cytosol of a cell. These compounds can be produced from compounds of the present invention according to well-known methods. Other examples of prodrugs are compounds, wherein the carboxylate in a compound of the present invention is for example converted into an alkyl-, aryl-, choline-, amino-, acyloxymethylester, linolenoyl-ester.

The term "solvates" means addition forms of the compounds of the present invention with solvents, preferably pharmaceutically acceptable solvents that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, e.g. a mono- or dihydrate. If the solvent is alcohol, the solvate formed is an alcoholate, e.g., a methanolate or ethanolate. If the solvent is an ether, the solvate formed is an etherate, e.g., diethyl etherate.

The term "N-oxides" means such compounds of the present invention that contain an amine oxide moiety, i.e. the oxide of a tertiary amine group.

The compounds of formula I-a, I-b or I-c may have one or more centres of chirality. They may accordingly occur in various enantiomeric and diastereomeric forms, as the case may be, and be in racemic or optically active form. The invention, therefore, also relates to the optically active forms, enantiomers, racemates, diastereomers, mixtures thereof in all ratios, collectively: "stereoisomers" for the purpose of the present invention, of these compounds. Since the pharmaceutical activity of the racemates or stereoisomers of the compounds according to the invention may differ, it may be desirable to use a specific stereoisomer, e.g. one specific enantiomer or diastereomer. In these cases, a compound according to the present invention obtained as a racemate - or even intermediates thereof - may be separated into the stereoisomeric (enantiomeric, diastereoisomeric) compounds by chemical or physical measures known to the person skilled in the art. Another approach that may be applied to obtain one or more specific stereoisomers of a compound of the present invention in an enriched or pure form makes use of stereoselective synthetic procedures, e.g. applying starting material in a stereoisomerically enriched or pure form (for instance using the pure or enriched (R)- or (S)-enantiomer of a particular starting material bearing a chiral center) or utilizing chiral reagents or catalysts, in particular enzymes. In the context of the present invention the term "pure enantiomer" usually refers to a relative purity of one enantiomer over the other (its antipode) of equal to or greater than 95%, preferably ≥ 98 %, more preferably ≥ 98.5%, still more preferably ≥ 99%.

Thus, for example, the compounds of the invention which have one or more centers of chirality and which occur as racemates or as mixtures of enatiomers or diastereoisomers can be fractionated or resolved by methods known per se into their optically pure or enriched isomers, i.e. enantiomers or diastereomers. The separation of the compounds of the invention can take place by chromatographic methods, e.g. column separation on chiral or nonchiral phases, or by recrystallization from an optionally optically active solvent or by use of an optically active acid or base or by derivatization with an optically active reagent such as, for example, an optically active alcohol, and subsequent elimination of the radical.

In the context of the present invention the term "tautomer" refers to compounds of the present invention that may exist in tautomeric forms and show tautomerism; for instance, carbonyl compounds may be present in their keto and/or their enol form and show keto-enol tautomerism. Those tautomers may occur in their individual forms, e.g., the keto or the enol form, or as mixtures thereof and are claimed separately and together as mixtures in any ratio. The same applies for cis/trans isomers, E/Z isomers, conformers and the like.

In one embodiment the compounds of the present invention are in the form of free base or acid - as the case may be -, i.e. in their non-salt (or salt-free) form. In another embodiment the compounds of the present invention are in the form of a pharmaceutically acceptable salt, a pharmaceutically acceptable solvate, or a pharmaceutically acceptable solvate of a pharmaceutically acceptable salt.

The term "pharmaceutically acceptable salts" refers to salts prepared from pharmaceutically acceptable bases or acids, including inorganic bases or acids and organic bases or acids. In cases where the compounds of the present invention contain one or more acidic or basic groups, the invention also comprises their corresponding pharmaceutically acceptable salts. Thus, the compounds of the present invention which contain acidic groups, such as carboxyl groups, can be present in salt form, and can be used according to the invention, for example, as alkali metal salts, alkaline earth metal salts, aluminium salts or as ammonium salts. More precise examples of such salts include lithium salts, sodium salts, potassium salts, calcium salts, magnesium salts, barium salts or salts with ammonia or organic amines such as, for example, ethylamine, ethanolamine, diethanolamine, triethanolamine, piperdine, N-methylglutamine or amino acids. These salts are readily available, for instance, by reacting the compound having an acidic group with a suitable base, e.g. lithium hydroxide, sodium hydroxide, sodium propoxide, potassium hydroxide, potassium ethoxide, magnesium hydroxide, calcium hydroxide or barium hydroxide. Other base salts of compounds of the present invention include but are not limited to copper(I), copper(II), iron(II), iron (III), manganese(II) and zinc salts. Compounds of the present invention which contain one or more basic groups, e.g. groups which can be protonated, can be present in salt form, and can be used according to the invention in the form of their addition salts with inorganic or organic acids. Examples of suitable acids include hydrogen chloride, hydrogen bromide, hydrogen iodide, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, embonic acid, mandelic acid, sulfaminic acid, phenylpropionic acid, gluconic acid, ascorbic acid, isonicotinic acid, citric acid, adipic acid, taurocholic acid, glutaric acid, stearic acid, glutamic acid or aspartic acid, and other acids known to the person skilled in the art. The salts which are formed are, inter alia, hydrochlorides, chlorides, hydrobromides, bromides, iodides, sulfates, phosphates, methanesulfonates (mesylates), tosylates, carbonates, bicarbonates, formates, acetates, sulfoacetates, triflates, oxalates, malonates, maleates, succinates, tartrates, malates, embonates, mandelates, fumarates, lactates, citrates, glutarates, stearates, aspartates and glutamates. The stoichiometry of the salts formed from the compounds of the invention may moreover be an integral or non-integral multiple of one.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

If the compounds of the present invention simultaneously contain acidic and basic groups in the molecule, the invention also includes, in addition to the salt forms mentioned, inner salts or betaines (zwitterions). The respective salts can be obtained by customary methods which are known to a person skilled in the art, for example by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Therefore, the following items are also in accordance with the invention:
(a) all stereoisomers or tautomers of the compounds, including mixtures thereof in all ratios;
(b) pharmaceutically acceptable salts of the compounds and of the items mentioned under (a);
(c) pharmaceutically acceptable solvates of the compounds and of the items mentioned under (a), and (b);
(d) N-oxides of the compounds and of the items mentioned under (a), (b), and (c).

It should be understood that all references to compounds above and below are meant to include these items, in particular pharmaceutically acceptable solvates of the compounds, or pharmaceutically acceptable solvates of their pharmaceutically acceptable salts.

There is furthermore intended that a compound of the present invention includes isotope-labelled forms thereof. An isotope-labelled form of a compound of the formula I-a, I-b or I-c is identical to this compound apart from the fact that one or more atoms of the compound have been replaced by an atom or atoms having an atomic mass or mass number which differs from the atomic mass or mass number of the atom which usually occurs naturally. Examples of isotopes which are readily commercially available and which can be incorporated into a compound of the present invention by well-known methods include isotopes of hydrogen, carbon, nitrogen, oxygen, phosphorus, sulfur, fluorine and chlorine, for example ²H, ³H, ¹³C, ¹⁴C, ¹⁵N, ¹⁸O, ¹⁷O, 31P, ³²P, ³³S, ³⁴S, ³⁵S, ³⁶S, ¹⁸F and ³⁶Cl, respectively. A compound of formula I-a, I-b or I-c or a pharmaceutically acceptable salt therof which contains one or more of the above-mentioned isotopes and/or other isotopes of other atoms is intended to be part of the present invention. An isotope-labelled compound of formula I-a, I-b or I-c can be used in a number of beneficial ways. For example, an isotope-labelled compound of the present invention into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to simple preparation and excellent detectability. Incorporation of heavier isotopes, for example deuterium (²H), into a compound of formula I-a, I-b or I-c has therapeutic advantages owing to the higher metabolic stability of this isotope-labelled compound. Higher metabolic stability translates directly into an increased in vivo half-life or lower dosages, which under most circumstances would represent a preferred embodiment of the present invention. An isotope-labelled compound of formula I-a, I-b or I-c can usually be prepared by carrying out the procedures disclosed in the synthesis schemes and the related description, in the example part and in the preparation part in the present text, replacing a non-isotope-labelled reactant by a readily available isotope-labelled reactant.

Deuterium (²H) can also be incorporated into a compound of formula I-a, I-b or I-c for the purpose of manipulating the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus cause a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M}/k_{D} = 2-7 are typical. If this rate difference is successfully applied to a compound of the formula la and Ib that is susceptible to oxidation, the profile of this compound in vivo can be drastically modified and result in improved pharmacokinetic properties.

When discovering and developing therapeutic agents, the person skilled in the art attempts to optimise pharmacokinetic parameters while retaining desirable in vitro properties. It is reasonable to assume that many compounds with poor pharmacokinetic profiles are susceptible to oxidative metabolism. In vitro liver microsomal assays currently available provide valuable information on the course of oxidative metabolism of this type, which in turn permits the rational design of deuterated compounds of the formula I-a, I-b or I-c with improved stability through resistance to such oxidative meta-bolism. Significant improvements in the pharmacokinetic profiles of compounds of the formula I-a, I-b or I-c are thereby obtained, and can be expressed quantitatively in terms of increases in the in vivo half-life (t1/2), concentration at maximum therapeutic effect (Cₘₐₓ), area under the dose response curve (AUC), and F; and in terms of reduced clearance, dose and materials costs.

The following is intended to illustrate the above: a compound of formula I-a, I-b or I-c which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent of the extent to which the improvement in resistance to oxidative metabolism has improved. In this way, it is deter-mined that the half-life of the parent compound can be extended by up to 100% as the result of deuterium-hydrogen exchange of this type.

Deuterium-hydrogen exchange in a compound of the present invention can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the unwanted metabolite, even if the particular oxidation is not a rate-determining step. Further information on the state of the art with respect to deuterium-hydrogen exchange may be found, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al, Biochemistry 33(10) 2927-2937, 1994, and Jarman et al. Carcinogenesis 16(4), 683-688, 1995.

Furthermore, the present invention relates to pharmaceutical compositions comprising at least one compound of formula I-a, I-b or I-c, or its derivatives, prodrugs, solvates, tautomers or stereoisomers thereof as well as the pharmaceutically acceptable salts of each of the foregoing, including mixtures thereof in all ratios, as active ingredient, together with a pharmaceutically acceptable carrier.

For the purpose of the present invention the term "pharmaceutical composition" (or "pharmaceutical formulation") refers to a composition or product comprising one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable carrier. It may further comprise physiologically acceptable excipients, auxiliaries, adjuvants, diluents and/or additional pharmaceutically active substance other than the compounds of the invention.

The pharmaceutical compositions include compositions and pharmaceutical formulations suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

A pharmaceutical composition of the present invention may additionally comprise one or more other compounds as active ingredients (drugs), such as one or more additional compounds of the present invention. In a particular embodiment the pharmaceutical composition further comprises a second active ingredient or its derivatives, prodrugs, solvates, tautomers or stereoisomers thereof as well as the pharmaceutically acceptable salts of each of the foregoing, including mixtures thereof in all ratios, wherein that second active ingredient is other than a compound of formula I-a, I-b or I-c; preferably, that second active ingredient is a compound that is useful in the treatment, prevention, suppression and/or amelioration of medicinal conditions or pathologies for which the compounds of the present invention are useful as well and which are listed elsewhere hereinbefore or hereinafter. Such combination of two or more active ingredients or drugs may be safer or more effective than either drug or active ingredient alone, or the combination is safer or more effective than it would be expected based on the additive properties of the individual drugs. Such other drug(s) may be administered, by a route and in an amount commonly used contemporaneously or sequentially with a compound of the invention. When a compound of the invention is used contemporaneously with one or more other drugs or active ingredients, a combination product containing such other drug(s) and the compound of the invention - also referred to as "fixed dose combination" - is preferred. However, combination therapy also includes therapies in which the compound of the present invention and one or more other drugs are administered on different overlapping schedules. It is contemplated that when used in combination with other active ingredients, the compound of the present invention or the other active ingredient or both may be used effectively in lower doses than when each is used alone. Accordingly, the pharmaceutical compositions of the present invention include those that contain one or more other active ingredients, in addition to a compound of the invention.

The compounds of the present invention - or N-oxides, solvates, tautomers or stereoisomers thereof and/or the pharmaceutically acceptable salts of each of the foregoing, including mixtures thereof in all ratios - can be used as medicaments. They have been found to exhibit pharmacological activity by inhibiting acetyl-CoA synthetase 2 (ACSS2). Furthermore, when tested in an MNT (micronuclei) in-vitro assay on genotoxicity, they may exhibit improved properties, i.e. a negative MNT assay read-out, in comparison to prior art ACSS2 inhibitors.

Thus, the compounds of the present invention being ACSS2 inhibitors are useful in particular in the treatment, prevention, suppression and/or amelioration of hyperproliferative disorders and cancer, in particular tumors including solid tumors, of bladder, breast, colorectal, colon, kidney, liver, lung, head and neck, esophagus, bladder, gall bladder, ovary, pancreas, stomach, cervix, thyroid, prostate, and skin, including squamous cell carcinoma; leukemia, acute lymphocytic leukemia, acute lymphoblastic leukemia, B-cell lymphoma, T- cell lymphoma, Hodgkins lymphoma, non-Hodgkins lymphoma, hairy cell lymphoma, mantle cell lymphoma, myeloma and Burkett's lymphoma; chronic lymphocytic leukemia ("CLL"),acute and chronic myelogenous leukemia, myelodysplastic syndrome and promyelocyte leukemia; fibrosarcoma, rhabdomyosarcoma; mantle cell lymphoma, myeloma; astrocytoma, neuroblastoma, glioma, glioblastoma, malignant glial tumors, astrocytoma, hepatocellular carcinoma, gastrointestinal stromal tumors ("GIST") and schwannomas; melanoma, multiple myeloma, seminoma, teratocarcinoma, osteosarcoma, xenoderma pigmentosum, keratoctanthoma, thyroid follicular cancer, endometrial cancer, gastrointestinal tract cancer and Kaposi's sarcoma; acanthoma, acinic cell carcinoma, acoustic neuroma, acral lentiginous melanoma, acrospiroma, acute eosinophilic leukemia, acute lymphoblastic leukemia, acute megakaryoblastic leukemia, acute monocytic leukemia, acute myeloblastic leukemia with maturation, acute myeloid dendritic cell leukemia, acute myeloid leukemia, acute promyelocytic leukemia, adamantinoma, adenocarcinoma, adenoid cystic carcinoma, adenoma, adenomatoid odontogenic tumor, adrenocortical carcinoma, adult T-cell leukemia, aggressive NK-cell leukemia, AIDS-related cancers, AIDS-related lymphoma, alveolar soft part sarcoma, ameloblastic fibroma, anal cancer, anaplastic large cell lymphoma, anaplastic thyroid cancer, angioimmunoblastic T-cell lymphoma, angiomyolipoma, angiosarcoma, appendix cancer, astrocytoma, atypical teratoid rhabdoid tumor, basal cell carcinoma, basallike carcinoma, B-cell leukemia, B-cell lymphoma, bellini duct carcinoma, biliary tract cancer, bladder cancer, blastoma, bone cancer, bone tumor, brain stem glioma, brain tumor, breast cancer, brenner tumor, bronchial tumor, bronchioloalveolar carcinoma, brown tumor, Burkitt's lymphoma, carcinoid tumor, carcinoma, carcinosarcoma, Castleman's disease, central nervous system embryonal tumor, cerebellar astrocytoma, cerebral astrocytoma, cervical cancer, cholangiocarcinoma, chondroma, chondrosarcoma, chordoma, choriocarcinoma, choroid plexus papilloma, chronic lymphocytic leukemia, chronic monocytic leukemia, chronic myelogenous leukemia, chronic myeloproliferative disorder, chronic neutrophilic leukemia, clear cell renal cell carcinoma, clear-cell tumor, colon cancer, colorectal cancer, craniopharyngioma, cutaneous T-cell lymphoma, dermatofibrosarcoma protuberans, dermoid cyst, desmoplastic small round cell tumor, diffuse large B cell lymphoma, dysembryoplastic neuroepithelial tumor, embryonal carcinoma, endodermal sinus tumor, endometrial cancer, endometrial uterine cancer, endometrioid tumor, enteropathyassociated T-cell lymphoma, ependymoblastoma, ependymoma, epithelioid sarcoma, erythroleukemia, esophageal cancer, esthesioneuroblastoma, Ewing's sarcoma, extracranial germ cell tumor, extragonadal germ cell tumor, extrahepatic bile duct cancer, extramammary Paget's disease, fallopian tube cancer, fibroma, fibrosarcoma, follicular lymphoma, follicular thyroid cancer, gallbladder cancer, ganglioglioma, ganglioneuroma, gastric cancer, gastric lymphoma, gastrointestinal cancer, gastrointestinal carcinoid tumor, gastrointestinal stromal tumor, germ cell tumor, germinoma, gestational choriocarcinoma, gestational trophoblastic tumor, giant cell tumor of bone, glioblastoma multiforme, glioma, gliomatosis cerebri, glomus tumor, glucagonoma, gonadoblastoma, granulosa cell tumor, hairy cell leukemia, head and neck cancer, heart cancer, hemangioblastoma, hemangiopericytoma, hemangiosarcoma, hematological malignancy, hepatocellular carcinoma, hepatosplenic T-cell lymphoma, Hodgkin lymphoma, hypopharyngeal cancer, hypothalamic glioma, inflammatory breast cancer, intraocular melanoma, islet cell carcinoma, juvenile myelomonocytic leukemia, Kaposi's sarcoma, kidney cancer, klatskin tumor, krukenberg tumor, laryngeal cancer, lentigo maligna melanoma, leukemia, lip and oral cavity cancer, liposarcoma, lung cancer, luteoma, lymphangioma, lymphangiosarcoma, lymphoepithelioma, lymphoid leukemia, lymphoma, macroglobulinemia, malignant fibrous histiocytoma, malignant glioma, malignant mesothelioma, malignant peripheral nerve sheath tumor, malignant rhabdoid tumor, malignant triton tumor, malt lymphoma, mantle cell lymphoma, mast cell leukemia, mediastinal germ cell tumor, mediastinal tumor, medullary thyroid cancer, medulloblastoma, medulloepithelioma, melanoma, meningioma, merkel cell carcinoma, mesothelioma, metastatic squamous neck cancer with occult primary, metastatic urothelial carcinoma, mixed mullerian tumor, monocytic leukemia, mouth cancer, mucinous tumor, multiple endocrine neoplasia syndrome, multiple myeloma, mycosis fungoides, myelodysplastic disease, myeloid leukemia, myeloid sarcoma, myeloproliferative disease, myxoma, nasal cavity cancer, nasopharyngeal cancer, neoplasm, neurinoma, neuroblastoma, neurofibroma, neuroma, nodular melanoma, non-Hodgkin lymphoma, nonmelanoma skin cancer, non-small cell lung cancer, ocular oncology, oligoastrocytoma, oligodendroglioma, oncocytoma, optic nerve sheath meningioma, oral cancer, oropharyngeal cancer, osteosarcoma, ovarian cancer, ovarian epithelial cancer, ovarian germ cell tumor, ovarian low malignant potential tumor, pancoast tumor, pancreatic cancer, papillary thyroid cancer, papillomatosis, paraganglioma, paranasal sinus cancer, parathyroid cancer, penile cancer, perivascular epithelioid cell tumor, pharyngeal cancer, pheochromocytoma, pineal parenchymal tumor of intermediate differentiation, pineoblastoma, pituicytoma, pituitary adenoma, pituitary tumor, plasma cell neoplasm, pleuropulmonary blastoma, polyembryoma, precursor T-lymphoblastic lymphoma, primitive neuroectodermal tumor, prostate cancer, pseudomyxoma peritonei, rectal cancer, renal cell carcinoma, retinoblastoma, rhabdomyoma, rhabdomyosarcoma, Richter's transformation, sacrococcygeal teratoma, salivary gland cancer, sarcoma, schwannomatosis, sebaceous gland carcinoma, secondary neoplasm, seminoma, serous tumor, Sertoli-Leydig cell tumor, sex cord-stromal tumor, sezary syndrome, signet ring cell carcinoma, skin cancer, small blue round cell tumor, small cell carcinoma, small cell lung cancer, small cell lymphoma, small intestine cancer, soft tissue sarcoma, somatostatinoma, soot wart, spinal tumor, splenic marginal zone lymphoma, squamous cell carcinoma, stomach cancer, superficial spreading melanoma, supratentorial primitive neuroectodermal tumor, surface epithelial-stromal tumor, synovial sarcoma, T-cell acute lymphoblastic leukemia, T-cell large granular lymphocyte leukemia, T-cell leukemia, T-cell lymphoma, T-cell prolymphocytic leukemia, teratoma, terminal lymphatic cancer, testicular cancer, thecoma, throat cancer, thymic carcinoma, thymoma, thyroid cancer, transitional cell cancer of renal pelvis and ureter, transitional cell carcinoma, urachal cancer, urethral cancer, urogenital neoplasm, uterine sarcoma, uveal melanoma, vaginal cancer, verner morrison syndrome, verrucous carcinoma, visual pathway glioma, vulvar cancer, Waldenstrom's macroglobulinemia, Warthin's tumor, Wilms' tumor or any combination thereof. However, since activity of ACSS2 plays a role in acetyl-CoA synthesis in normal, i.e. non-cancer cells too, the compounds of the present invention are useful also in the the treatment, prevention, suppression and/or amelioration of an inflammatory disorder or disease, in particular Crohn's disease, ulcerative colitis, idiopathic pulmonary fibrosis, muscular dystrophy, rheumatoid arthritis, and systemic sclerosis (scleroderma); a neurogenerative disorder or disease, in particular Huntington's disease; Lipid metabolism disorders, e.g. NASH (non-alcoholic steatohepatitis), NAFLD (non-alcoholic fatty liver disease) , fatty liver disease; viral infections, e.g. with cytomegalovirus; post-traumatic stress disorder (PTSD); bipolar disorder, depression, Tourettes's Syndrome, schizophrenia, obsessive-compulsive disorder, anxiety disorder, panic disorders, phobias, addiction to e.g. alcohol, tobacco, opioids, sedatives, hypnotics, anxiolytics, cocaine, cannabis, amphetamines, hallucinogens, inhalants, phencyclidine, impulse control disorders, behavioral addictions.

In a particular embodiment the compounds of the present invention are for use in the prevention and/or treatment, especially in the treatment of any of the disorders or diseases listed above, preferably of cancer, in particular tumors including solid tumors, of the specific types of cancer disclosed in the previous paragraph; of an inflammatory disorder or disease, in particular Crohn's disease, ulcerative colitis, idiopathic pulmonary fibrosis, muscular dystrophy, rheumatoid arthritis, and systemic sclerosis (scleroderma); a neurogenerative disorder or disease, in particular Huntington's disease; Lipid metabolism disorders, e.g. NASH (non-alcoholic steatohepatitis), NAFLD (non-alcoholic fatty liver disease), fatty liver disease; viral infections, e.g. with cytomegalovirus; post-traumatic stress disorder (PTSD); bipolar disorder, depression, Tourettes's Syndrome, schizophrenia, obsessive-compulsive disorder, anxiety disorder, panic disorders, phobias, addiction to e.g. alcohol, tobacco, opioids, sedatives, hypnotics, anxiolytics, cocaine, cannabis, amphetamines, hallucinogens, inhalants, phencyclidine, impulse control disorders, behavioral addictions.

Another particular embodiment of the present invention is a compound for use in a method for preventing and/or treating, preferably treating a disorder or disease selected from the group consisting of hyperproliferative disorders and cancer, in particular tumors including solid tumors, of the specific types of cancer disclosed in the previous paragraphs; of an inflammatory disorder or disease, in particular Crohn's disease, ulcerative colitis, idiopathic pulmonary fibrosis, muscular dystrophy, rheumatoid arthritis, and systemic sclerosis (scleroderma); a neurogenerative disorder or disease, in particular Huntington's disease; Lipid metabolism disorders, e.g. NASH (non-alcoholic steatohepatitis), NAFLD (non-alcoholic fatty liver disease) , fatty liver disease; viral infections, e.g. with cytomegalovirus; post-traumatic stress disorder (PTSD); bipolar disorder, depression, Tourettes's Syndrome, schizophrenia, obsessive-compulsive disorder, anxiety disorder, panic disorders, phobias, addiction to e.g. alcohol, tobacco, opioids, sedatives, hypnotics, anxiolytics, cocaine, cannabis, amphetamines, hallucinogens, inhalants, phencyclidine, impulse control disorders, behavioral addictions.

Still another particular embodiment of the invention is the use of a compound of the present invention - or N-oxides, solvates tautomers or stereoisomers thereof and/or the pharmaceutically acceptable salts of each of the foregoing, including mixtures thereof in all ratios - for the manufacturing of a medicament, in particular for preventing and/or treating, preferably treating a disorder or disease selected from the group consisting of hyperproliferative disorders and cancer, in particular tumors including solid tumors, of the specific types of cancer disclosed in the previous paragraphs; of an inflammatory disorder or disease, in particular Crohn's disease, ulcerative colitis, idiopathic pulmonary fibrosis, muscular dystrophy, rheumatoid arthritis, and systemic sclerosis (scleroderma); a neurogenerative disorder or disease, in particular Huntington's disease; Lipid metabolism disorders, e.g. NASH (non-alcoholic steatohepatitis), NAFLD (non-alcoholic fatty liver disease) , fatty liver disease; viral infections, e.g. with cytomegalovirus; post-traumatic stress disorder (PTSD); bipolar disorder, depression, Tourettes's Syndrome, schizophrenia, obsessive-compulsive disorder, anxiety disorder, panic disorders, phobias, addiction to e.g. alcohol, tobacco, opioids, sedatives, hypnotics, anxiolytics, cocaine, cannabis, amphetamines, hallucinogens, inhalants, phencyclidine, impulse control disorders, behavioral addictions.

Preferably, the present invention relates to a compound of the present invention for use in the prevention and/or treatment of a disease - or, alternatively, a method for preventing and/or treating a disease by administering an effective amount of a compound of the present invention; or, in another alternative, a use of a compound of the present invention for the manufacturing of a medicament for the prevention and/or treatment of a disease - wherein that disease is a cancer, in particular tumors including solid tumors, of the specific types of cancer disclosed in the previous paragraphs; and more preferably, wherein administration of the compound is simultaneous, sequential or in alternation with administration of at least one other active drug agent.

The disclosed compounds of formulas I-a, I-b and I-c can be administered in combination with other known therapeutic agents, including anticancer agents. As used herein, the term "anticancer agent" relates to any agent which is administered to a patient with cancer for the purposes of treating the cancer. The anti-cancer treatment defined above may be applied as a monotherapy or may involve, in addition to the herein disclosed compounds of formulas I-a, I-b and I-c, conventional surgery or radiotherapy or medicinal therapy. Such medicinal therapy, e.g. a chemotherapy or a targeted therapy, may include one or more, but preferably one, of the following anti-tumor agents:

### Alkylating agents

such as altretamine, bendamustine, busulfan, carmustine, chlorambucil, chlormethine, cyclophosphamide, dacarbazine, ifosfamide, improsulfan, tosilate, lomustine, melphalan, mitobronitol, mitolactol, nimustine, ranimustine, temozolomide, thiotepa, treosulfan, mechloretamine, carboquone; apaziquone, fotemustine, glufosfamide, palifosfamide, pipobroman, trofosfamide, uramustine, TH-302⁴, VAL-083⁴;

### Platinum Compounds

such as carboplatin, cisplatin, eptaplatin, miriplatine hydrate, oxaliplatin, lobaplatin, nedaplatin, picoplatin, satraplatin;
lobaplatin, nedaplatin, picoplatin, satraplatin;
DNA altering agents
such as amrubicin, bisantrene, decitabine, mitoxantrone, procarbazine, trabectedin, clofarabine;
amsacrine, brostallicin, pixantrone, laromustine^{1,3};

### Topoisomerase Inhibitors

such as etoposide, irinotecan, razoxane, sobuzoxane, teniposide, topotecan;
amonafide, belotecan, elliptinium acetate, voreloxin;

### Microtubule modifiers

such as cabazitaxel, docetaxel, eribulin, ixabepilone, paclitaxel, vinblastine, vincristine, vinorelbine, vindesine, vinflunine; fosbretabulin, tesetaxel;

### Antimetabolites

such as asparaginase³, azacitidine, calcium levofolinate, capecitabine, cladribine, cytarabine, enocitabine, floxuridine, fludarabine, fluorouracil, gemcitabine, mercaptopurine, methotrexate, nelarabine, pemetrexed, pralatrexate, azathioprine, thioguanine, carmofur; doxifluridine, elacytarabine, raltitrexed, sapacitabine, tegafur^{2,3}, trimetrexate;

### Anticancer antibiotics

such as bleomycin, dactinomycin, doxorubicin, epirubicin, idarubicin, levamisole, miltefosine, mitomycin C, romidepsin, streptozocin, valrubicin, zinostatin, zorubicin, daunurobicin, plicamycin; aclarubicin, peplomycin, pirarubicin;

### Hormones/Antagonists

such as abarelix, abiraterone, bicalutamide, buserelin, calusterone, chlorotrianisene, degarelix, dexamethasone, estradiol, fluocortolone, fluoxymesterone, flutamide, fulvestrant, goserelin, histrelin, leuprorelin, megestrol, mitotane, nafarelin, nandrolone, nilutamide, octreotide, prednisolone, raloxifene, tamoxifen, thyrotropin alfa, toremifene, trilostane, triptorelin, diethylstilbestrol; acolbifene, danazol, deslorelin, epitiostanol, orteronel, enzalutamide^{1,3};

### Aromatase inhibitors

such as aminoglutethimide, anastrozole, exemestane, fadrozole, letrozole, testolactone; formestane;

### Small molecule kinase inhibitors

such as crizotinib, dasatinib, erlotinib, imatinib, lapatinib, nilotinib, pazopanib, regorafenib, ruxolitinib, sorafenib, sunitinib, vandetanib, vemurafenib, bosutinib, gefitinib, axitinib; afatinib, alisertib, dabrafenib, dacomitinib, dinaciclib, dovitinib, enzastaurin, nintedanib, lenvatinib, linifanib, linsitinib, masitinib, midostaurin, motesanib, neratinib, orantinib, perifosine, ponatinib, radotinib, rigosertib, tepotinib, tipifarnib, tivantinib, tivozanib, trametinib, pimasertib, brivanib alaninate, cediranib, apatinib⁴, cabozantinib S-malate^{1,3}, ibrutinib^{1,3}, icotinib⁴, buparlisib², cipatinib⁴, cobimetinib^{1,3}, idelalisib^{1,3}, fedratinib¹, XL-647⁴;

### Photosensitizers

such as methoxsalen³; porfimer sodium, talaporfin, temoporfin;

### Antibodies

such as alemtuzumab, besilesomab, brentuximab vedotin, cetuximab, denosumab, ipilimumab, ofatumumab, panitumumab, rituximab, tositumomab, trastuzumab, bevacizumab, pertuzumab^{2,3}; catumaxomab, elotuzumab, epratuzumab, farletuzumab, mogamulizumab, necitumumab, nimotuzumab, obinutuzumab, ocaratuzumab, oregovomab, ramucirumab, rilotumumab, siltuximab, tocilizumab, zalutumumab, zanolimumab, matuzumab, dalotuzumab^{1,2,3}, onartuzumab^{1,3}, racotumomab¹, tabalumab^{1,3}, EMD-525797⁴, atezolizumab, durvalumab, pembrolizumab, nivolumab^{1,3};

### Cytokines

such as aldesleukin, interferon alfa², interferon alfa2a³, interferon alfa2b^{2,3}; celmoleukin, tasonermin, teceleukin, oprelvekin^{1,3}, recombinant interferon beta-1a⁴;

### Drug Conjugates

such as denileukin diftitox, ibritumomab tiuxetan, iobenguane I123, prednimustine, trastuzumab emtansine, estramustine, gemtuzumab, ozogamicin, aflibercept; cintredekin besudotox, edotreotide, inotuzumab ozogamicin, naptumomab estafenatox, oportuzumab monatox, technetium (99mTc) arcitumomab^{1,3}, vintafolide^{1,3};

### Vaccines

such as sipuleucel³; vitespen³, emepepimut-S³, oncoVAX⁴, rindopepimut³, troVax⁴, MGN-1601⁴, MGN-1703⁴;

### Miscellaneous

alitretinoin, bexarotene, bortezomib, everolimus, ibandronic acid, imiquimod, lenalidomide, lentinan, metirosine, mifamurtide, pamidronic acid, pegaspargase, pentostatin, sipuleucel³, sizofiran, tamibarotene, temsirolimus, thalidomide, tretinoin, vismodegib, zoledronic acid, vorinostat; celecoxib, cilengitide, entinostat, etanidazole, ganetespib, idronoxil, iniparib, ixazomib, lonidamine, nimorazole, panobinostat, peretinoin, plitidepsin, pomalidomide, procodazol, ridaforolimus, tasquinimod, telotristat, thymalfasin, tirapazamine, tosedostat, trabedersen, ubenimex, valspodar, gendicine⁴, picibanil⁴, reolysin⁴, retaspimycin hydrochloride^{1,3}, trebananib^{2,3}, virulizin⁴, carfilzomib^{1,3}, endostatin⁴, immucothel⁴, belinostat³, MGN-1703⁴;

### PARP inhibitors

Olaparib, Veliparib.

### MCT1 inhibitors

AZD3965⁴, BAY-8002⁴.
**¹ Prop. INN** (**P**roposed **I**nternational **N**onproprietary **N**ame)
**² Rec. INN** (**R**ecommended **I**nternational **N**onproprietary **N**ames)
**³ USAN** (**U**nited **S**tates **A**dopted **N**ame)
**⁴ no INN.**

A further embodiment of the present invention is a process for the manufacture of the pharmaceutical compositions of the present invention, characterized in that one or more compounds according to the invention and one or more compounds selected from the group consisting of solid, liquid or semiliquid excipients, auxiliaries, adjuvants, diluents, carriers and pharmaceutically active agents other than the compounds according to the invention, are converted in a suitable dosage form.

In another aspect of the invention, a set or kit is provided comprising a therapeutically effective amount of at least one compound of the invention and/or at least one pharmaceutical composition as described herein and a therapeutically effective amount of at least one further pharmacologically active substance other than the compounds of the invention. It is preferred that this set or kit comprises separate packs of
a) an effective amount of a compound of formula I-a, I-b or I-c, or its solvates, tautomers or stereoisomers thereof as well as the physiologically acceptable salts of each of the foregoing, including mixtures thereof in all ratios, and
b) an effective amount of a further active ingredient that further active ingredient not being a compound of formula I-a, I-b or I-c.

The pharmaceutical compositions (formulations) of the present invention may be administered by any means that achieve their intended purpose. For example, administration may be via oral, parenteral, topical, enteral, intravenous, intramuscular, inhalant, nasal, intraarticular, intraspinal, transtracheal, transocular, subcutaneous, intraperitoneal, transdermal, or buccal routes. Alternatively, or concurrently, administration may be via the oral route. The dosage administered will be dependent upon the age, health, and weight of the recipient, kind of concurrent treatment, if any, frequency of treatment, and the nature of the effect desired. Parenteral administration is preferred. Oral administration is especially preferred.

Suitable dosage forms include, but are not limited to capsules, tablets, pellets, dragees, semi-solids, powders, granules, suppositories, ointments, creams, lotions, inhalants, injections, cataplasms, gels, tapes, eye drops, solution, syrups, aerosols, suspension, emulsion, which can be produced according to methods known in the art, for example as described below:
Tablets: mixing of active ingredient/s and auxiliaries, compression of said mixture into tablets (direct compression), optionally granulation of part of mixture before compression.

Capsules: mixing of active ingredient/s and auxiliaries to obtain a flowable powder, optionally granulating powder, filling powders/granulate into opened capsules, capping of capsules.

Semi-solids (ointments, gels, creams): dissolving/dispersing active ingredient/s in an aqueous or fatty carrier; subsequent mixing of aqueous/fatty phase with complementary fatty/ aqueous phase, homogenization (creams only).

Suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms.

Aerosols: dispersing/dissolving active agent/s in a propellant, bottling said mixture into an atomizer.

In general, non-chemical routes for the production of pharmaceutical compositions and/or pharmaceutical preparations comprise processing steps on suitable mechanical means known in the art that transfer one or more compounds of the invention into a dosage form suitable for administration to a patient in need of such a treatment. Usually, the transfer of one or more compounds of the invention into such a dosage form comprises the addition of one or more compounds, selected from the group consisting of carriers, excipients, auxiliaries and pharmaceutical active ingredients other than the compounds of the invention. Suitable processing steps include, but are not limited to combining, milling, mixing, granulating, dissolving, dispersing, homogenizing, casting and/or compressing the respective active and nonactive ingredients. Mechanical means for performing said processing steps are known in the art, for example from Ullmann's Encyclopedia of Industrial Chemistry, 5th Edition. In this respect, active ingredients are preferably at least one compound of the invention and optionally one or more additional compounds other than the compounds of the invention, which show valuable pharmaceutical properties, preferably those pharmaceutical active agents other than the compounds of the invention, which are disclosed herein.

Particularly suitable for oral use are tablets, pills, coated tablets, capsules, powders, granules, syrups, juices or drops, suitable for rectal use are suppositories, suitable for parenteral use are solutions, preferably oil-based or aqueous solutions, furthermore suspensions, emulsions or implants, and suitable for topical use are ointments, creams or powders. The compounds of the invention may also be lyophilised and the resultant lyophilisates used, for example, for the preparation of injection preparations. The preparations indicated may be sterilised and/or comprise assistants, such as lubricants, preservatives, stabilisers and/or wetting agents, emulsifiers, salts for modifying the osmotic pressure, buffer substances, dyes, flavours and/or a plurality of further active ingredients, for example one or more vitamins.

Suitable excipients are organic or inorganic substances, which are suitable for enteral (for example oral), parenteral or topical administration and do not react with the compounds of the invention, for example water, vegetable oils, benzyl alcohols, alkylene glycols, polyethylene glycols, glycerol triacetate, gelatine, carbohydrates, such as lactose, sucrose, mannitol, sorbitol or starch (maize starch, wheat starch, rice starch, potato starch), cellulose preparations and/or calcium phosphates, for example tricalcium phosphate or calcium hydrogen phosphate, magnesium stearate, talc, gelatine, tragacanth, methyl cellulose, hydroxypropylmethylcellulose, sodium carboxymethylcellulose, polyvinyl pyrrolidone and/or vaseline.

If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries include, without limitation, flow-regulating agents and lubricants, for example, silica, talc, stearic acid or salts thereof, such as magnesium stearate or calcium stearate, and/or polyethylene glycol. Dragee cores are provided with suitable coatings, which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices or to provide a dosage form affording the advantage of prolonged action, the tablet, dragee or pill can comprise an inner dosage and an outer dosage component the latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol, solutions of suitable cellulose preparations such as acetyl-cellulose phthalate, cellulose acetate or hydroxypropylmethyl-cellulose phthalate, are used. Dye stuffs or pigments may be added to the tablets or dragee coatings, for example, for identification or in order to characterize combinations of active compound doses.

Suitable carrier substances are organic or inorganic substances which are suitable for enteral (e.g. oral) or parenteral administration or topical application and do not react with the novel compounds, for example water, vegetable oils, benzyl alcohols, polyethylene glycols, gelatin, carbohydrates such as lactose or starch, magnesium stearate, talc and petroleum jelly. In particular, tablets, coated tablets, capsules, syrups, suspensions, drops or suppositories are used for enteral administration, solutions, preferably oily or aqueous solutions, furthermore suspensions, emulsions or implants, are used for parenteral administration, and ointments, creams or powders are used for topical application. The compounds of the invention can also be lyophilized and the lyophilizates obtained can be used, for example, for the production of injection preparations.

Other pharmaceutical preparations, which can be used orally include push-fit capsules made of gelatine, as well as soft, sealed capsules made of gelatine and a plasticizer such as glycerol or sorbitol. The push-fit capsules can contain the active compounds in the form of granules, which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatine.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400).

Aqueous injection suspensions may contain substances, which increase the viscosity of the suspension, including, for example, sodium carboxymethyl cellulose, sorbitol, and/or dextran, optionally, the suspension may also contain stabilizers.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

Possible pharmaceutical preparations, which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatine rectal capsules, which consist of a combination of the active compounds with a base. Possible base materials include, for example, liquid triglycerides, polyethylene glycols, or paraffin hydrocarbons.

For use in medicine, the compounds of the present invention may be in the form of pharmaceutically acceptable salts. Other salts may, however, be useful in the preparation of the compounds of the invention or of their pharmaceutically acceptable salts. Suitable pharmaceutically acceptable salts of the compounds of this invention are those described hereinbefore and include acid addition salts which may, for example be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic bases, e.g. quaternary ammonium salts.

The pharmaceutical preparations can be employed as medicaments in human and veterinary medicine. As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. Said therapeutic effective amount of one or more of the compounds of the invention is known to the skilled artisan or can be easily determined by standard methods known in the art.

The compounds of the present invention and the optional additional active substances are generally administered analogously to commercial preparations. Usually, suitable doses that are therapeutically effective lie in the range between 0.0005 mg and 1000 mg, preferably between 0.005 mg and 500 mg and especially between 0.5 mg and 100 mg per dose unit. The daily dose is preferably between about 0.001 mg/kg and 10 mg/kg of body weight.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

The specific dose for the individual patient, in particular for the individual human patient, depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician, which advises or attends the therapeutic treatment.

The compounds of the present invention can be prepared according to the procedures of the following Schemes and Examples, using appropriate materials, and as further exemplified by the following specific examples. They may also be prepared by methods known per se, as described in the literature (for example in standard works, such as Houben-Weyl, Methoden der Organischen Chemie [Methods of Organic Chemistry], Georg Thieme Verlag, Stuttgart; Organic Reactions, John Wiley & Sons, Inc., New York), to be precise under reaction conditions which are known and suitable for the said reactions. Use can also be made of variants which are known per se, but are not mentioned here in greater detail.

Likewise, the starting materials for the preparation of compounds of the present invention can be prepared by methods as described in the examples or by methods known per se, as described in the literature of synthetic organic chemistry and known to the skilled person, or can be obtained commercially. The starting materials for the processes claimed and/or utilized may, if desired, also be formed in situ by not isolating them from the reaction mixture, but instead immediately converting them further into the compounds of the invention or intermediate compounds. On the other hand, in general it is possible to carry out the reaction stepwise.

Preferably, the reaction of the compounds is carried out in the presence of a suitable solvent, which is preferably inert under the respective reaction conditions. Examples of suitable solvents comprise but are not limited to hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methyl pyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents or mixtures with water.

The reaction temperature is between about -100° C and 300° C, depending on the reaction step and the conditions used.

Reaction times are generally in the range between a fraction of a minute and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range between 10 minutes and 48 hours.

Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The present invention also refers to a process for manufacturing a compound of formula I-a, I-b or I-c in its most general form as well as any of the particular embodiments, PE1, PE2, PE3, PE3a, PE3b, PE4, PE5, PE5a, PE5b, PE5c, PE5d, PE6, PE6a, PE6b, PE7, PE7a, PE7b, PE7c, PE8, PE8a, PE8b, PE8c, PE9, PE9a, PE9a, PE9b, PE9c, PE10, described herein, or derivatives, N-oxides, prodrugs, solvates, tautomers or stereoisomers thereof as well as the pharmaceutically acceptable salts of each of the foregoing, the process being characterized in that
(a) in case of an amide-1H-imidazo[4.5-b]pyridine derivative of formula I-a the carboxylic acid of general formula II-a 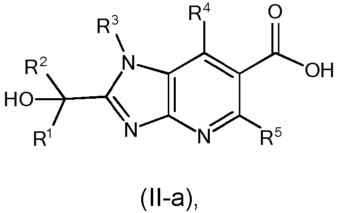
   wherein R¹, R², R³, R⁴ and R⁵ are as described for formula I-a above and in the accompanying claims,
   is subjected to an amidation reaction with a compound of formula III:

      R⁶-NH₂ (III)
   wherein R⁶ is as defined above and in the accompanying claims, optionally in the presence of a suitable catalyst, e.g. triethylamine and HATU ([dimethylamino-([1,2,3]triazolo[4,5-b]pyridin-3-yloxy)-methylene]-dimethylammonium hexafluoro phosphate) to yield the amide derivative of formula I-a: 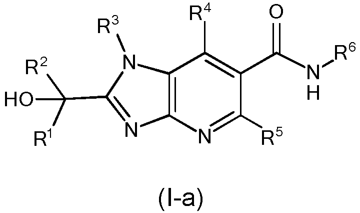
   wherein R¹, R², R³, R⁴, R⁵ and R⁶ are as described for formula I-a above and in the accompanying claims;
   or
(b) in case of an amide-1-pyrazolo[1,5-a]pyridine derivative of formula I-b the carboxylic acid of general formula II-b 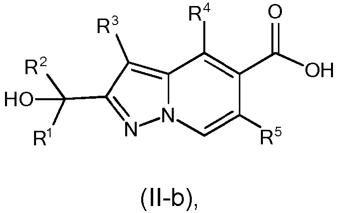
   wherein R¹, R², R³, R⁴ and R⁵ are as described for formula I-b above and in the accompanying claims,
   is subjected to an amidation reaction with a compound of formula III:

      R⁶-NH₂ (III)
   wherein R⁶ is as defined above and in the accompanying claims, optionally in the presence of a suitable catalyst, e.g. triethylamine and HATU ([dimethylamino-([1,2,3]triazolo[4,5-b]pyridin-3-yloxy)-methylene]-dimethylammonium hexafluoro phosphate) to yield the amide derivative of formula I-b: 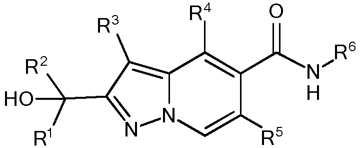 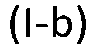
   wherein R¹, R², R³, R⁴, R⁵ and R⁶ are as described for formula I-b above and in the accompanying claims;
   or
(c) in case of an amide-imidazo[1,2-a]pyridine derivative of formula I-c
   the carboxylic acid of general formula II-c 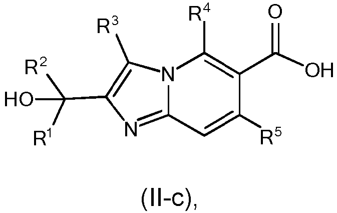
   wherein R¹, R², R³, R⁴ and R⁵ are as described for formula I-c above and in the accompanying claims,
   is subjected to an amidation reaction with a compound of formula III:

      R⁶-NH₂ (III)
   wherein R⁶ is as defined above and in the accompanying claims, optionally in the presence of a suitable catalyst, e.g. triethylamine and HATU ([dimethylamino-([1,2,3]triazolo[4,5-b]pyridin-3-yloxy)-methylene]-dimethylammonium hexafluoro phosphate) to yield the amide derivative of formula I-c 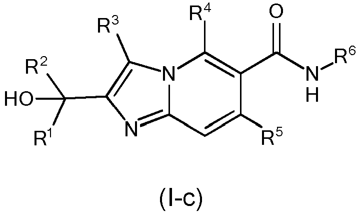
   wherein R¹, R², R³, R⁴, R⁵ and R⁶ are as described for formula I-c above and in the accompanying claims.

As will be understood by the person skilled in the art of organic synthesis compounds of the present invention, in particular compounds of formula I-a, I-b or I-c, are readily accessible by various synthetic routes, some of which are exemplified in the accompanying Experimental Part. The skilled artisan will easily recognize which kind of reagents and reactions conditions are to be used and how they are to be applied and adapted in any particular instance - wherever necessary or useful - in order to obtain the compounds of the present invention. Furthermore, some of the compounds of the present invention can readily be synthesized by reacting other compounds of the present invention under suitable conditions, for instance, by converting one particular functional group being present in a compound of the present invention, or a suitable precursor molecule thereof, into another one by applying standard synthetic methods, like reduction, oxidation, addition or substitution reactions; those methods are well known to the skilled person. Likewise, the skilled artisan will apply - whenever necessary or useful - synthetic protecting (or protective) groups; suitable protecting groups as well as methods for introducing and removing them are well-known to the person skilled in the art of chemical synthesis and are described, in more detail, in, e.g., P.G.M. Wuts, T.W. Greene, "Greene's Protective Groups in Organic Synthesis", 4th edition (2006) (John Wiley & Sons).

In the following general synthetic routes that may be utilized to prepare compounds of the present invention are described in more detail in Schemes A to E:

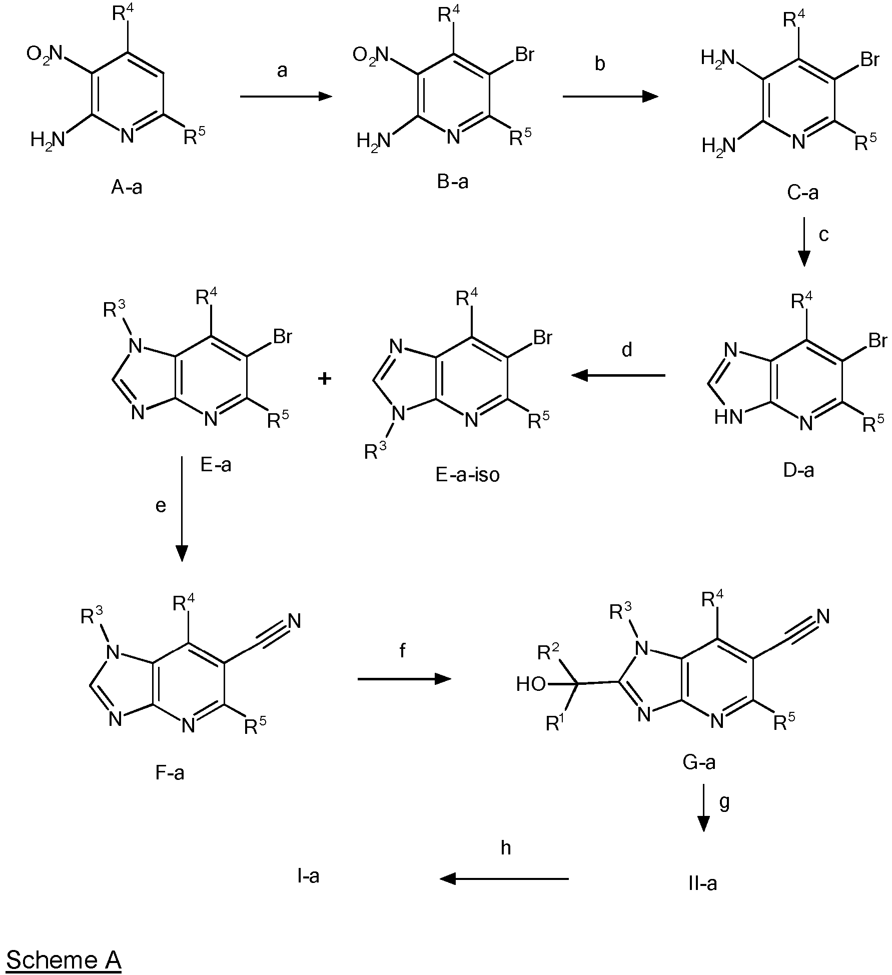

Schema A above depicts a general synthesis route for preparing amide compounds of formula I-a. The 2-amino-3-nitro-pyridine derivative A-a, which is readily available by utilizing well-know synthetic methods or through commercial sources, is converted into the 5-bromo-substituted derivative B-a by means of a suitable bromination reaction (step a), for instance, by using N-bromo succinimide (NBS), preferably in slight excess of about 1.05 to 1.15 equivalents related to A-a, in a suitable solvent, e.g., dimethylformamide (DMF). The nitro-substituent of the bromo-substituted pyridine derivative B-a may then be converted into an amino-group by means of a reduction reaction (step b) with gaseous hydrogen in the presence of a suitable metal catalyst, e.g., a sponge nickel catalyst, thereby yielding 2,3-diamino-5-bromo-substituted pyridine derivative C-a. This derivative may be isolated or, preferably, be reacted without isolation with a reaction partner that is suitable for the desired cyclization under adequate reaction conditions to yield the 3H-imidazo[4.5-b]pyridine derivative of formula D-a (step c); such reaction partner and conditions, respectively, can be, for instance, addition of triethyl orthoformate and formic acid and subsequent heating. After usual work-up compound D-a is obtained which can subsequently be converted into 1H-imidazo[4.5-b]pyridine derivative of formula E-a as well as its regioisomer E-a-iso (step d). An example for such a conversion into E-a (and E-a-iso) is the alkylation with a suitable alkylhalogenide, R³-Hal, for instance alkyliodide in the presence of a strong base, e.g. sodium hydride, with subsequent neutralization with, e.g., ammonium chloride solution. Usually the desired regioisomer E-a is obtained as the major product and the other isomer E-a-iso as the minor product which products are subsequently be separated by usual techniques, e.g., chromatography on silica gel. 1H-imidazo[4.5-b]pyridine derivative E-a is then converted into the carbonitrile derivative of formula F-a by bromine/cyanide exchange (step e); this exchange can, for instance, be effected by adding K₄[Fe(CN)₆] and potassium acetate in a suitable solvent, e.g. dioxane, and then adding a suitable catalyst, e.g. an appropriate palladium catatyst like tBuBrettPhos Pd G3 and subsequently heating the resulting reaction mixture. Carbonitrile F-a may then be converted into the tertiary alcohol G-a by reacting the carbonitrile with a ketone of formula R¹-C(=O)-R² (wherein R¹ and R² are as defined for amides of formula I-a and may be the same or different) in the presence of a strong base (step f), e.g., lithium dimethylsilylamide in a suitable solvent, e.g. THF. Carbonitrile G-a may then be converted into the respective carboxylic acid II-a under conditions typical for saponification of nitriles (step g), e.g. by reacting it with a strong base like sodium hydroxide in a suitable solvent, e.g. ethanol. In a final step (step h) the desired amide derivative I-a may be obtained by reacting the compound of formula II-a with an amine of formula III (R⁶-NH₂) under conditions which are typical for amidation reactions (step h), e.g. in the presence of a tertiary amine, like triethylamine or DIPEA (ethyldi(propan-2-yl)amineN-ethyl-N-isopropylpropan-2-amine), and suitable catalyst, like 1-[Bis(dimethylamino)methylene]-1H-1,2,3-triazolo[4,5-b]pyridinium 3-oxide hexafluorophosphate (HATU) and N,N-Dimethylpyridin-4-amine (DMAP).

Alternatively, bromide E-a may be converted into the respective carboxylic acid ester H-a (with R being alkyl) 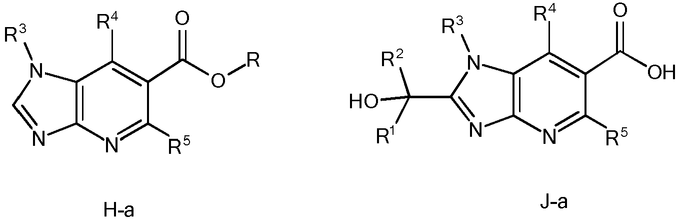 under carbonylation reaction conditions, e.g. gaseous carbon monoxide in the presence of a suitable catalyst like 1,1'-bis(diphenylphoshino)-ferrocen)dichloropalladium (II) (dichlormethane complex), 1,1-bis-(diphenylphosphino)-ferrocen, and triethylamine in a suitable dry solvent or solvent mixture like dry methanol/tetrahydrofuran. That carboxylic acid ester H-a may then be used instead of carbonitrile F-a in the subsequent reaction steps f (introduction of the tertiary alcohol and instant hydrolyzation of the carboxylic ester to provide carboxylic acid J-a) and h (amidation) to prepare amide derivative I-a thereby saving one reaction step compared to the route utilizing carbonitrile F-a.

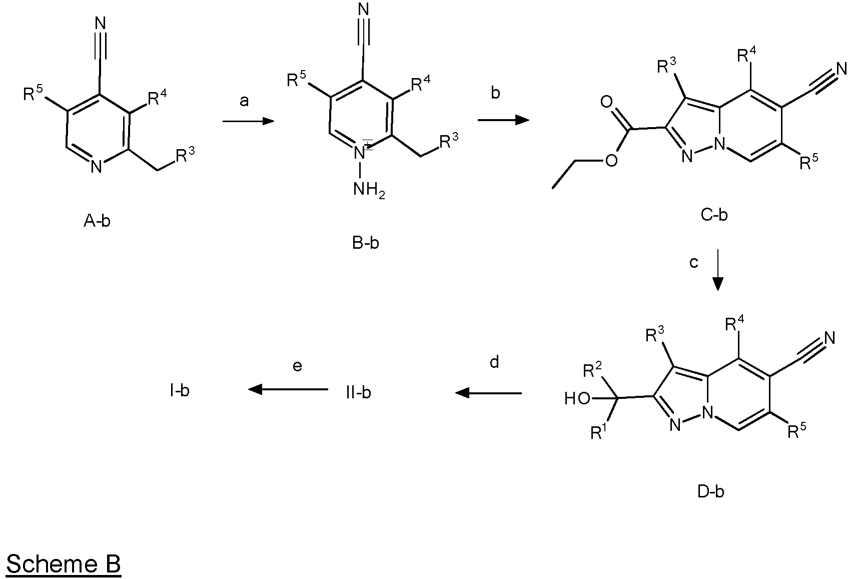

Amide compounds of formula I-b with R¹ and R² being identical are readily available via the synthetic route depicted in Scheme B above: 2-propyl-4-carbonitrile substituted pyridine derivative A-b, which is available either from commercial sources or via synthetic procedures well-known to the skilled artisan, is converted into the respective 1-amino-pyridinium compound B-b by using a suitable reagent, e.g., amino 2,4,6-trimethylbenzene-1-sulfonate in dichormethan (step a). B-b is then subjected to a cyclization reaction under typical conditions with an appropriate reaction partner like ethyl oxalochloridate (H₅C₂-O-C(=O)-C(=O)-Cl) to yield the pyrazolo[1,5-a]pyridine derivative C-b (step b). C-b may then be converted into the tertiary alcohol D-b by reacting with a suitable C-nucleophile, for instance, in a classical Grignard reaction with, e.g., R¹-Mg-Cl (or R²-Mg-Cl) (step c). Simliar to step g in Scheme A the carbontrile II-b is converted into carboxylic acid II-b (step d) which in turn may then be converted into the amide derivative I-b under conditions similar to those described for step h in Scheme A (step e).

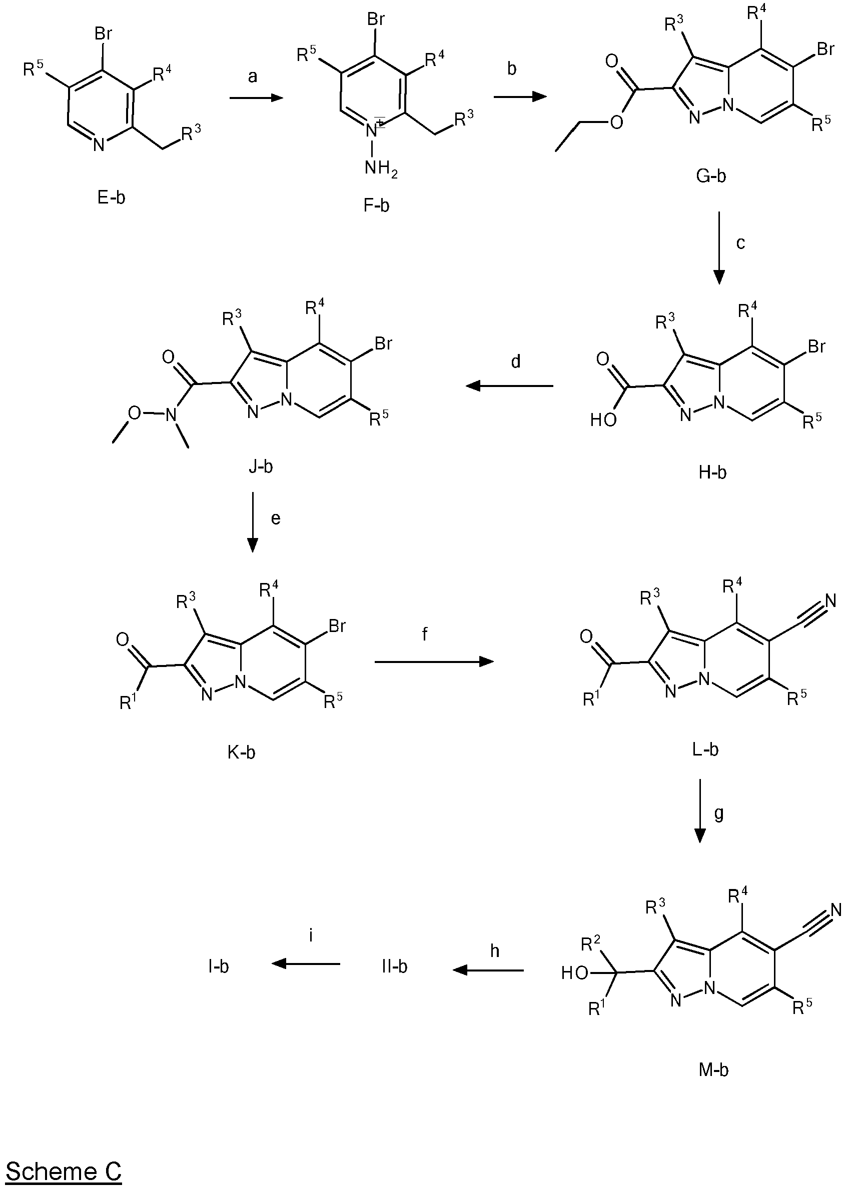

Amide compounds of formula I-b with R¹ and R² not being identical but different are readily available via the synthetic route depicted in Scheme C above: 2-propyl-4-bromo substituted pyridine derivative E-b, which is available either from commercial sources or via synthetic procedures well-known to the skilled artisan, is converted into the respective 1-amino-pyridinium compound F-b by using a suitable reagent, e.g., amino 2,4,6-trimethylbenzene-1-sulfonate in dichormethan (step a). F-b is then subjected to a cyclization reaction under typical conditions with an appropriate reaction partner like substituted alpha-halogen-substituted oxalic acid ethyl esters (C₂H₅-O-C(=O)-C(=O)-Hal, e.g. C₂H₅-O-C(=O)-C(=O)-Hal if Hal = Cl) to yield the pyrazolo[1,5-a]pyridine derivative G-b (step b). G-b is then converted into its carboxylic acid H-b under usual saponification conditions, e.g., by adding a base, for instance LiOH, NaOH or KOH. That carboxylic acid H-b is subsequently converted into the carboxamide J-b in a reaction with methoxy(methyl)amine hydrochloride under appropriate conditions, for instance, in the presence of EDC N-(3-dimethylaminopropyl)-N'-ethylcarbodiimide hydrochloride, HOBt (1-hydroxybenzotriazole) and an amine base like triethylamine. Carboxamide J-b can then be reacted with 1 equivalent of a suitable Grignard reagent R¹-Mg-Hal to provide the ketone K-b (step e). Subsequent bromine-cyanide exchange (step f) by reacting K-b, for instance, with Zn(CN)₂ and a Palladium catalyst like Pd₂(dba)₃ and XantPhos in DMF yields carbonitrile L-b which in turn is reacted with a suitable organometallic compound, e.g., R²-Li - that may be prepared in situ by reacting the appropriate halogenide R²-Hal with a suitable lithium-organic base, e.g., n-butyl lithium - to provide the tertiary alcohol M-b (step g). Similar to step g in Scheme A the carbontrile M-b is converted into carboxylic acid II-b (step h) which in turn may then be converted into the amide derivative I-b under conditions similar to those described for step h in Scheme A (step i).

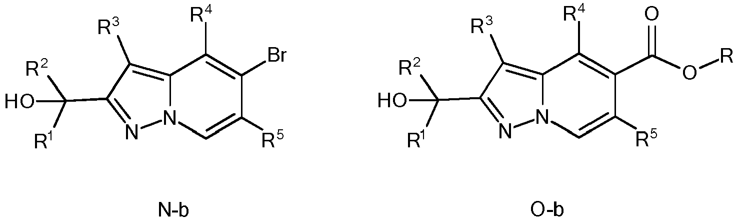

In an alternative approach amide derivative I-b can be obtained by converting ketone K-b into the tertiary alcohol N-b by utilizing a suitable organometallic compound, e.g., R²-Li - similar to step g described above; the subsequent carbonylation reaction (e.g., reaction with gaseous carbon monoxide in the presence of a suitable catalyst like 1,1'-bis(diphenylphoshino)-ferrocen)dichloropalladium (II) (dichlormethane complex), 1,1-bis-(diphenylphosphino)-ferrocen, and triethylamine in a suitable dry solvent or solvent mixture like dry methanol/tetrahydrofuran) yields ester derivative O-b which in turn may be converted into amide derivative I-b by means of saponification and subsequent amidation reactions.

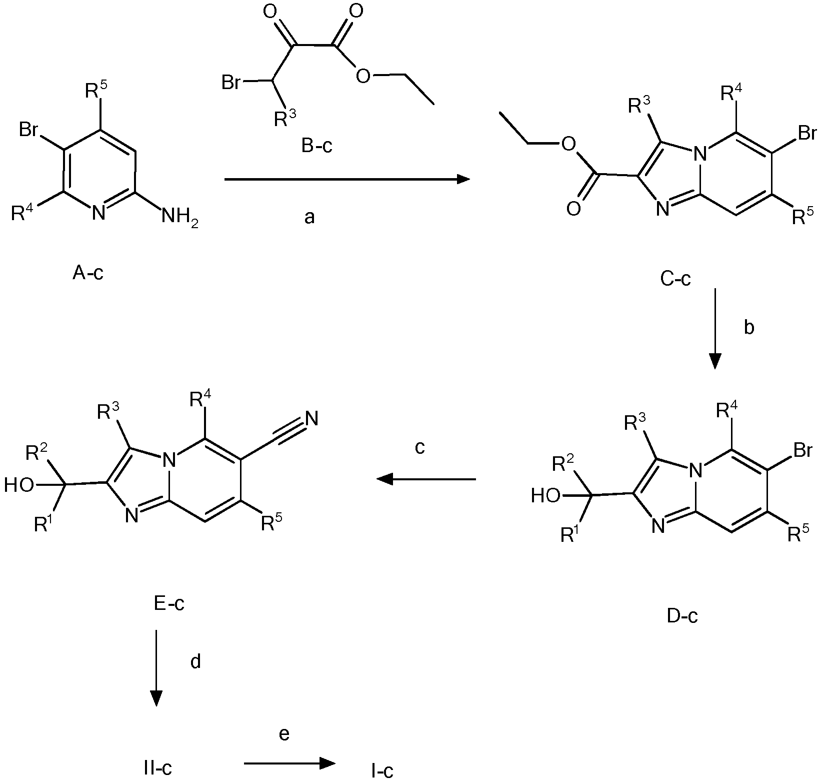

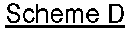

Amide compounds of formula I-c with R¹ and R² being identical are readily available via the synthetic route depicted in Scheme D above: 2-amino-5-bromo-substituted pyridine derivative A-c, which is available either from commercial sources or via synthetic procedures well-known to the skilled artisan, is subjected to a cyclization reaction under typical conditions with an appropriate reaction partner like substituted alpha-halogen-oxalic acid ethyl esters (C₂H₅-O-C(=O)-C(=O)-CHHal-R³, e.g. C₂H₅-O-C(=O)-C(=O)-CHBr-C₂H₅ if Hal = Br and R³ = ethyl (B-c)) to yield the imidazo[1,2-a]pyridine derivative C-c (step a). C-c is subsequently converted into the tertiary alcohol D-c by reacting C-c with a suitable C-nucleophile, for instance, in a classical Grignard reaction with, e.g., R¹-Mg-Cl (or R²-Mg-Cl) (step b). Subsequent bromine-cyanide exchange (step c) by reacting D-c, for instance, with Zn(CN)₂ and a Palladium catalyst like Pd(PPh₃)₄ in DMF yields carbonitrile E-c which is then converted (step d) into carboxylic acid II-c under condtions similar to step g in Scheme A. Carboxylic acid II-c may then be converted into the amide derivative I-c under conditions similar to those described for step h in Scheme A (step e).

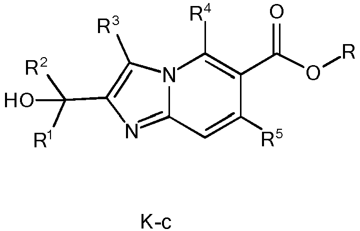

Alternatively, amide derivative I-c can be obtained by converting the bromo-substituted derivative D-c via a carbonylation reaction into the corresponding ester K-c and subsequent saponifaction and amidation reactions.

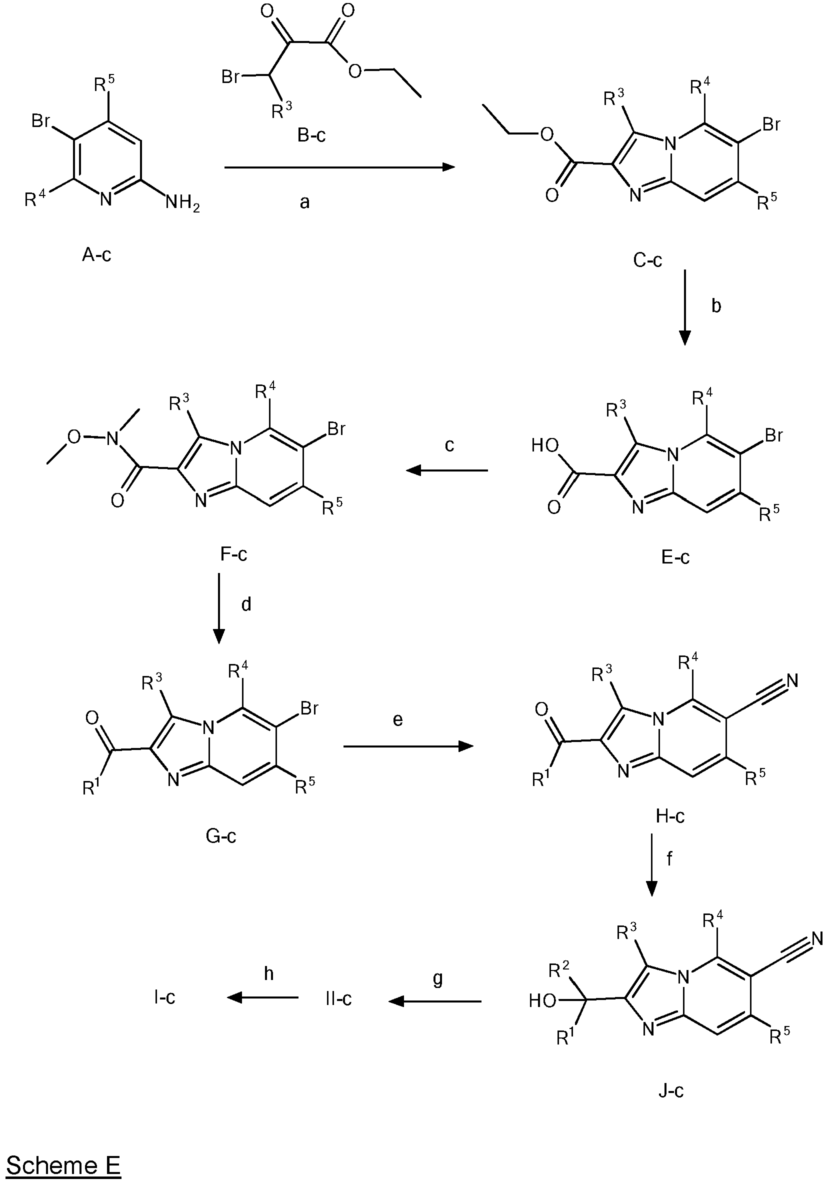

Amide compounds of formula I-c with R¹ and R² not being the same but different are readily available via the synthetic route depicted in Scheme E above: As shown in and described for Scheme D 2-amino-5-bromo-substituted pyridine derivative A-c is converted into the imidazo[1,2-a]pyridine derivative C-c (step a). Ester C-c is then converted into its carboxylic acid E-c under usual saponification conditions, e.g., by adding a base, for instance LiOH, NaOH or KOH (step b). That carboxylic acid E-c is subsequently converted into the carboxamide F-c in a reaction with methoxy(methyl)amine hydrochloride under appropriate conditions, for instance, in the presence of EDC N-(3-dimethylaminopropyl)-N'-ethylcarbodiimide hydrochloride, HOBt (1-hydroxybenzotriazole) and an amine base like triethylamine (step c). Carboxamide F-c can then be reacted with 1 equivalent of a suitable Grignard reagent R¹-Mg-Hal to provide the ketone G-c (step d). Subsequent bromine-cyanide exchange (step e) by reacting K-b, for instance, with Zn(CN)₂ in the presence of a Palladium catalyst like Pd₂(dba)₃ and XantPhos in DMF yields carbonitrile H-c which in turn is reacted with a suitable organometallic compound, e.g., R²-Li - that may be prepared in situ by reacting the appropriate halogenide R²-Hal with a suitable lithium-organic base, e.g., n-butyl lithium - to provide the tertiary alcohol J-c (step f). Similar to step g in Scheme A the compound J-c is converted into carboxylic acid II-c (step g) which in turn may then be converted into the amide derivative I-c under conditions similar to those described for step h in Scheme A (step h). Alternatively, bromide G-c may be transformed to the corresponding tertiary alcohol by using, for instance, the organo-lithium compound R²-Li; subsequent carbonylation reaction providing the respective alkyl ester and amidation reaction would then yield amide derivative I-c.

The present invention also refers to carboxylic acids of formula II-a, II-b or II-c which are useful intermediates for making amides of the present invention of formula I-a, I-b or I-c, respectively: 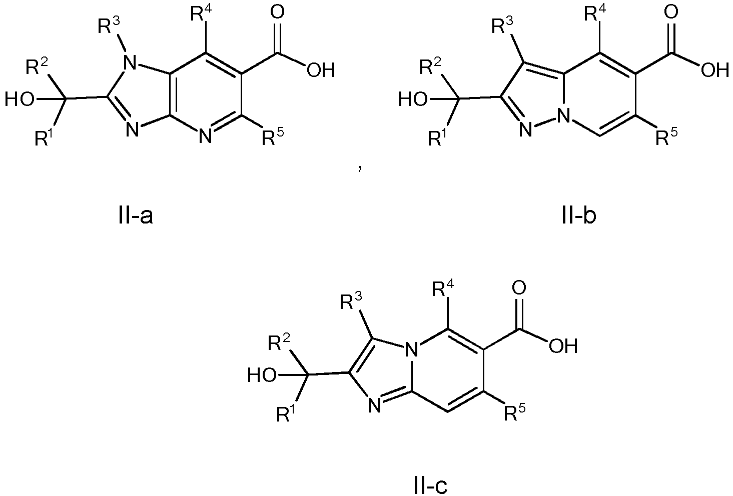 wherein R¹, R², R³, R⁴, R⁵ and R⁶ are as defined above for formulas I-a, I-b and I-c.

"Treating" ot "treatment" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with an amide of formula I-a, I-b or I-c refers to an amount (of a compound, drug, pharmaceutical compositions, etc.) capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as inflammatory conditions, immunological conditions, cancer or metabolic conditions.

It is to be noted that - except for instances where it is specifically stated or the context provides for a different meaning - in general the number of a term, i.e. its singular and plural form, is used and can be read interchangeably. For example, the term "compound" in its singular form may also comprise or refer to a plurality of compounds, while the term "compounds" in its plural form may also comprise or refer to a singular compound.

### Experimental Part

### Abbreviations

The compounds of the present invention can be prepared according to the procedures of the following Schemes and Examples, using appropriate materials and are further exemplified by the following specific examples. The compounds are shown in Table 1 which is divided in Tables 1a, 1b, 1c and 1d. Analytical data of compounds made according to the following examples are shown in Table 1 (Tables 1a, 1b, 1c and 1d) too.

The invention will be illustrated, but not limited, by reference to the specific embodiments described in the following examples. Unless otherwise indicated in the schemes, the variables have the same meaning as described above and in the claims.

Unless otherwise specified, all starting materials are obtained from commercial suppliers and used without further purifications. Unless otherwise specified, all temperatures are expressed in °C and all reactions are conducted at RT (room temperature). Compounds are purified by either silica chromatography or preparative HPLC.

### ¹H NMR:

¹H-NMR data is provided in Table 1 below. ¹H NMR spectra were usually acquired on a Bruker Avance DRX 500, Bruker Avance 400 or a Bruker DPX 300 NMR spectrometer under standard conditions using TMS (tetramethylsilan) as internal reference and DMSO-d6 as standard solvents, if not reported otherwise. NS (Number of Scans): 32, SF (Spectrometer Frequence) as indicated. TE (Temperatur): 297 K. Chemical shifts (δ) are reported in ppm relative to the residual solvent signal (δ= 2.5 ppm for ¹H NMR in DMSO-d6, δ= 7.27 ppm for ¹H NMR in CDCl₃, δ= 3.31 ppm for Methanol-d4). ¹H NMR data are reported as follows: chemical shift (multiplicity, coupling constants and number of hydrogens). Multiplicity is abbreviated as follows: s (singlet), d (doublet), t (triplet), q (quartet), m (multiplet), dd (doublet of doublets), tt (triplet of triplets), td (triplet of doublets) br (broad) and coupling constants (J) are reported in Hz.

### HPLC-MS:

HPLC-MS data provided in Table 1 are given with mass in m/z. The results can be obtained by one of the methods described below. HPLC-MS analyses were usually performed on a Shimadzu LCMS-2020, Shimadzu SP-M20A 2010EV or Shimadzu UFLC-MS 2010EV system utilizing one of the following columns: Shim-pack VP-ODS, Shim-pack XR-ODS, Kinetex XB-C18 100A, Xbridge BEH C18, Gemini-NX 3u C18 110A or ACE UltraCore 2.5 SuperC18. Standard conditions applied:
Standard solvent gradients using
   A: Water + 0.1vol.% formic acid, B: acetonitrile + 0.1vol.% formic acid, or
   A: Water + 0.05vol.% trifluoroacetic acid, B: acetonitrile + 0.05 vol.% trifluoroacetic acid
Detection wavelength: 220 nm, MS-Typ: API-ES

### General Synthesis 1

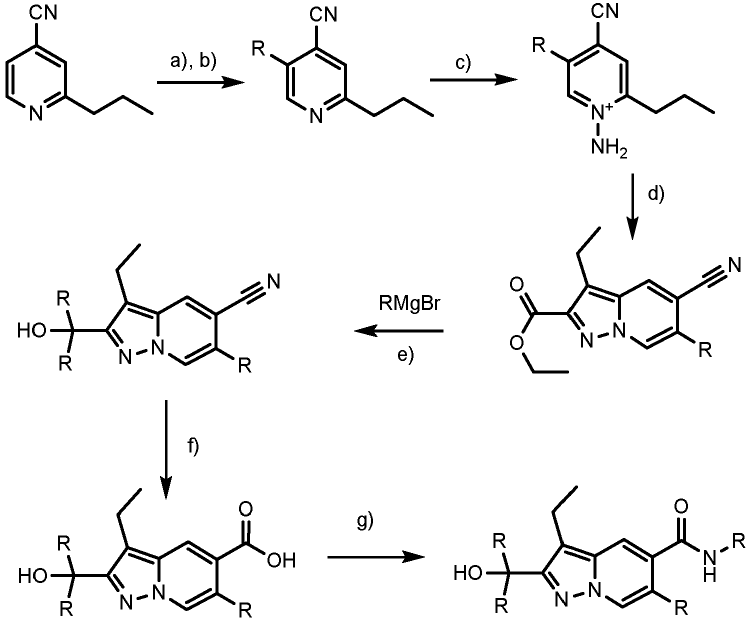

### Example 1 (carbonitrile precursor)

### 2-[bis(2-fluorophenyl)(hydroxy)methyl]-3-ethyl-6-methoxypyrazolo[1,5-a]pyridine-5-carbonitrile

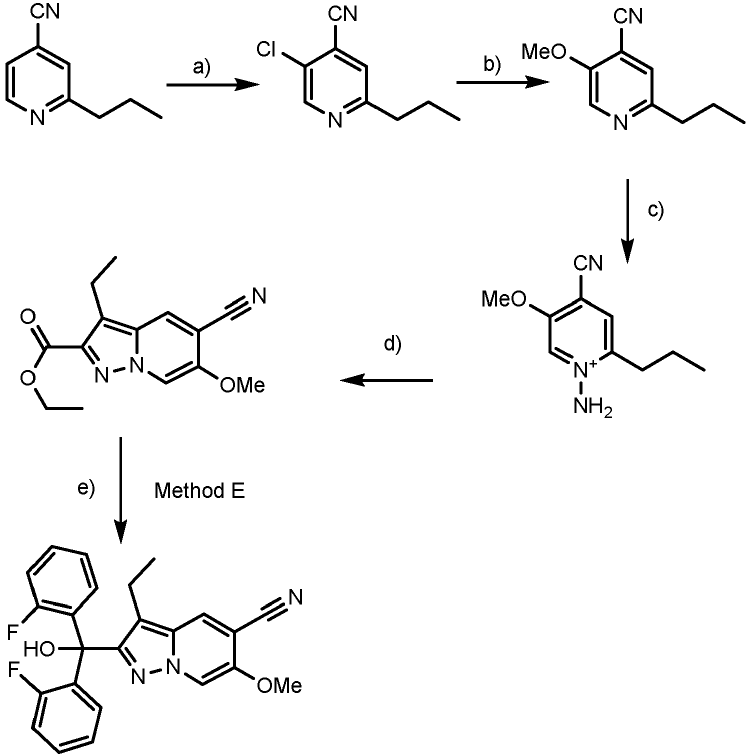

a) To a solution of 2,2,6,6-tetramethylpiperidine (15 g, 106.19 mmol) in THF (250 mL) was added nBuLi (42.5 mL, 2.5 M) at -30 °C dropwise. The resulting mixture was stirred at -30 °C for 30 min and then cooled to -78 °C. A solution of 2-propylpyridine-4-carbonitrile (7.8 g, 53.36 mmol) in THF (20 mL) was added slowly to the mixture. The reaction mixture was stirred at -78 °C for 30 min after which hexachloroethane (25 g, 105.6 mmol) was added slowly. The reaction mixture was slowly warmed to room temperature and stirred at room temperature for 30 min. Then the reaction mixture was carefully quenched with saturated NH₄Cl solution (100 mL) and diluted with water (300 mL). The resulting mixture was extracted with EtOAc (300 mL x 3) and the organic phases were combined, washed with brine and dried over Na₂SO₄. The solvent was removed under reduced pressure and the residue was purified by flash chromatography eluting with EtOAc in petroleum ether (1% to 10% gradient) to yield 5-chloro-2-propylpyridine-4-carbonitrile as brow oil (1 g, 10.4%). LC/MS [M+H]⁺ 181.1.
b) To a solution of 5-chloro-2-propylpyridine-4-carbonitrile (500 mg, 2.77 mmol) in DMSO (8 mL) was slowly added NaOMe solution (30% wt in MeOH, 1.35 g, 9.1 mmol). The resulting mixture was stirred at room temperature for 2 h. Then the reaction mixture was quenched with water (30 mL) and extracted with EtOAc (40 mL x 3). The organic phases were combined, washed with brine and dried over Na₂SO₄. The solvent was removed under reduced pressure and the residue was purified by flash chromatography eluting with EtOAc in petroleum ether (5% to 50% gradient) to yield 5-methoxy-2-propylpyridine-4-carbonitrile as yellow oil (700 mg, 90%). LC/MS [M+H]⁺ 177.1.
c) To a solution of 5-methoxy-2-propylpyridine-4-carbonitrile (650 mg, 3.69 mmol) in DCM (10 mL) was slowly added amino 2,4,6-trimethylbenzene-1-sulfonate (3.68 g, 17.08 mmol). The resulting mixture was stirred at room temperature for 2 h. The reaction mixture was concentrated under reduced pressure to yield 1-amino-4-cyano-5-methoxy-2-propylpyridin-1-ium 2,4,6-trimethylbenzene-1-sulfonate as white solid (700 mg) which was used in next step without further purification. LC/MS [M]⁺ 192.1.
d) A mixture of 1-amino-4-cyano-5-methoxy-2-propylpyridin-1-ium 2,4,6-trimethylbenzene-1-sulfonate (2.8 g, 6.16 mmol) and ethyl oxalochloridate (4.0 g, 27.8 mmol) in pyridine (10 mL) was stirred at 100 °C for 2 h. Then the reaction mixture was diluted with water (30 mL) and extracted with EtOAc (40 mL x 4). The organic phases were combined, washed with brine and dried over Na₂SO₄. The solvent was removed under reduced pressure and the residue was purified by flash chromatography eluting with EtOAc in petroleum ether (10% to 50% gradient) to yield ethyl 5-cyano-3-ethyl-6-methoxypyrazolo[1,5-a]pyridine-2-carboxylate as yellow oil (500 mg). LC/MS [M+H]⁺ 274.0.
e) 2-[bis(2-fluorophenyl)(hydroxy)methyl]-3-ethyl-6-methoxypyrazolo[1,5-a]pyridine-5-carbonitrile was prepared from ethyl 5-cyano-3-ethyl-6-methoxypyrazolo[1,5-a]pyridine-2-carboxylate using Method E as described below for Example 5. LC/MS [M-OH]⁺ 366.0.

### Example 2

### 2-[Bis(2-fluorophenyl)(hydroxy)methyl]-3-ethyl-N-(1-ethyl-1H-1,2,4-triazol-3-yl)-6-methoxypyrazolo[1,5-a]pyridine-5-carboxamide

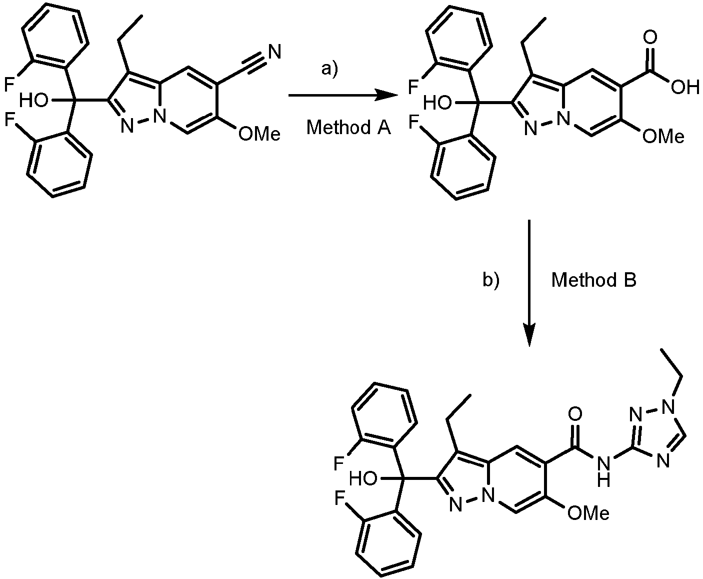

a) **Method A:** To a solution of 2-[bis(2-fluorophenyl)(hydroxy)methyl]-3-ethyl-6-methoxypyrazolo[1,5-a]pyridine-5-carbonitrile (90 mg, 0.16 mmol) (Example 1) in EtOH (4 mL) and H₂O (1 mL) was added NaOH (104 mg, 2.46 mmol) slowly. The resulting mixture was stirred at 80 °C for 3 h. Then the reaction mixture was cooled to room temperature and the pH value was adjusted to 6 with 6 M HCl solution. The resulting mixture was extracted with DCM and the organic phases were combined, washed with brine and dried over Na₂SO₄. The solvent was removed under reduced pressure to yield 2-[bis(2-fluorophenyl)(hydroxy)methyl]-3-ethyl-6-methoxypyrazolo[1,5-a]pyridine-5-carboxylic acid as yellow solid (60 mg, 82%) which was used in next step without further purification. LCMS [M+H]⁺ 439.0.
b) **Method B:** To a solution of 2-[bis(2-fluorophenyl)(hydroxy)methyl]-3-ethyl-6-methoxypyrazolo[1,5-a]pyridine-5-carboxylic acid (21 mg, 0.05 mmol) in DCM (3 mL) was added 1-[Bis(dimethylamino)methylene]-1H-1,2,3-triazolo[4,5-b]pyridinium 3-oxide hexafluorophosphate (HATU) (21.9 mg, 0.05 mmol), ethyldi(propan-2-yl)amineN-ethyl-N-isopropylpropan-2-amine (DIPEA) (74.3 mg, 0.56 mmol), *N,N*-Dimethylpyridin-4-amine (DMAP) (1.1 mg, 0.01 mmol) and 1-ethyl-1H-1,2,4-triazol-3-amine hydrochloride (53.7 mg, 0.36 mmol). The resulting mixture was stirred at room temperature for 16 h. The reaction mixture was concentrated under reduced pressure and the residue was purified by prep-HPLC under the following conditions: column, XBridge Prep C18 OBD Column, 19 x 150 mm, 5 µm; mobile phase, MeCN in water (with 10 mM NH₄HCO₃ and 0.1% NH₃.H₂O), 40% to 43% gradient in 8 min; detector, UV 254/220 nm. 2-[Bis(2-fluorophenyl)(hydroxy)methyl]-3-ethyl-N-(1-ethyl-1H-1,2,4-triazol-3-yl)-6-methoxypyrazolo[1,5-a]pyridine-5-carboxamide was obtained as a white solid (2.9 mg, 11%). LCMS [M+H]⁺ 533.3.

### General Synthesis 2

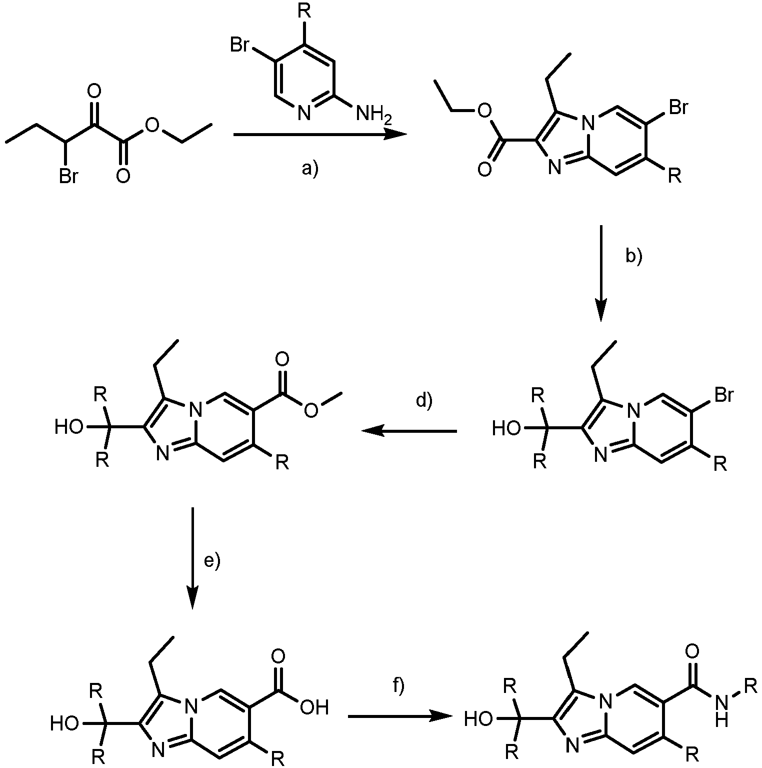

### Example 3 - bromo-substituted precursor

### [6-bromo-3-ethyl-7-methoxyimidazo[1,2-a]pyridin-2-yl]diphenylmethanol

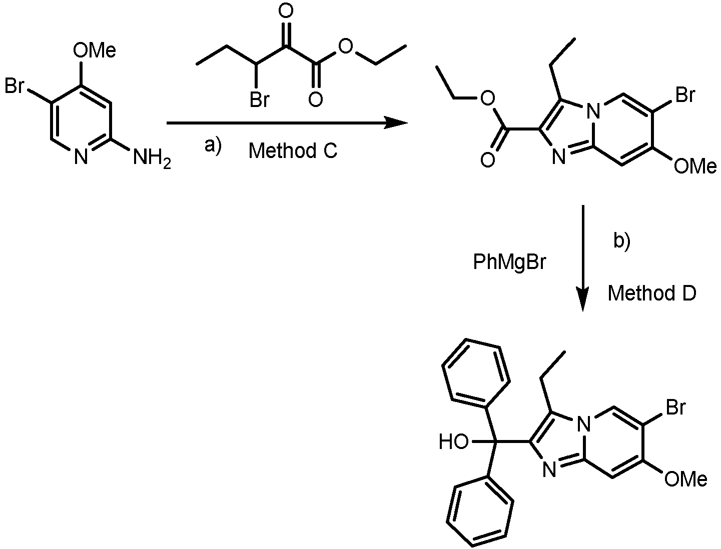

a) **Method C:** A mixture of 5-bromo-4-methoxypyridin-2-amine (4 g, 19.70 mmol) and ethyl 3-bromo-2-oxopentanoate (8 g, 35.86 mmol) in EtOH (40 mL) was stirred at 80 °C for 16 h. Then the reaction mixture was cooled to room temperature and the pH value was adjusted to 8 with NaHCO₃. The mixture was diluted with water (100 mL) and extracted with DCM (100 mL x 3). The combined organic phases were washed with brine and dried over Na₂SO₄. The solvent was removed under reduced pressure and the residue was purified by flash chromatography eluting with EtOAc in petroleum ether (5% to 30% gradient) to yield ethyl 6-bromo-3-ethyl-7-methoxyimidazo[1,2-a]pyridine-2-carboxylate as light yellow solid (2.8 g, 38%). LC/MS: [M+H]⁺ 327.1/329.1.
b) **Method D:** To a solution of ethyl 6-bromo-3-ethyl-7-methoxyimidazo[1,2-a]pyridine-2-carboxylate (1.3 g, 3.97 mmol) in THF (130 mL) was added PhMgBr (1 M in THF, 11.9 mL, 11.9 mmol) dropwise at 0 °C. The resulting mixture was kept stirring at 0 °C for 1 h. Then the reaction mixture was carefully quenched with saturated NH₄Cl solution (20 mL) and diluted with water (100 mL). The aqueous phase was extracted with DCM (150 mL x 2) and the organic phases were combined, washed with brine and dried over Na₂SO₄. The solvent was removed under reduced pressure and the residue was purified by flash chromatography eluting with MeOH in DCM (1% to 5% gradient) to yield [6-bromo-3-ethyl-7-methoxyimidazo[1,2-a]pyridin-2-yl]diphenylmethanol as yellow solid (1.4 g, 80%). LC/MS [M+H]⁺ 437.1/439.1.

### Example 4

### 3-Ethyl-2-(hydroxy-diphenyl-methyl)-7-methoxy-imidazo[1,2-a]pyridine-6-carboxylic acid (1-ethyl-1H-[1,2,4]triazol-3-yl)-amide

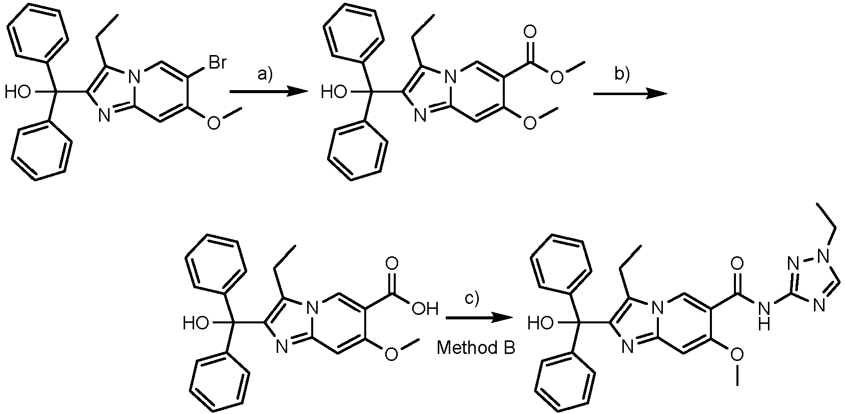

a) A mixture of [6-bromo-3-ethyl-7-methoxyimidazo[1,2-a]pyridin-2-yl]diphenylmethanol (300 mg, 0.68 mmol) (Example 3), Pd(dppf)Cl₂ (50 mg, 0.067 mmol) and triethylamine (208 mg, 2.06 mmol) in MeOH (13 mL) was stirred for 6 h at 100 °C under the atmosphere of CO (10 atm). Then the reaction mixture was diluted with water (40 mL) and extracted with DCM (40 mL x 3). The combined organic phase was washed with brine and dried over Na₂SO₄. The solvent was removed under reduced pressure and the residue was purified by flash chromatography eluting with MeOH in DCM (1% to 5% gradient) to yield methyl 3-ethyl-2-(hydroxydiphenylmethyl)-7-methoxyimidazo[1,2-a]pyridine-6-carboxylate as light brown solid (230 mg, 80%). LCMS [M+H]⁺ 417.3.
b) To a solution of methyl 3-ethyl-2-(hydroxydiphenylmethyl)-7-methoxyimidazo[1,2-a]pyridine-6-carboxylate (650 mg, 1.56 mmol) in THF (10 mL) and H₂O (5 mL) was added LiOH (36 mg, 1.51 mmol) slowly. The resulting mixture was stirred at room temperature for 16 h. Then the pH value of the reaction mixture was adjusted to ~6 with 2 M HCl solution. The resulting mixture was extracted with DCM (30 mL x 5) and the organic phases were combined, washed with brine and dried over Na₂SO₄. The solvent was removed under reduced pressure to yield 3-ethyl-2-(hydroxydiphenylmethyl)-7-methoxyimidazo[1,2-a]pyridine-6-carboxylic acid as yellow solid (600 mg, 95%) which was used in next step without further purification. LCMS [M+H]⁺ 403.2.
c) 3-Ethyl-2-(hydroxy-diphenyl-methyl)-7-methoxy-imidazo[1,2-a]pyridine-6-carboxylic acid (1-ethyl-1H-[1,2,4]triazol-3-yl)-amide was prepared from 3-ethyl-2-(hydroxydiphenylmethyl)-7-methoxyimidazo[1,2-a]pyridine-6-carboxylic acid using **Method B.** The product was purified by prep-HPLC under the following conditions: column, XBridge Shield RP18 OBD Column, 30 x 150 mm, 5 µm; mobile phase, MeCN in water (with 10 mM NH₄HCO₃ and 0.1% NH₃.H₂O), 35% to 50% gradient in 8 min; detector, UV 254/220 nm. 3-Ethyl-N-(1-ethyl-1H-1,2,4-triazol-3-yl)-2-(hydroxydiphenylmethyl)-7-methoxyimidazo[1,2-a]pyridine-6-carboxamide was obtained as white solid (50 mg, 22%). LCMS [M+H]⁺ 497.2.

### Example 5 - carbontrile prescursor

### 2-[bis(2-fluorophenyl)(hydroxy)methyl]-3-ethyl-7-methoxyimidazo[1,2-a]pyridine-6-carbonitrile

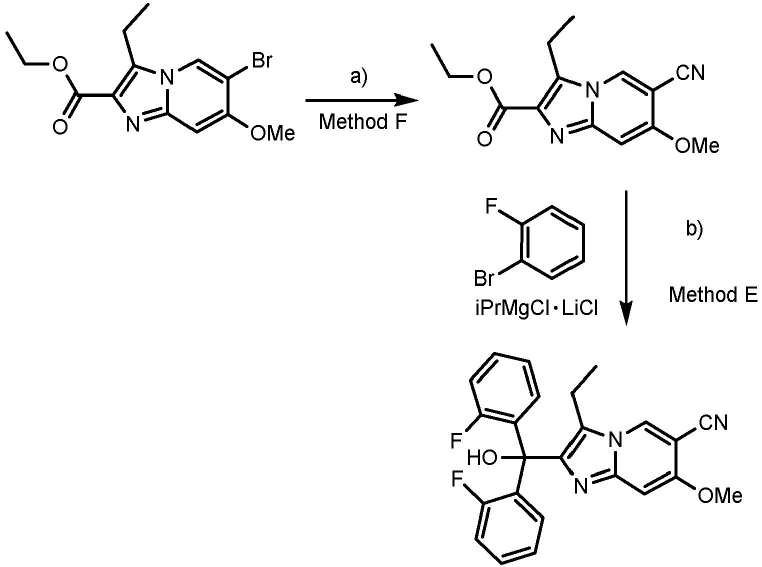

a) **Method F:** A mixture of ethyl 6-bromo-3-ethyl-7-methoxyimidazo[1,2-a]pyridine-2-carboxylate (1.5 g, 4.58 mmol) (see Example 3 a)), Zn(CN)₂ (0.3 g, 2.51 mmol), Pd₂(dba)₃ (1.2 g, 1.3 mmol) and XantPhos (0.7 g, 1.3 mmol) in DMF (30 mL) was stirred for 3 h at 90 °C under nitrogen atmosphere. Then the reaction mixture was diluted with water and extracted with CH₂Cl₂. The organic phases were combined, washed with brine and dried over Na₂SO₄. The solvent was removed under reduced pressure and the residue was purified by flash chromatography eluting with MeOH in DCM (1% to 7% gradient) to yield ethyl 6-cyano-3-ethyl-7-methoxyimidazo[1,2-a]pyridine-2-carboxylate as yellow solid (1.2 g, 92%). LC/MS [M+H]⁺ 274.1.
b) **Method E:** To a solution of 1-bromo-2-fluorobenzene(2.37 g, 13.54 mmol) in THF (30 mL) was added iPrMgCl•LiCl (10.4 mL, 1.3 M in THF) dropwise at -15 °C. The resulting mixture was stirred at -15 °C for 2 h after which a solution of ethyl 6-cyano-3-ethyl-7-methoxyimidazo[1,2-a]pyridine-2-carboxylate (1.2 g, 4.40 mmol) in THF (10 mL) was added slowly. The reaction mixture was kept stirring at -15 °C for additional 2 h. Then the reaction mixture was carefully quenched with saturated NH₄Cl solution (20 mL) and diluted with water (30 mL). The resulting mixture was extracted with DCM (80 mL x 3) and the organic phases were combined, washed with brine and dried over Na₂SO₄. The solvent was removed under reduced pressure and the residue was purified by flash chromatography eluting with MeOH in DCM (1% to 5% gradient) to yield 2-[bis(2-fluorophenyl)(hydroxy)methyl]-3-ethyl-7-methoxyimidazo[1,2-a]pyridine-6-carbonitrile as yellow solid (900 mg, 44%). LC/MS [M+H]⁺ 420.1.

### Example 6

### 2-[Bis(2-fluorophenyl)(hydroxy)methyl]-3-ethyl-N-(1-ethyl-1H-1,2,4-triazol-3-yl)-7-methoxyimidazo[1,2-a]pyridine-6-carboxamide

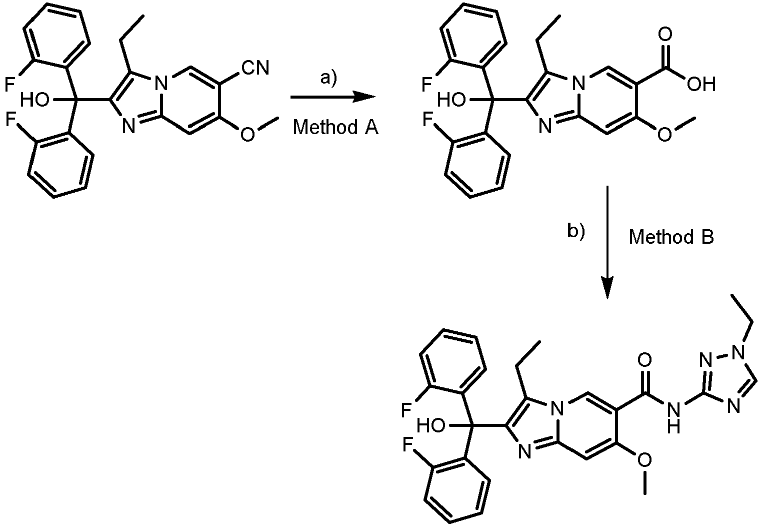

2-[Bis(2-fluorophenyl)(hydroxy)methyl]-3-ethyl-N-(1-ethyl-1H-1,2,4-triazol-3-yl)-7-methoxyimidazo[1,2-a]pyridine-6-carboxamide was prepared from 2-[bis(2-fluorophenyl)(hydroxy)methyl]-3-ethyl-7-methoxyimidazo[1,2-a]pyridine-6-carbonitrile using **Method A** and **B.** The final product was purified by prep-HPLC under the following conditions: column, XBridge Shield RP18 OBD Prep Column, 30 x 150 mm, 5 µm; mobile phase, MeCN in water (with 10 mM NH₄HCO₃ and 0.1% NH₃.H₂O), 35% to 40% gradient in 8 min; detector, UV 254/220 nm. 2-[Bis(2-fluorophenyl)(hydroxy)methyl]-3-ethyl-N-(1-ethyl-1H-1,2,4-triazol-3-yl)-7-methoxyimidazo[1,2-a]pyridine-6-carboxamide was obtained as white solid (30 mg, 14%). LCMS [M+H]⁺ 533.1.

### Example 7 - synthesis of carbonitrile precursors with R¹ being different to R²

### 7-chloro-3-ethyl-2-[hydroxy(phenyl)(pyridin-2-yl)methyl]imidazo[1,2-a]pyridine-6-carbonitrile

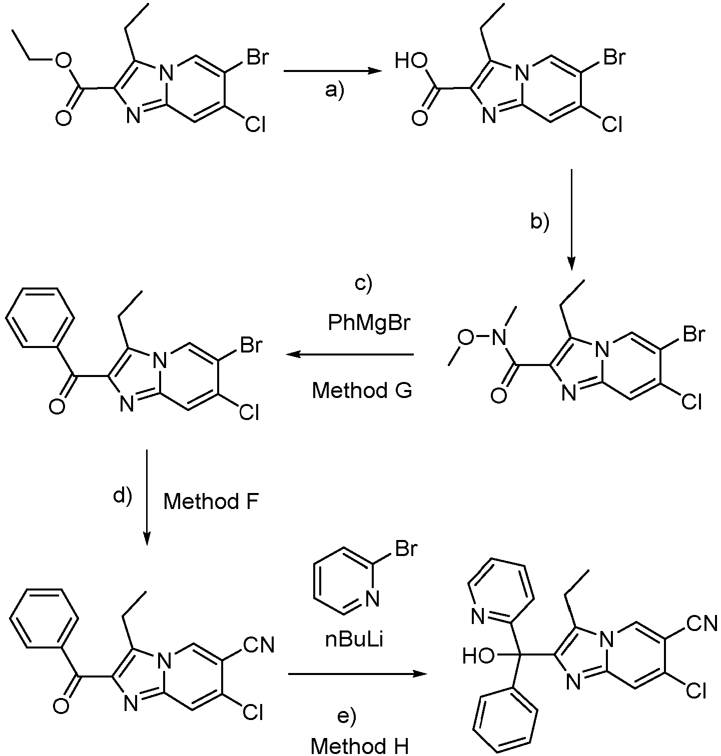

Ethyl 6-bromo-7-chloro-3-ethylimidazo[1,2-a]pyridine-2-carboxylate was prepared from 5-bromo-4-chloropyridin-2-amine using **Method C.**
a) To a solution of ethyl 6-bromo-7-chloro-3-ethylimidazo[1,2-a]pyridine-2-carboxylate (2.5 g, 7.54 mmol) in THF (50 mL) and H₂O (10 mL) was added LiOH (632 mg, 26.4 mmol) in portions. The resulting mixture was stirred at room temperature for 16 h. Then the pH value of the reaction mixture was carefully adjusted to 5-6 with 6 M HCl aq. The organic solvent was removed under reduced pressure and the resulting solid was collected by filtration. The solid was washed with water and dried under high vacuum to yield 6-bromo-7-chloro-3-ethylimidazo[1,2-a]pyridine-2-carboxylic acid as yellow solid (2.1 g, 91%). LC/MS [M+H]⁺ 302.8/304.8.
b) To a solution of 6-bromo-7-chloro-3-ethylimidazo[1,2-a]pyridine-2-carboxylic acid (2.1 g, 6.91 mmol) in CH₂Cl₂ (200 mL) was added N-(3-Dimethylaminopropyl)-N'-ethylcarbodiimide hydrochloride (2.65 g, 13.8 mmol), 1-hydroxybenzotriazole (1.87 g, 13.8 mmol), NEt₃ (2.8 g, 27.7 mmol), and methoxy(methyl)amine hydrochloride (2.02 g, 20.76 mmol). The reaction mixture was stirred at room temperature for 16 h. Then it was diluted with water and extracted with CH₂Cl₂. The combined organic phase was washed with brine and dried over Na₂SO₄. The solvent was removed under reduced pressure and the residue was purified by flash chromatography eluting with EtOAc in petroleum ether (5% to 50% gradient) to yield 6-bromo-7-chloro-3-ethyl-N-methoxy-N-methylimidazo[1,2-a]pyridine-2-carboxamide as yellow solid (1.5 g, 63%). LC/MS [M+H]⁺ 345.8./347.9.
c) **Method G:** To a solution of 6-bromo-7-chloro-3-ethyl-N-methoxy-N-methylimidazo[1,2-a]pyridine-2-carboxamide(1.5 g, 4.33 mmol) in THF (200 mL) was added PhMgBr (5.8 mL, 1 M in THF) at -78 °C dropwise. The mixture was kept stirring at -78 °C for 2 h after which it was carefully quenched with saturated NH₄Cl solution and diluted with water. The aqueous layer was extracted with CH₂Cl₂ and the combined organic phases were washed with brine and dried over Na₂SO₄. The solvent was removed under reduced pressure and the residue was purified by flash chromatography eluting with EtOAc in petroleum ether (10% to 50% gradient) to yield 2-benzoyl-6-bromo-7-chloro-3-ethylimidazo[1,2-a]pyridine as yellow solid (1.2 g, 72%). LC/MS [M+H]⁺ 362.9. / 364.9
d) 2-benzoyl-7-chloro-3-ethylimidazo[1,2-a]pyridine-6-carbonitrile was prepared from 2-benzoyl-6-bromo-7-chloro-3-ethylimidazo[1,2-a]pyridine using **Method F.** LC/MS [M+H]⁺ 310.0.
e) **Method H:** To a solution of 2-bromopyridine (893 mg, 5.65 mmol) in THF (100 mL) was added nBuLi (1.72 mL, 2.5 M in THF) at -78 °C dropwise. The resulting mixture was kept stirring at -78 °C for 1 h, followed by the slow addition of 2-benzoyl-7-chloro-3-ethylimidazo[1,2-a]pyridine-6-carbonitrile (700 mg, 2.26 mmol) in THF (20 mL). The mixture was kept stirring at -78 °C for additional 1 h after which it was carefully quenched with saturated NH₄Cl solution and diluted with water. The aqueous layer was extracted with CH₂Cl₂ and the combined organic phases were washed with brine and dried over Na₂SO₄. The solvent was removed under reduced pressure and the residue was purified by flash chromatography eluting with MeOH in DCM (1% to 8% gradient) to yield 7-chloro-3-ethyl-2-[hydroxy(phenyl)(pyridin-2-yl)methyl]imidazo[1,2-a]pyridine-6-carbonitrile as yellow solid (500 mg, 53%). LC/MS [M+H]⁺ 389.1.

Amide derivatives of the present invention with different substituents R¹ and R² can be prepared starting from carbontriles prepared in accordance or similar to the procedures given in Example 7 by utilizing the methods described in Examples 2, 4, and 6.

### Example 8

### 1-Ethyl-2-(hydroxy-diphenyl-methyl)-5-methoxy-1H-imidazo[4,5-b]pyridine-6-carboxylic acid-(1-ethyl-1H-[1,2,4]triazol-3-yl)-amide

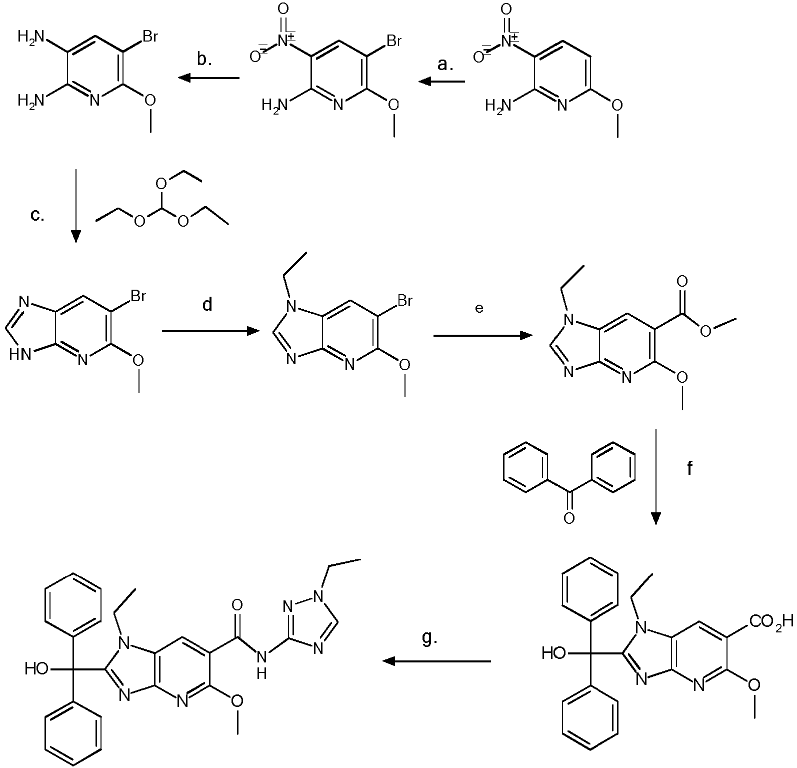

a) Synthesis of 5-bromo-6-methoxy-3-nitro-pyridin-2-ylamine In a 1 L two-necked flask 6-methoxy-3-nitro-pyridin-2-ylamine (50 g, 286.75 mmol) was dissolved in dry N,N-dimethylformamide (500 ml). N-bromosuccinimide (56.40 g, 313.69 mmol) was added in portions over the time course of 20 min at 14 - 18 °C. The reaction mixture was stirred for 1 hr at room temp. whereas complete conversion to the desired product was observed.
   The reaction mixture was poured into 1.5 L of water and stirred for further 30 min at room temp. The solid was filtered off with suction and washed with water. The remaining residue was dried under vacuum at 50°C overnight to yield pure 5-bromo-6-methoxy-3-nitro-pyridin-2-ylamine (69.76 g. 280.14 mmol). [M+H]⁺ = 247.0 - 249.9 (single bromine isotope distribution).
b) Synthesis of 5-bromo-6-methoxy-pyridine-2,3-diamine &
c) 6-Bromo-5-methoxy-3H-imidazo[4.5-b]pyridine 5-Bromo-6-methoxy-3-nitro-pyridin-2-ylamine (10 g, 40.32 mmol) was dissolved in tetrahydrofuran (100 ml). Subsequently, the reaction mixture was treated overnight (16 hours) with sponge nickel catalyst (3g, pH neutral, THF) and hydrogen under standard pressure at room temp. After filtration and having rinsed the filter cake with additional tetrahydrofuran, a solution of 5-bromo-6-methoxy-pyridine-2,3-diamine (8.79g, 40.3 mmol) in approx. 300 mL of THF was obtained and used in the next step without further purification. To the solution in a 1 L three-necked flask with condenser, triethyl orthoformate (219.75 ml) and formic acid (strength 98-100%, 3.84 ml, 100.78 mmol) were added. Then, the reaction mixture was heated at 90°C under an argon atmosphere for 3 hours. For work-up, the reaction mixture was evaporated in vacuo. Subsequently, the residue was dissolved in methanol (approx. 150 ml), diluted with aqueous HCl solution (strength 2.0 M, approx. 500 ml) and demineralized water (approx. 300 ml) followed by extraction with ethyl acetate (twice, 300 ml each). The organic layer was discarded. The aqueous layer was cooled in an ice bath and neutralized with aqueous KOH solution (strength 47%, approx. 50 ml) under stirring during a time course of 30 min to obtain a solution of pH 6. The formed precipitate was filtered off with suction, washed twice with demineralized water (50 ml each), and dried in the vacuum drying cabinet (approx. 60 mbar at 65°C for 63 hours) to yield 6-bromo-5-methoxy-3H-imidazo[4.5-b] pyridine as a solid (6.39 g, purity 97.9%, 27.44 mmol, yield 68.1 %). A second crop of the title product was obtained by extracting the remaining aqueous layer with ethyl acetate (twice, 200 ml each). The combined organic layers were dried over sodium sulfate, filtered with suction, and evaporated in vacuo to yield 6-bromo-5-methoxy-3H-imidazo[4.5-b] pyridine (1.17 g, purity 83.9%, 4.30 mmol, yield 10.7 %). [M+H]⁺ = 228.0/230.0 (single bromine isotope distribution).
d) Synthesis of 6-bromo-1-ethyl-5-methoxy-1H-imidazo[4.5-b]pyridine 6-Bromo-5-methoxy-3H-imidazo[4.5-b]pyridine (11.50 g, 47.86 mmol) was suspended in a mixture of dry tetrahydrofuran (20.13 ml) and dry 1.4-dioxane (60.38 ml). The flask was rendered inert with argon and the suspension was cooled in an ice bath while being kept at 0-5°C. Subsequently, a sodium hydride suspension (strength 60% in paraffin oil, 2.39 g, 59.82 mmol) was added in portions (twice 1.20g each, caution: hydrogen formation). After completed additions, the flask was again rendered inert with argon and the suspension was stirred at a temp. of 0-5°C for 15 min. Then, iodoethane (4.54 ml, 55.04 mmol, stabilised with silver) was added dropwise within 5 minutes and stirring was continued for 30 min. The reaction mixture was allowed to warm up to room temp. while stirring was continued for 63 hours (approx. 50% conversion). A second crop of sodium hydride suspension (strength 60% in paraffin oil, 2.39 g, 59.82 mmol) was added in two portions (1.12 g each) and stirring was continued for 15 min, followed by the addition of iodoethane (4.54 ml, 55.04 mmol). After stirring at room temp. for 19 hours (approx. 32% of starting material remained), the procedure was repeated for a third time with sodium hydride suspension (strength 60% in paraffin oil, 4.31g, 107.68 mmol) and iodoethane (8.48 ml, 102.89 mmol) whereas the suspension was diluted with additional dry tetrahydrofuran (20.13 ml). After warming up to room temp., stirring was continued for further 17 hours until the reaction was completed. For work-up, the reaction mixture was quenched under stirring using saturated aqueous ammonium chloride solution (approx. 20 ml), followed by dilution with demineralized water (approx. 300 ml), and ethyl acetate (approx. 500 ml). After being stirred for additional 30 min, the mixture was filtered and extracted twice with ethyl acetate (200 ml each). The combined organic layers were dried over sodium sulfate, filtered with suction, and evaporated to dryness. The obtained crude product was purified by flash chromatography on silica gel (330g, solvent gradient dichloromethane/0-0.6 vol.% ethanol) to yield the title product 6-bromo-1-ethyl-5-methoxy-1H-imidazo[4.5-b]pyridine (7.95 g, purity 96.7%, 29.99 mmol, yield 62.7 %, HPTLC Silica Gel 60 F254 with Rf 0.61 using solvent mixture dichloromethane - ethanol 10:1, vol./vol.) and byproduct 6-bromo-3-ethyl-5-methoxy-3Himidazo[4.5-b]pyridine (2.40 g, purity 94.7%, 8.88 mmol, yield 18.6 %). [M+H]⁺ = 256.0/258.0 (single bromine isotope distribution).
e) Synthesis of 1-ethyl-5-methoxy-1 H-imidazo[4,5-b]pyridine-6-carboxylic acid methyl ester
   In an autoclave, 6-bromo-1-ethyl-5-methoxy-1H-imidazo[4,5-b]pyridine (859 mg, 3 mmol), 1,1'-bis(diphenylphoshino)-ferrocen)dichloropalladium (II) (dichlormethane complex, 75 mg, 0.092 mmol), 1,1-bis-(diphenylphosphino)-ferrocen (51 mg, 0.092 mmol), and triethylamine (596 µl, 4.3 mmol) were dissolved in dry methanol (15 ml) and tetrahydrofuran (15 ml). Subsequently, the reaction mixture was treated with carbon monoxide (grade 3.7, 68 ml) at a CO pressure of 3-5 bar at 100°C for 21.5 hours. For work-up, the obtained solution was neutralized with saturated aqueous ammonium chloride solution (5 ml), diluted with demineralized water (45 ml), and extracted four times with ethyl acetate (40 ml each). The combined organic layers were dried over sodium sulfate, filtered with suction, and evaporated to dryness. The crude product was purified by flash chromatography on silca gel (24g, solvent gradient dichloromethane/0-5 vol.% ethanol) to yield solid 1-ethyl-5-methoxy-1H-imidazo[4,5-b]pyridine-6-carboxylic acid methyl ester (733.1 mg, purity 94.8%, 2.95 mmol, yield 96,2%). [M+H]⁺ = 236.1, HPTLC: dichloromethane/ethanol 20:1 (vol./vol.) R_{f} 0.33.
f) Synthesis of 1-ethyl-2-(hydroxy-diphenyl-methyl)-5-methoxy-1H-imidazo[4,5-b]pyridine-6-carboxylic acid
   Under an atmosphere of argon, 1-ethyl-5-methoxy-1H-imidazo[4,5-b]pyridine-6-carboxylic acid methyl ester (366 mg, 1.48 mmol) and benzophenone (339 mg, 1.84 mmol) were dissolved in dry tetrahydrofuran (5.5 ml). The suspension was cooled to 0 - 5°C in an ice bath. Then, lithium bis(trimethylsilyl) amide solution (1.0 M in THF, 1.77 ml, 1.77 mmol) was added dropwise within 2 minutes and the solution was stirred for an additional 1 hr. Thereafter, tetrahydrofuran (5,5 ml), demineralized water (3.55 ml), and lithium hydroxide (144 mg, 5.9 mmol) were added and stirring was continued at room temp. for further 16 hrs. For work-up, the reaction mixture was diluted with demineralized water (30 ml), basified with NaOH (strength 2.0 M, 15 ml) to pH 14, and extracted twice with dichloromethane (20 ml each). The organic layer was discarded. The combined aqueous layers were acidified with HCl (strength 25%, approx. 8 ml) to pH 2 and extracted three times with ethyl acetate (40 ml each). The combined organic layers were dried over sodium sulfate, filtered with suction, and evaporated in vacuo to give 1-ethyl-2-(hydroxy-diphenyl-methyl)-5-methoxy-1 H-imidazo[4,5-b]pyridine-6-carboxylic acid (459.6 mg, purity 82.9%, 0.94 mmol, yield 64.1 %), which was used without further purification in the next step. [M+H]⁺ = 404.1
g) Synthesis of 1-ethyl-2-(hydroxy-diphenyl-methyl)-5-methoxy-1H-imidazo[4,5-b]pyridine-6-carboxylic acid (1-ethyl-1H-[1,2,4]triazol-3-yl)-amide Under an argon atmosphere, 1-ethyl-2-(hydroxy-diphenyl-methyl)-5-methoxy-1H-imidazo[4,5-b]pyridine-6-carboxylic acid (140 mg, purity 82.9%, 0.29 mmol) and 1-ethyl-1H-[1,2,4]triazol-3-ylamine (96.8 mg, 0.86 mmol) were dissolved in dry N,N-dimethylformamide (2.80 ml), followed by the addition of triethylamine (163 µl, 1.15 mmol) and [dimethylamino-([1,2,3]triazolo[4,5-b]pyridin-3-yloxy)-methylene]-dimethyl-ammonium hexafluoro phosphate (HATU, 219 mg, 0.58 mmol). The reaction mixture was stirred at room temp. for 19 hrs. The crude product was initially purified by chromatography (RP, pHPLC, solvent gradient water + 0.1 vol.% TFA/15-33 vol.% acetonitrile + 0.1 vol.% TFA). Product fractions were combined and basified with saturated sodium bicarbonate solution (5 ml) and, subsequently, extracted twice with ethyl acetate (40 ml each). The combined organic layers were dried over sodium sulfate, filtered with suction, and evaporated in vacuo. Then, the remaining residue was finally purified by flash chromatography on silica gel (4g, solvent gradient: dichloromethane/0-4.5 vol.% ethanol) to yield pure 1-ethyl-2-(hydroxy-diphenyl-methyl)-5-methoxy-1H-imidazo[4,5-b]pyridine-6-carboxylic acid (1-ethyl-1H-[1,2,4]triazol-3-yl)-amide (108.9 mg, purity 96.8%, 0.21 mmol, yield 73.6 %). [M+H]⁺ = 498.2, HPTLC: dichloromethane/ethanol 10:1, R_{f} 0.46.

### Reference compound

Reference compound 3-Ethyl-2-[hydroxy(diphenyl)methyl]-N-[(2R)-2-hydroxypropyl]benzimidazole-5-carboxamide is available as described in WO 2015/175845 (Compound No. 79).

### Table 1

Table 1 which is divided in Tables 1a, 1b, 1c and 1d shows exemplary compounds of the present invention. All compounds depicted in Table 1 including its sub-Tables 1a, 1b, 1c and 1d have been synthesized in accordance or similar to the methods described above for General Syntheses 1 to 3 and Examples 1 to 8.

**Table 1a - Examplary compounds of formula I-b**

| **Compound No.** | **Structure** | **Name** |
|---|---|---|
| 1 | 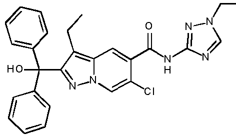 | 6-Chloro-3-ethyl-2-(hydroxy-diphenylmethyl)-pyrazolo[1,5-a]pyridine-5-carboxylic acid (1-ethyl-1H-[1,2,4]triazol-3-yl)-amide |
| | MW 501.0 | ¹H NMR (400 MHz, CD₃OD, ppm) δ = 8.61 (s, 1 H), 8.34 (s, 1 H), 7.86 (br s, 1 H), 7.35-7.27 (m, 10 H), 4.27-4.20 (m, 2 H), 2.64 (q, *J =* 7.6 Hz, 2 H), 1.54-1.48 (m, 3 H), 0.88 (t, *J* = 7.6 Hz, 3 H). |
| | MS [M+H]⁺ 501.1 | |
| 2 | 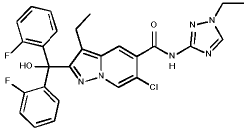 | 2-[Bis-(2-fluoro-phenyl)-hydroxy-methyl]-6-chloro-3-ethyl-pyrazolo[1,5-a]pyridine-5-carboxylic acid (1-ethyl-1H-[1,2,4]triazol-3-yl)-amide |
| | MW 537.0 | 1H NMR (300 MHz, Methanol-d4, ppm) δ 8.59 (s, 1H), 8.35 (s, 1H), 7.88 (s, 1H), 7.46 - 7.26 (m, 4H), 7.22 -7.01 (m, 4H), 4.25 (q, J = 7.3 Hz, 2H), 2.73 (q, J = 7.4 Hz, 2H), 1.51 (d, J = 7.8 Hz, 3H), 0.94 (t, J = 7.5 Hz, 3H) ppm. |
| | MS [M+H]⁺ 537.3 | |
| 3 | 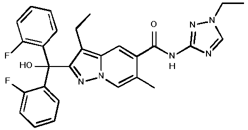 | 2-[Bis-(2-fluoro-phenyl)-hydroxy-methyl]-3-ethyl-6-methyl-pyrazolo[1,5-a]pyridine-5-carboxylic acid (1-ethyl-1H-[1,2,4]triazol-3-yl)-amide |
| | MW 516.6 | ¹H NMR (400 MHz, CD₃OD, ppm) δ = 8.24 (s, 1 H), 8.13 (s, 1 H), 7.36 (s, 1 H), 7.29-7.24 (m, 2 H), 7.20-7.16 (m, 2 H), 7.05-7.01 (m, 2 H), 6.98-6.93 (m, 2 H), 4.15 (q, *J =* 7.2 Hz, 2 H), 2.59 (q, *J =* 7.6 Hz, 2 H), 2.30 (s, 3 H), 1.40 (t, *J* = 7.2 Hz, 3 H), 0.81 (t, *J =* 7.6 Hz, 3 H). |
| | MS [M+H]⁺ 517.3 | |
| 4 | 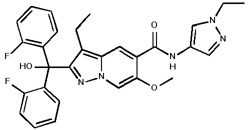 | 2-[Bis-(2-fluoro-phenyl)-hydroxy-methyl]-3-ethyl-6-methoxy-pyrazolo[1,5-a]pyridine-5-carboxylic acid (1-ethyl-1H-pyrazol-4-yl)-amide |
| | MW 531.6 | ¹H NMR (400 MHz, CD₃OD) δ = 8.20 (s, 1 H), 8.08 (s, 2 H), 7.67 (s, 1 H), 7.38-7.27 (m, 4 H), 7.16-7.02 (m, 4 H), 4.18 (q, *J* = 7.2 Hz, 2 H), 3.95 (s, 3 H), 2.69 (q, *J* = 7.2 Hz, 2 H), 1.46 (t, *J =* 7.2 Hz, 3 H), 0.93 (t, *J* = 7.2 Hz, 3 H) ppm. |
| | MS [M+H]⁺ 532.1 | |
| 5 | 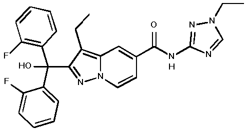 | 2-[Bis-(2-fluoro-phenyl)-hydroxy-methyl]-3-ethyl-pyrazolo[1,5-a]pyridine-5-carboxylic acid (1-ethyl-1H-[1,2,4]triazol-3-yl)-amide |
| | MW 502.5 | ¹H NMR (400 MHz, DMSO-*d₆*, ppm) δ = 10.91 (br s, 1 H), 8.56 (d, *J =* 7.2 Hz, 1 H), 8.46 (s, 1 H), 8.38 (s, 1 H), 7.39-7.34 (m, 4 H), 7.20-7.07 (m, 5 H), 6.55 (s, 1 H), 4.18 (q, *J =* 7.2 Hz, 2 H), 2.69 (q, *J =* 7.6 Hz, 2 H), 1.41 (t, *J =* 7.2 Hz, 3 H), 0.96 (t, *J* = 7.6 Hz, 3 H). |
| | MS [M+H]⁺ 503.4 | |
| 6 | 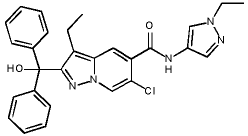 | 6-Chloro-3-ethyl-2-(hydroxy-diphenylmethyl)-pyrazolo[1,5-a]pyridine-5-carboxylic acid (1-ethyl-1H-pyrazol-4-yl)-amide |
| | MW 500.0 | ¹H NMR (400 MHz, CD₃OD, ppm) δ = 8.61 (s, 1 H), 8.09 (s, 1 H), 7.81 (s, 1 H), 7.62 (s, 1 H), 7.36-7.26 (m, 10 H), 4.20 (q, *J =* 7.2 Hz, 2 H), 2.64 (q, *J* = 7.6 Hz, 2 H), 1.48 (t, *J =* 7.2 Hz, 3 H), 0.90 (t, *J* = 7.6 Hz, 3 H). |
| | MS [M+H]⁺ 500.1 | |
| 7 | 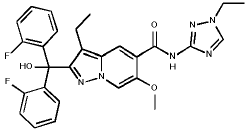 | 2-[Bis-(2-fluoro-phenyl)-hydroxy-methyl]-3-ethyl-6-methoxy-pyrazolo[1,5-a]pyridine-5-carboxylic acid (1-ethyl-1H-[1,2,4]triazol-3-yl)-amide |
| | MW 532.6 | ¹H NMR (400 MHz, CD₃OD, ppm) δ = 8.37 (s, 1 H), 8.28 (br s, 2 H), 7.40-7.27 (m, 4 H), 7.16-7.13 (m, 2 H), 7.08-7.04 (m, 2 H), 4.24 (q, *J =* 7.2 Hz, 2 H), 4.02 (s, 3 H), 2.72 (q, *J =* 7.6 Hz, 2 H), 1.53 (t, *J* = 7.2 Hz, 3 H), 0.93 (t, *J* = 7.6 Hz, 3 H). |
| | MS [M+H]⁺ 533.3 | |
| 8 | 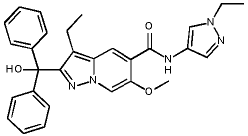 | 3-Ethyl-2-(hydroxy-diphenyl-methyl)-6-methoxy-pyrazolo[1,5-a]pyridine-5-carboxylic acid (1-ethyl-1H-pyrazol-4-yl)-amide |
| | MW 495.6 | ¹H NMR (400 MHz, CD₃OD, ppm) δ = 8.23 (s, 1 H), 8.09-8.07 (m, 2 H), 7.69 (s, 1 H), 7.34-7.29 (m, 10 H), 4.17 (q, *J* = 7.2 Hz, 2 H), 3.97 (s, 3 H), 2.62-2.58 (m, 2 H), 1.47 (t, *J* = 6.8 Hz, 3 H), 0.90 (t, *J* = 6.8 Hz, 3 H). |
| | MS [M+H]⁺ 496.3 | |
| 9 | 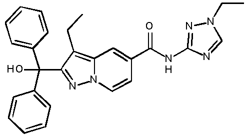 | 3-Ethyl-2-(hydroxy-diphenyl-methyl)-pyrazolo[1,5-a]pyridine-5-carboxylic acid (1-ethyl-1H-[1,2,4]triazol-3-yl)-amide |
| | MW 466.5 | ¹H NMR (400 MHz, CD₃OD) δ = 8.46 (d, *J =* 7.2 Hz, 1 H), 8.36 (s, 1 H), 8.28 (s, 1 H), 7.36-7.26 (m, 11 H), 4.26 (q, *J =* 7.2 Hz, 2 H), 2.69 (q, *J =* 7.2 Hz, 2 H), 1.53 (t, *J* = 7.2 Hz, 3 H), 0.93 (t, *J* = 7.2 Hz, 3 H) ppm. |
| | MS [M+H]⁺ 467.2 | |
| 10 | 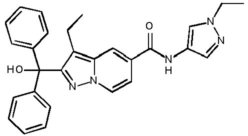 | 3-Ethyl-2-(hydroxy-diphenyl-methyl)-pyrazolo[1,5-a]pyridine-5-carboxylic acid (1-ethyl-1H-pyrazol-4-yl)-amide |
| | MW 465.6 | ¹H NMR (400 MHz, Methanol-d4, ppm) δ = 8.46 (dd, J = 7.3, 0.9 Hz, 1 H), 8.21 (dd, J = 2.0, 1.0 Hz, 1 H), 8.11 (s, 1 H), 7.69 (s, 1 H), 7.40 - 7.23 (m, 11 H), 4.20 (q, J = 7.3 Hz, 2 H), 2.69 (q, J = 7.5 Hz, 2 H), 1.48 (t, J = 7.3 Hz, 3 H), 0.94 (t, J = 7.5 Hz, 3 H). |
| | MS [M+H]⁺ 466.2 | |
| 11 | 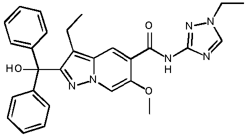 | 3-Ethyl-2-(hydroxy-diphenyl-methyl)-6-methoxy-pyrazolo[1,5-a]pyridine-5-carboxylic acid (1-ethyl-1H-[1,2,4]triazol-3-yl)-amide |
| | MW 496.6 | ¹H NMR (400 MHz, CD₃OD) δ = 8.36 (s, 1 H), 8.29 (s, 1 H), 8.15 (s, 1 H), 7.36-7.25 (m, 10 H), 4.26 (q, *J* = 7.2 Hz, 2 H), 4.03 (s, 3 H), 2.63 (q, *J =* 7.2 Hz, 2 H), 1.53 (t, *J* = 7.2 Hz, 3 H), 0.92 (t, *J* = 7.2 Hz, 3 H) ppm. |
| | MS [M+H]⁺ 497.2 | |
| 12 | 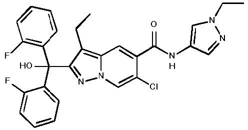 | 2-[Bis-(2-fluoro-phenyl)-hydroxy-methyl]-6-chloro-3-ethyl-pyrazolo[1,5-a]pyridine-5-carboxylic acid (1-ethyl-1H-pyrazol-4-yl)-amide |
| | MW 536.0 | ¹H NMR (400 MHz, CD₃OD, ppm) δ = 8.56 (s, 1 H), 8.06 (s, 1 H), 7.80 (s, 1 H), 7.59 (s, 1 H), 7.39-7.34 (m, 2 H), 7.31-7.27 (m, 2 H), 7.15-7.11 (m, 2 H), 7.08-7.03 (m, 2 H), 4.18 (q, *J =* 7.2 Hz, 2 H), 2.70 (q, *J* = 7.6 Hz, 2 H), 1.46 (t, *J =* 7.6 Hz, 3 H), 0.91 (t, *J =* 7.6 Hz, 3 H). |
| | MS [M+H]⁺ 536.0 | |

**Table 1b Examplary compounds of formula I-c**

| **Compound No.** | **Structure** | **Name** |
|---|---|---|
| 13 | 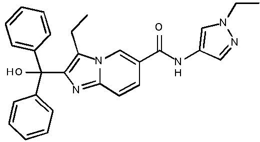 | 3-Ethyl-2-(hydroxy-diphenyl-methyl)-imidazo[1,2-a]pyridine-6-carboxylic acid (1-ethyl-1H-pyrazol-4-yl)-amide |
| | MW 465.6 | ¹H NMR (400 MHz, DMSO-*d₆*, ppm) δ = 10.47 (s, 1 H), 8.84 (s, 1 H), 8.06 (s, 1 H), 7.70-7.67 (m, 1 H), 7.60-7.57 (m, 2 H), 7.35-7.20 (m, 10 H), 6.34 (s, 1 H), 4.13 (q, *J* = 7.2 Hz, 2 H), 2.96(q, *J* = 7.2 Hz, 2 H), 1.36 (t, *J =* 7.2 Hz, 3 H), 0.97 (t, *J* = 7.2 Hz, 3 H). |
| | MS [M+H]⁺ 466.2 | |
| 14 | 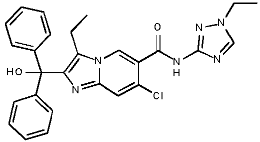 | 7-Chloro-3-ethyl-2-(hydroxy-diphenylmethyl)-imidazo[1,2-a]pyridine-6-carboxylic acid (1-ethyl-1H-[1,2,4]triazol-3-yl)-amide |
| | MW 501.0 | ¹H NMR (400 MHz, CD₃OD, ppm) δ = 8.59 (s, 1 H), 8.34 (s, 1 H), 7.61 (s, 1 H), 7.37-7.27 (m, 10 H), 4.28-4.21 (m, 2 H), 2.86 (q, *J =* 7.2 Hz, 2 H), 1.52-1.49 (m, 3 H), 0.98 (t, *J =* 7.2 Hz, 3 H). |
| | MS [M+H]⁺ 501.2 | |
| 15 | 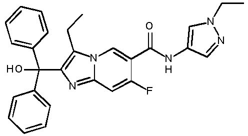 | 3-Ethyl-7-fluoro-2-(hydroxy-diphenylmethyl)-imidazo[1,2-a]pyridine-6-carboxylic acid (1-ethyl-1H-pyrazol-4-yl)-amide |
| | MW 483.5 | ¹H NMR (400 MHz, CD₃OD) δ 8.67 (d, J = 6.7 Hz, 1H), 8.08 (s, 1H), 7.66 (s, 1H), 7.50 (s, 1H), 7.40 - 7.23 (m, 11H), 4.20 (q, J = 7.3 Hz, 2H), 2.84 (q, J = 7.4 Hz, 2H), 1.48 (t, J = 7.3 Hz, 3H), 0.99 (t, J = 7.4 Hz, 3H) ppm. |
| | MS [M+H]⁺ 484.2 | |
| 16 | 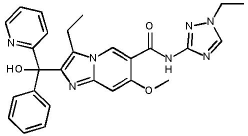 | 3-Ethyl-2-(hydroxy-phenyl-pyridin-2-yl-methyl)-7-methoxy-imidazo[1,2-a]pyridine-6-carboxylic acid (1-ethyl-1H-[1,2,4]triazol-3-yl)-amide |
| | racemate | ¹H NMR (400 MHz, CD₃OD, ppm) δ = 8.80 (br s, 1 H), 8.56 (dd, *J =* 4.8, 0.8 Hz, 1 H), 8.36 (s, 1 H), 7.81-7.77 (m, 1 H), 7.52 (d, *J =* 2.0 Hz, 1 H), 7.41-7.39 (m, 2 H), 7.36-7.30 (m, 4 H), 6.99 (s, 1 H), 4.26 (q, *J =* 7.2 Hz, 2 H), 4.09 (s, 3 H), 2.81 (q, *J* = 7.6 Hz, 2 H), 1.53 (t, *J=* 7.2 Hz, 3 H), 1.03 (t, *J =* 7.6 Hz, 3 H). |
| | MW 497.2 | |
| | MS [M+H]⁺ 498.2 | |
| 17 | 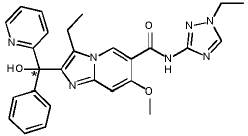 | 3-ethyl-N-(1-ethyl-1,2,4-triazol-3-yl)-2-[hydroxy-phenyl-(2-pyridyl)methyl]-7-methoxy-imidazo[1,2-a]pyridine-6-carboxamide |
| | Isolated enantiomer | ¹H NMR (300 MHz, CD₃OD, ppm) δ = 8.83 (s, 1 H), 8.57 (d, *J =* 5.6 Hz, 1 H), 8.38 (s, 1 H), 7.83-7.78 (m, 1 H), 7.53 (d, *J =* 10.8 Hz, 1 H), 7.43-7.30 (m, 6 H), 7.00 (s, 1 H), 4.26 (q, *J =* 7.5 Hz, 2 H), 4.09 (s, 3 H), 2.82 (q, *J =* 7.2 Hz, 2 H), 1.53 (t, *J =* 7.5 Hz, 3 H), 1.04 (t, *J =* 7.5 Hz, 3 H). |
| | | |
| | MW 497.6 | |
| | MS [M+H]⁺ 498.2 | |
| | | Chiral HPLC: Column: CHIRALPAK ID, 4.6mm*25cm L(5µm); mobile phase, MTBE and EtOH (+ 1% DEA) 70:30, rt 19.03 min. |
| 18 | 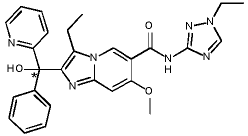 | 3-ethyl-N-(1-ethyl-1,2,4-triazol-3-yl)-2-[hydroxy-phenyl-(2-pyridyl)methyl]-7-methoxy-imidazo[1,2-a]pyridine-6-carboxamide |
| | Isolated enantiomer | ¹H NMR (300 MHz, CD₃OD, ppm) δ = 8.83 (s, 1 H), 8.57 (d, *J =* 6.4 Hz, 1 H), 8.38 (s, 1 H), 7.83-7.78 (m, 1 H), 7.54 (d, *J =* 10.4 Hz, 1 H), 7.43-7.40 (m, 2 H), 7.39-7.29 (m, 4 H), 7.00 (s, 1 H), 4.26 (q, *J =* 7.2 Hz, 2 H), 4.09 (s, 3 H), 2.82 (q, *J =* 7.2 Hz, 2 H), 1.53 (t, *J* = 7.2 Hz, 3 H), 1.04 (t, *J =* 7.5 Hz, 3 H). |
| | MW 497.6 | |
| | MS [M+H]⁺ 498.2 | |
| | | Chiral HPLC: Column: CHIRALPAK ID, 4.6mm*25cm L(5µm); mobile phase, MTBE and EtOH (+ 1% DEA) 70:30, rt 11.05 min. |
| 19 | 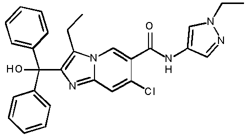 | 7-Chloro-3-ethyl-2-(hydroxy-diphenylmethyl)-imidazo[1,2-a]pyridine-6-carboxylic acid (1-ethyl-1H-pyrazol-4-yl)-amide |
| | MW 500.0 | ¹H NMR (400 MHz, Methanol-d4, ppm) δ = 8.55 (s, 1 H), 8.08 (s, 1 H), 7.60 (s, 2 H), 7.40 - 7.23 (m, 10H), 4.20 (q, J = 7.3 Hz, 2 H), 2.84 (q, J = 7.4 Hz, 2 H), 1.48 (t, J = 7.3 Hz, 3 H), 0.97 (t, J = 7.4 Hz, 3 H). |
| | MS [M+H]⁺ 500.1 | |
| 20 | 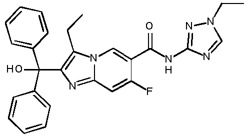 | 3-Ethyl-7-fluoro-2-(hydroxy-diphenylmethyl)-imidazo[1,2-a]pyridine-6-carboxylic acid (1-ethyl-1H-[1,2,4]triazol-3-yl)-amide |
| | MW 484.5 | ¹H NMR (400 MHz, CD₃OD) δ = 8.68 (d, *J* = 6.4 Hz, 1 H), 8.33 (s, 1 H), 7.53-7.23 (m, 11 H), 4.23 (q, *J =* 7.2 Hz, 2 H), 2.82 (q, *J* = 7.2 Hz, 2 H), 1.49 (t, *J* = 7.2 Hz, 3 H), 0.97 (t, *J =* 7.2 Hz, 3 H) ppm. |
| | MS [M+H]⁺ 485.1 | |
| 21 | 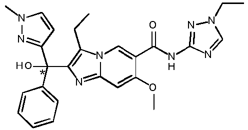 | 3-Ethyl-2-[hydroxy-(1-methyl-1H-pyrazol-3-yl)-phenyl-methyl]-7-methoxy-imidazo[1,2-a]pyridine-6-carboxylic acid (1-ethyl-1H-[1,2,4]triazol-3-yl)-amide |
| | Isolated enantiomer | ¹H NMR (400 MHz, CD₃OD) δ = 8.80 (s, 1 H), 8.36 (s, 1 H), 7.53 (d, *J =* 2.4 Hz, 1 H), 7.47 (d, *J =* 6.8 Hz, 2 H), 7.33-7.25 (m, 3 H), 7.00 (s, 1 H), 6.12 (d, *J* = 2.4 Hz, 1 H), 4.25 (q, *J =* 7.2 Hz, 2 H), 4.10 (s, 3 H), 3.88 (s, 3 H), 2.81-2.79 (m, 2 H), 1.53 (t, *J =* 7.2 Hz, 3 H), 1.00 (t, *J =* 7.2 Hz, 3 H) ppm. |
| | MW 500.6 | |
| | MS [M+H]⁺ 501.2 | |
| | | Chiral HPLC: Column: CHIRALPAK ID, 4.6mm*25cm L(5µm); mobile phase, MTBE and EtOH (+ 1% DEA) 60:40, rt 10.90 min. |
| 22 | 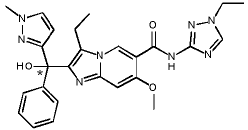 | 3-Ethyl-2-[hydroxy-(1-methyl-1H-pyrazol-3-yl)-phenyl-methyl]-7-methoxy-imidazo[1,2-a]pyridine-6-carboxylic acid (1-ethyl-1H-[1,2,4]triazol-3-yl)-amide |
| | Isolated enantiomer | ¹H NMR (400 MHz, CD₃OD) δ = 8.80 (s, 1 H), 8.36 (s, 1 H), 7.53-7.46 (m, 3 H), 7.34-7.26 (m, 3 H), 7.01 (s, 1 H), 6.12 (d, *J* = 2.0 Hz, 1 H), 4.25 (q, *J =* 7.2 Hz, 2 H), 4.10 (s, 3 H), 3.88 (s, 3 H), 2.87-2.70 (m, 2 H), 1.52 (t, *J =* 7.2 Hz, 3 H), 1.00 (t, *J =* 7.2 Hz, 3 H) ppm. |
| | MW 500.6 | |
| | MS [M+H]⁺ 501.2 | Chiral HPLC: Column: CHIRALPAK ID, 4.6mm*25cm L(5µm); mobile phase, MTBE and EtOH (+ 1% DEA) 60:40, rt 15.54 min. |
| 23 | 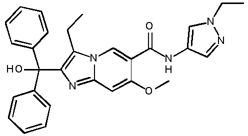 | 3-Ethyl-2-(hydroxy-diphenyl-methyl)-7-methoxy-imidazo[1,2-a]pyridine-6-carboxylic acid (1-ethyl-1H-pyrazol-4-yl)-amide |
| | MW 495.6 | ¹H NMR (300 MHz, CD₃OD, ppm) δ = 8.65 (s, 1 H), 8.07 (s, 1 H), 7.67 (s, 1 H), 7.37-7.27 (m, 10 H), 6.95 (s, 1 H), 4.17 (q, *J =* 7.2 Hz, 2 H), 4.03 (s, 3 H), 2.71 (q, *J =* 7.2 Hz, 2 H), 1.46 (t, *J* = 7.2 Hz, 3 H), 0.95 (t, *J =* 7.2 Hz, 3 H). |
| | MS [M+H]⁺ 496.0 | |
| 24 | 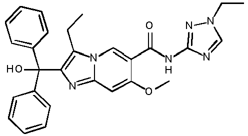 | 3-Ethyl-2-(hydroxy-diphenyl-methyl)-7-methoxy-imidazo[1,2-a]pyridine-6-carboxylic acid (1-ethyl-1H-[1,2,4]triazol-3-yl)-amide |
| | MW 496.6 | ¹H NMR (400 MHz, Methanol-d4) δ 8.80 (s, 1H), 8.36 (s, 1H), 7.55- 7.08 (m, 10H), 7.00 (s, 1H), 4.25 (q, J = 7.3 Hz, 2H), 4.09 (s, 3H), 2.75 (q, J = 7.4 Hz, 2H), 1.52 (t, J = 7.3 Hz, 3H), 0.97 (t, J = 7.4 Hz, 3H) ppm. |
| | MS [M+H]⁺ 497.2 | |
| 25 | 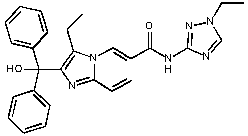 | 3-Ethyl-2-(hydroxy-diphenyl-methyl)-imidazo[1,2-a]pyridine-6-carboxylic acid (1-ethyl-1H-[1,2,4]triazol-3-yl)-amide |
| | MW 466.5 | ¹H NMR (400 MHz, DMSO-d6, ppm) δ = 10.92 (s, 1 H), 8.99 (s, 1 H), 8.45 (s, 1 H), 7.64 (dd, J = 9.5, 1.7 Hz, 1 H), 7.55 (d, J = 9.4 Hz, 1 H), 7.38 - 7.18 (m, 10H), 6.35 (s, 1 H), 4.17 (q, J = 7.2 Hz, 2 H), 2.94 (t, J = 7.4 Hz, 2 H), 1.40 (t, J = 7.3 Hz, 3 H), 0.97 (t, J = 7.4 Hz, 3 H). |
| | MS [M+H]⁺ 467.3 | |
| 26 | 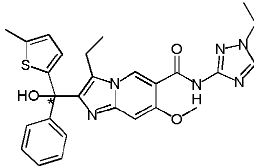 | 3-Ethyl-2-[hydroxy-(5-methyl-thiophen-2-yl)-phenyl-methyl]-7-methoxy-imidazo[1,2-a]pyridine-6-carboxylic acid (1-ethyl-1H-[1,2,4]triazol-3-yl)-amide |
| | Isolated enantiomer | ¹H NMR (400 MHz, CD₃OD, ppm) δ = 8.81 (s, 1 H), 8.37 (s, 1 H), 7.48-7.46 (m, 2 H), 7.32-7.27 (m, 3 H), 7.01 (s, 1 H), 6.62-6.58 (m, 2 H), 4.26 (q, *J =* 7.2 Hz, 2 H), 4.10 (s, 3 H), 2.86-2.76 (m, 2 H), 2.45 (s, 3 H), 1.53 (t, *J =* 7.2 Hz, 3 H), 1.02 (t, *J =* 7.6 Hz, 3 H); Chiral SFC: |
| | MW 516.6 | |
| | MS [M+H]⁺ 517.2 | Column, CHIRALPAK AD-H, 4.6mm*25cm, 5µm; mobile phase, CO₂ and EtOH (+0.5% DEA) 60:40, rt 10.75 min. |
| 27 | 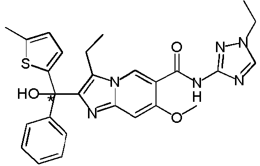 | 3-Ethyl-2-[hydroxy-(5-methyl-thiophen-2-yl)-phenyl-methyl]-7-methoxy-imidazo[1,2-a]pyridine-6-carboxylic acid (1-ethyl-1H-[1,2,4]triazol-3-yl)-amide |
| | Isolated enantiomer | ¹H NMR (400 MHz, CD₃OD, ppm) δ = 8.81 (s, 1 H), 8.36 (s, 1 H), 7.48-7.46 (m, 2 H), 7.32-7.27 (m, 3 H), 7.01 (s, 1 H), 6.62-6.58 (m, 2 H), 4.26 (q, *J =* 7.2 Hz, 2 H), 4.10 (s, 3 H), 2.86-2.76 (m, 2 H), 2.45 (s, 3 H), 1.53 (t, *J =* 7.2 Hz, 3 H), 1.02 (t, *J =* 7.6 Hz, 3 H). |
| | MW 516.6 | |
| | MS [M+H]⁺ 517.2 | Chiral SFC: Column, CHIRALPAK AD-H, 4.6mm*25cm, 5µm; mobile phase, CO₂ and EtOH (+0.5% DEA) 60:40, rt 6.17 min. |

**Table 1c Examplary compounds of formula I-a**

| **Compound No.** | **Structure** | **Name** |
|---|---|---|
| 28 | 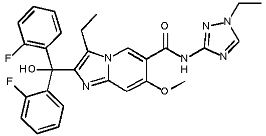 | 2-[Bis-(2-fluoro-phenyl)-hydroxy-methyl]-3-ethyl-7-methoxy-imidazo[1,2-a]pyridine-6-carboxylic acid (1-ethyl-1H-[1,2,4]triazol-3-yl)-amide |
| | MW 532.6 | ¹H NMR (400 MHz, CD₃OD) δ = 8.81 (s, 1 H), 8.36 (s, 1 H), 7.42-7.33 (m, 2 H), 7.33 (t, *J* = 7.6 Hz, 2 H), 7.17-7.06 (m, 4 H), 7.01 (s, 1 H), 4.26 (q, *J* = 7.2 Hz, 2 H), 4.23 (s, 3 H), 2.90 (q, *J* = 7.2 Hz, 2 H), 1.53 (t, *J* = 7.2 Hz, 3 H), 0.94 (t, *J =* 7.2 Hz, 3 H) ppm. |
| | MS [M+H]⁺ 533.1 | |
| 29 | 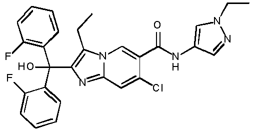 | 2-[Bis-(2-fluoro-phenyl)-hydroxy-methyl]-7-chloro-3-ethyl-imidazo[1,2-a]pyridine-6-carboxylic acid (1-ethyl-1H-pyrazol-4-yl)-amide |
| | MW 536.0 | ¹H NMR (300 MHz, CD₃OD, ppm) δ = 8.57 (s, 1 H), 8.10 (s, 1 H), 7.62 (s, 2 H), 7.44-7.29 (m, 4 H), 7.19-7.06 (m, 4 H), 4.21 (q, *J* = 7.2 Hz, 2 H), 2.96 (q, *J = 7.5* Hz, 2 H), 1.48 (t, J= 7.5 Hz, 3 H), 0.93 (t, *J* = 7.2 Hz, 3 H). |
| | MS [M+H]⁺ 536.1 | |
| 30 | 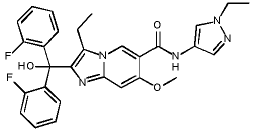 | 2-[Bis-(2-fluoro-phenyl)-hydroxy-methyl]-3-ethyl-7-methoxy-imidazo[1,2-a]pyridine-6-carboxylic acid (1-ethyl-1H-pyrazol-4-yl)-amide |
| | MW 531.6 | ¹H NMR (400 MHz, Methanol-d4, ppm) |
| | | δ = 8.68 (s, 1 H), 8.09 (d, J = 0.7 Hz, 1 H), 7.69 (d, J = 0.8 Hz, 1 H), 7.44 - 7.33 (m, 2 H), 7.32-7.27 (m, 2 H), 7.19 - 7.03 (m, 4 H), 6.97 (s, 1 H), 4.19 (q, J = 7.3 Hz, 2 H), 4.04 (s, 3 H), 2.88 (q, J = 7.5 Hz, 2 H), 1.48 (t, J = 7.3 Hz, 3 H), 0.94 (t, J = 7.4 Hz, 3 H). |
| | MS [M+H]⁺ 532.1 | |
| 31 | 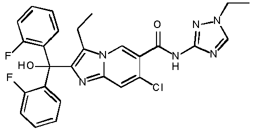 | 2-[Bis-(2-fluoro-phenyl)-hydroxy-methyl]-7-chloro-3-ethyl-imidazo[1,2-a]pyridine-6-carboxylic acid (1-ethyl-1H-[1,2,4]triazol-3-yl)-amide |
| | MW 537.0 | ¹H NMR (400 MHz, Methanol-d4, ppm) δ = 8.59 (s, 1 H), 8.33 (s, 1 H), 7.61 (s, 1 H), 7.42-7.37 (m, 2 H), 7.31-7.28 (m, 2 H), 7.18-7.07 (m, 4 H), 4.24 (d, J = 7.4 Hz, 2 H) , 2.96 (q, J = 7.4 Hz, 2H), 1.52-1.47 (m, 3 H), 0.94 (t, J = 7.4 Hz, 3 H). |
| | MS [M+H]⁺ 537.1 | |
| 32 | 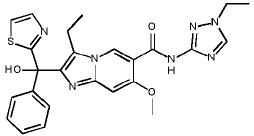 | 3-ethyl-N-(1-ethyl-1H-1,2,4-triazol-3-yl)-2-[hydroxy(phenyl)(1,3-thiazol-2-yl)methyl]-7-methoxyimidazo[1,2-a]pyridine-6-carboxamide |
| | racemate | 1H NMR (500 MHz, DMSO-d6) δ 10.46 (s, 1H), 8.45 - 8.41 (m, 1H), 8.40 - 8.36 (m, 1H), 7.74 (d, J = 3.3 Hz, 1H), 7.64 (d, J = 3.2 Hz, 1H), 7.57 - 7.54 (m, 2H), 7.32 - 7.28 (m, 2H), 7.26 - 7.23 (m, 1H), 7.04 - 7.02 (m, 1H), 7.01 (s, 1H), 4.14 (q, J = 7.2 Hz, 2H), 3.90 - 3.83 (m, 3H), 2.67 (q, J = 7.6 Hz, 2H), 1.37 (t, J = 7.3 Hz, 3H), 0.84 (t, J = 7.4 Hz, 3H). |
| | MW 503.6 | |
| | MS [M+H]⁺ 504.2 | |
| 33 | 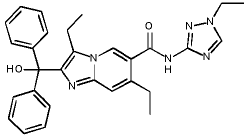 | 3,7-diethyl-N-(1-ethyl-1H-1,2,4-triazol-3-yl)-2-(hydroxydiphenylmethyl)imidazo[1,2-a]pyridine-6-carboxamide |
| | racemate | 1H NMR (500 MHz, DMSO-d6) δ 10.82 (s, 1H), 8.40 (s, 1H), 8.39 - 8.37 (m, 1H), 7.34 - 7.31 (m, 5H), 7.29 - 7.25 (m, 4H), 7.23 - 7.19 (m, 2H), 6.22 (s, 1H), 4.15 (q, J = 7.3 Hz, 2H), 2.85 (q, J = 7.4 Hz, 2H), 2.78 (q, J = 7.4 Hz, 2H), 1.39 (t, J = 7.3 Hz, 3H), 1.17 (t, J = 7.4 Hz, 3H), 0.93 (t, J = 7.4 Hz, 3H). |
| | MW 494.6 | |
| | MS [M+H]⁺ 495.2 | |

**Table 1d**

| **Compound No.** | **Structure** | **Name** |
|---|---|---|
| 34 | 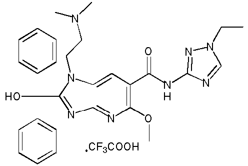 | 1-[2-(dimethylamino)ethyl]-N-(1-ethyl-1H-1,2,4-triazol-3-yl)-2-(hydroxydiphenylmethyl)-5-methoxy-1H-imidazo[4,5-b]pyridine-6-carboxamide trifluoroacetate |
| | MW 540.6 | 1H NMR (500 MHz, DMSO-d6, TFA) δ 9.03 - 8.95 (m, 1H), 8.56 - 8.53 (m, 1H), 7.38 - 7.28 (m, 10H), 4.59 - 4.54 (m, 2H), 4.20 (q, J = 7.1 Hz, 2H), 3.98 (s, 3H), 3.33 - 3.27 (m, 2H), 2.77 (s, 6H), 1.40 (t, J = 7.3 Hz, 3H). |
| | MS [M+H]⁺ 541.3 | |
| 35 | 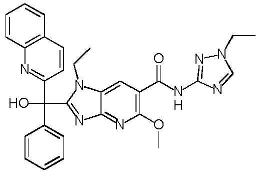 | 1-ethyl-N-(1-ethyl-1H-1,2,4-triazol-3-yl)-2-[hydroxy(phenyl)(quinolin-2-yl)methyl]-5-methoxy-1H-imidazo[4,5-b]pyridine-6-carboxamide |
| | racemate MW 548.6 | 1H NMR (500 MHz, DMSO-d6) δ 10.37 (s, 1H), 8.44 - 8.38 (m, 2H), 8.38 - 8.34 (m, 1H), 8.03 - 8.00 (m, 2H), 7.81 - 7.77 (m, 1H), 7.79 - 7.66 (m, 1H), 7.71 (d, J = 8.7 Hz, 1H), 7.67 - 7.63 (m, 1H), 7.47 - 7.43 (m, 2H), 7.40 - 7.36 (m, 2H), 7.35 - 7.31 (m, 1H), 4.31 - 4.17 (m, 2H), 4.15 (q, J = 7.2 Hz, 2H), 4.00 - 3.91 (m, 3H), 1.39 (t, J = 7.2 Hz, 3H), 1.09 (t, J = 7.1 Hz, 3H). |
| | MS [M+H]⁺ 549.3 | |
| 36 | 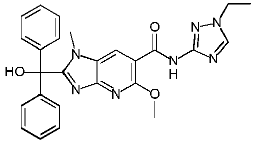 | N-(1-ethyl-1H-1,2,4-triazol-3-yl)-2-(hydroxydiphenylmethyl)-5-methoxy-1-methyl-1H-imidazo[4,5-b]pyridine-6-carboxamide |
| | MW 483.5 | 1H NMR (500 MHz, DMSO-d6) δ 10.35 (s, 1H), 8.44 - 8.38 (m, 1H), 8.38 - 8.34 (m, 1H), 7.37 - 7.33 (m, 4H), 7.32 - 7.27 (m, 7H), 4.15 (q, J = 7.2 Hz, 2H), 4.02 - 3.94 (m, 3H), 3.63 (s, 3H), 1.39 (t, J = 7.2 Hz, 3H). |
| | MS [M+H]⁺ 484.2 | |
| 37 | 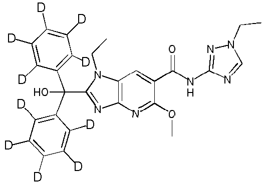 | 1-ethyl-N-(1-ethyl-1H-1,2,4-triazol-3-yl)-2-{hydroxybis[(2,3,4,5,6-²H5)phenyl]methyl}-5-methoxy-1H-imidazo[4,5-b]pyridine-6-carboxamide |
| | MW 507.6 | 1H NMR (500 MHz, DMSO-d6) δ 10.33 (s, 1H), 8.42 - 8.37 (m, 1H), 8.35 - 8.29 (m, 1H), 7.26 (s, 1H), 4.28 (q, J = 7.0 Hz, 2H), 4.15 (q, J = 7.2 Hz, 2H), 4.03 - 3.92 (m, 3H), 1.39 (t, J = 7.3 Hz, 3H), 1.03 (t, J = 7.1 Hz, 3H). |
| | MS [M+H]⁺ 508.3 | |
| 38 | 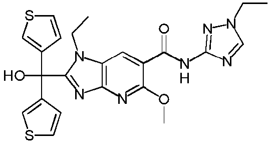 | 1-ethyl-N-(1-ethyl-1H-1,2,4-triazol-3-yl)-2-[hydroxybis(thiophen-3-yl)methyl]-5-methoxy-1H-imidazo[4,5-b]pyridine-6-carboxamide |
| | MW 509.6 | 1H NMR (500 MHz, DMSO-d6) δ 10.34 - 10.31 (m, 1H), 8.42 - 8.37 (m, 1H), 8.34 - 8.30 (m, 1H), 7.49 (dd, J = 5.0, 3.0 Hz, 2H), 7.25 (dd, J = 3.0, 1.4 Hz, 2H), 7.23 - 7.20 (m, 1H), 7.07 (dd, J = 5.1, 1.4 Hz, 2H), 4.37 (q, J = 7.1 Hz, 2H), 4.15 (q, J = 7.2 Hz, 2H), 4.03 - 3.95 (m, 3H), 1.39 (t, J = 7.2 Hz, 3H), 1.01 (t, J = 7.1 Hz, 3H). |
| | MS [M+H]⁺ 510.1 | |
| 39 | 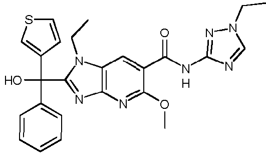 | 1-ethyl-N-(1-ethyl-1H-1,2,4-triazol-3-yl)-2-[hydroxy(phenyl)(thiophen-3-yl)methyl]-5-methoxy-1H-imidazo[4,5-b]pyridine-6-carboxamide |
| | Racemate MW 503.6 | 1H NMR (500 MHz, DMSO-d6) δ 10.34 (s, 1H), 8.42 - 8.37 (m, 1H), 8.34 - 8.29 (m, 1H), 7.48 (dd, J = 5.0, 3.1 Hz, 1H), 7.38 - 7.34 (m, 4H), 7.33 - 7.28 (m, 1H), 7.30 - 7.22 (m, 1H), 7.14 - 7.12 (m, 2H), 4.39 - 4.21 (m, 2H), 4.15 (q, J = 7.3 Hz, 2H), 4.04 - 3.95 (m, 3H), 1.39 (t, J = 7.2 Hz, 3H), 0.98 (t, J = 7.1 Hz, 3H). |
| | MS [M+H]⁺ 504.2 | |
| 40 | 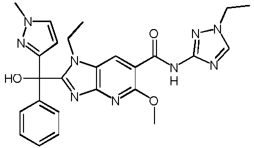 | 1-ethyl-N-(1-ethyl-1H-1,2,4-triazol-3-yl)-2-[hydroxy(1-methyl-1H-pyrazol-3-yl)phenylmethyl]-5-methoxy-1H-imidazo[4,5-b]pyridine-6-carboxamide |
| | Racemate MW 501.5 | 1H NMR (500 MHz, DMSO-d6) δ 10.33 (s, 1H), 8.42 - 8.37 (m, 1H), 8.33 - 8.27 (m, 1H), 7.66 (d, J = 2.2 Hz, 1H), 7.44 - 7.40 (m, 2H), 7.36 - 7.31 (m, 2H), 7.30 - 7.25 (m, 1H), 7.14 - 6.86 (m, 1H), 6.23 (d, J = 2.3 Hz, 1H), 4.27 (q, J = 7.1 Hz, 2H), 4.15 (q, J = 7.3 Hz, 2H), 4.03 - 3.93 (m, 3H), 3.78 (s, 3H), 1.39 (t, J = 7.3 Hz, 3H), 1.01 (t, J = 7.1 Hz, 3H). |
| | MS [M+H]⁺ 502.2 | |
| 41 | 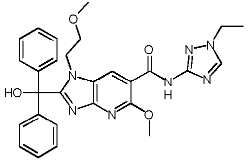 | N-(1-ethyl-1H-1,2,4-triazol-3-yl)-2-(hydroxydiphenylmethyl)-5-methoxy-1-(2-methoxyethyl)-1H-imidazo[4,5-b]pyridine-6-carboxamide |
| | MW 526.6 | 1H NMR (700 MHz, DMSO-d6) δ 10.29 (s, 1H), 8.42 - 8.39 (m, 1H), 8.39 - 8.34 (m, 1H), 7.38 - 7.34 (m, 4H), 7.33 - 7.30 (m, 6H), 7.30 - 7.27 (m, 1H), 4.39 (t, J = 5.5 Hz, 2H), 4.15 (q, J = 7.3 Hz, 2H), 4.04 - 3.94 (m, 3H), 3.34 (t, J = 5.6 Hz, 2H), 3.08 (s, 3H), 1.39 (t, J = 7.2 Hz, 3H). |
| | MS [M+H]⁺ 528.3 | |
| 42 | 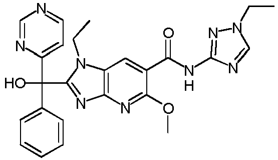 | 1-ethyl-N-(1-ethyl-1H-1 ,2,4-triazol-3-yl)-2-[hydroxy(phenyl)(pyrimidin-4-yl)methyl]-5-methoxy-1H-imidazo[4,5-b]pyridine-6-carboxamide |
| | Racemate MW 499.5 | 1H NMR (700 MHz, DMSO-d6) δ 10.36 (s, 1H), 9.17 (d, J = 1.4 Hz, 1H), 8.86 (d, J = 5.3 Hz, 1H), 8.45 - 8.37 (m, 1H), 8.37 - 8.29 (m, 1H), 7.69 (dd, J = 5.4, 1.4 Hz, 1H), 7.69 - 7.66 (m, 1H), 7.46 - 7.43 (m, 2H), 7.41 - 7.37 (m, 2H), 7.35 - 7.32 (m, 1H), 4.24 - 4.11 (m, 4H), 4.03 - 3.90 (m, 3H), 1.43 - 1.34 (m, 3H), 1.04 (t, J = 7.1 Hz, 3H). |
| | MS [M+H]⁺ 500.3 | |
| 43 | 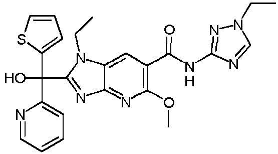 | 1-ethyl-N-(1-ethyl-1H-1,2,4-triazol-3-yl)-2-[hydroxy(pyridin-2-yl)(thiophen-2-yl)methyl]-5-methoxy-1H-imidazo[4,5-b]pyridine-6-carboxamide |
| | Racemate MW 504.6 | 1H NMR (500 MHz, DMSO-d6) δ 10.39 (s, 1H), 8.55 - 8.52 (m, 1H), 8.44 - 8.39 (m, 1H), 8.36 - 8.31 (m, 1H), 7.97 - 7.92 (m, 1H), 7.75 - 7.72 (m, 1H), 7.53 - 7.51 (m, 1H), 7.43 - 7.40 (m, 1H), 7.01 - 6.98 (m, 2H), 4.33 - 4.17 (m, 2H), 4.15 (q, J = 7.1 Hz, 2H), 4.03 - 3.95 (m, 3H), 1.39 (t, J = 7.3 Hz, 3H), 0.99 (t, J = 7.1 Hz, 3H). |
| | MS [M+H]⁺ 505.2 | |
| 44 | 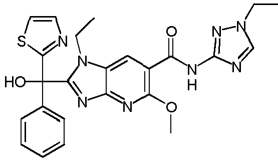 | 1-ethyl-N-(1-ethyl-1H-1,2,4-triazol-3-yl)-2-[hydroxy(phenyl)(1,3-thiazol-2-yl)methyl]-5-methoxy-1H-imidazo[4,5-b]pyridine-6-carboxamide |
| | Racemate MW 504.6 | 1H NMR (700 MHz, DMSO-d6) δ 10.42 - 10.34 (m, 1H), 8.44 - 8.36 (m, 1H), 8.36 - 8.29 (m, 1H), 7.79 (d, J = 3.2 Hz, 1H), 7.78 (d, J = 3.2 Hz, 1H), 7.53 - 7.51 (m, 2H), 7.40 - 7.37 (m, 2H), 7.35 - 7.32 (m, 1H), 4.27 - 4.22 (m, 2H), 4.18 - 4.12 (m, 2H), 4.05 - 3.93 (m, 3H), 1.41 - 1.36 (m, 3H), 0.94 (t, J = 7.2 Hz, 3H). |
| | MS [M+H]⁺ 505.2 | |
| 45 | 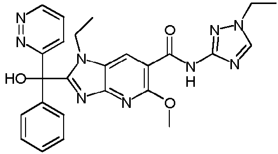 | 1-ethyl-N-(1-ethyl-1H-1,2,4-triazol-3-yl)-2-[hydroxy(phenyl)(pyridazin-3-yl)methyl]-5-methoxy-1H-imidazo[4,5-b]pyridine-6-carboxamide |
| | Racemate MW 499.5 | 1H NMR (500 MHz, DMSO-d6) δ 10.36 (s, 1H), 9.21 (dd, J = 4.8, 1.6 Hz, 1H), 8.41 - 8.38 (m, 1H), 8.37 - 8.33 (m, 1H), 7.84 (dd, J = 8.7, 1.6 Hz, 1H), 7.80 (s, 1H), 7.76 (dd, J = 8.7, 4.9 Hz, 1H), 7.43 - 7.36 (m, 4H), 7.36 - 7.32 (m, 1H), 4.27 - 4.12 (m, 4H), 4.01 - 3.92 (m, 3H), 1.39 (t, J = 7.3 Hz, 3H), 1.04 (t, J = 7.1 Hz, 3H). |
| | MS [M+H]⁺ 500.2 | |
| 46 | 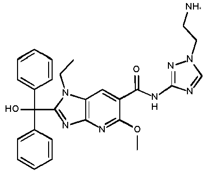 | N-[1-(2-aminoethyl)-1H-1,2,4-triazol-3-yl]-1-ethyl-2-(hydroxydiphenylmethyl)-5-methoxy-1H-imidazo[4,5-b]pyridine-6-carboxamide |
| | MW 512.2 | 1H NMR (700 MHz, DMSO-d6) δ 10.42 (s, 1H), 8.41 - 8.38 (m, 1H), 8.34 - 8.30 (m, 1H), 8.20 (s, 1H), 7.38 - 7.34 (m, 4H), 7.33 - 7.27 (m, 7H), 7.02 - 5.60 (m, 1H), 4.28 (q, J = 7.1 Hz, 2H), 4.22 - 4.16 (m, 2H), 4.03 - 3.94 (m, 3H), 3.08 - 3.02 (m, 2H), 1.02 (t, J = 7.1 Hz, 3H). |
| | MS [M+H]⁺ 513.2 | |
| 47 | 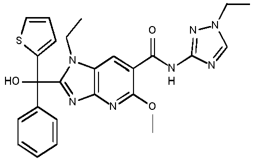 | 1-ethyl-N-(1-ethyl-1H-1,2,4-triazol-3-yl)-2-[hydroxy(phenyl)(thiophen-2-yl)methyl]-5-methoxy-1H-imidazo[4,5-b]pyridine-6-carboxamide |
| | Racemate MW 503.6 | 1H NMR (500 MHz, DMSO-d6) δ 10.36 (s, 1H), 8.41 - 8.37 (m, 1H), 8.34 - 8.29 (m, 1H), 7.67 - 7.60 (m, 1H), 7.49 (dd, J = 5.1, 1.3 Hz, 1H), 7.41 - 7.35 (m, 4H), 7.33 - 7.29 (m, 1H), 6.98 (dd, J = 5.1, 3.6 Hz, 1H), 6.92 (dd, J = 3.6, 1.3 Hz, 1H), 4.39 - 4.30 (m, 1H), 4.28 - 4.21 (m, 1H), 4.15 (q, J = 7.3 Hz, 2H), 4.04 - 3.95 (m, 3H), 1.38 (t, J = 7.3 Hz, 3H), 0.97 (t, J = 7.1 Hz, 3H). |
| | MS [M+H]⁺ 504.1 | |
| 48 | 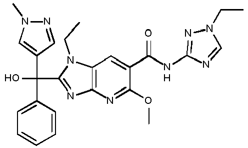 | 1-ethyl-N-(1-ethyl-1H-1,2,4-triazol-3-yl)-2-[hydroxy(1-methyl-1H-pyrazol-4-yl)phenylmethyl]-5-methoxy-1H-imidazo[4,5-b]pyridine-6-carboxamide |
| | Racemate MW 501.6 | 1H NMR (500 MHz, DMSO-d6) δ 10.32 (s, 1H), 8.41 - 8.38 (m, 1H), 8.32 - 8.27 (m, 1H), 7.50 - 7.48 (m, 1H), 7.39 - 7.33 (m, 4H), 7.30 - 7.26 (m, 2H), 7.05 (s, 1H), 4.41 - 4.32 (m, 1H), 4.25 - 4.17 (m, 1H), 4.15 (q, J = 7.2 Hz, 2H), 4.05 - 3.96 (m, 3H), 3.81 (s, 3H), 1.39 (t, J = 7.2 Hz, 3H), 0.94 (t, J = 7.1 Hz, 3H). |
| | MS [M+H]⁺ 502.2 | |
| 49 | 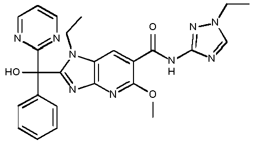 | 1-ethyl-N-(1-ethyl-1H-1,2,4-triazol-3-yl)-2-[hydroxy(phenyl)(pyrimidin-2-yl)methyl]-5-methoxy-1H-imidazo[4,5-b]pyridine-6-carboxamide |
| | Racemate MW 499.5 | 1H NMR (500 MHz, DMSO-d6) δ 10.34 (s, 1H), 8.88 (d, J = 4.9 Hz, 2H), 8.39 (s, 1H), 8.35 - 8.28 (m, 1H), 7.51 (t, J = 4.9 Hz, 1H), 7.48 - 7.44 (m, 2H), 7.38 - 7.33 (m, 2H), 7.33 - 7.28 (m, 1H), 7.27 - 7.19 (m, 1H), 4.24 - 4.10 (m, 4H), 3.95 (s, 3H), 1.39 (t, J = 7.2 Hz, 3H), 1.05 (t, J = 7.1 Hz, 3H). |
| | MS [M+H]⁺ 500.2 | |
| 50 | 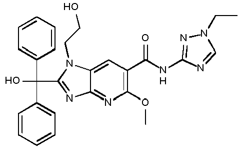 | N-(1-ethyl-1H-1,2,4-triazol-3-yl)-2-(hydroxydiphenylmethyl)-1-(2-hydroxyethyl)-5-methoxy-1H-imidazo[4,5-b]pyridine-6-carboxamide |
| | MW 513.6 | 1H NMR (500 MHz, DMSO-d6) δ 10.30 (s, 1H), 8.47 - 8.41 (m, 1H), 8.40 (s, 1H), 7.38 - 7.33 (m, 4H), 7.33 - 7.28 (m, 6H), 7.26 (s, 1H), 4.97 (t, J = 5.3 Hz, 1H), 4.24 (t, J = 5.7 Hz, 2H), 4.15 (q, J = 7.3 Hz, 2H), 3.99 (s, 3H), 3.50 (q, J = 5.5 Hz, 2H), 1.39 (t, J = 7.3 Hz, 3H). |
| | MS [M+H]⁺ 514.2 | |
| 51 | 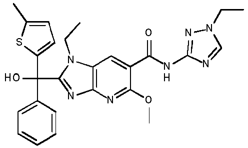 | 1-ethyl-N-(1-ethyl-1H-1,2,4-triazol-3-yl)-2-[hydroxy(5-methylthiophen-2-yl)phenylmethyl]-5-methoxy-1H-imidazo[4,5-b]pyridine-6-carboxamide |
| | Racemate MW 517.6 | 1H NMR (500 MHz, DMSO-d6) δ 10.35 (s, 1H), 8.43 - 8.38 (m, 1H), 8.33 - 8.29 (m, 1H), 7.56 - 7.45 (m, 1H), 7.40 - 7.34 (m, 4H), 7.33 - 7.29 (m, 1H), 6.66 - 6.63 (m, 2H), 4.40 - 4.32 (m, 1H), 4.28 - 4.19 (m, 1H), 4.15 (q, J = 7.3 Hz, 2H), 4.03 - 3.97 (m, 3H), 2.43 - 2.40 (m, 3H), 1.39 (t, J = 7.2 Hz, 3H), 0.99 (t, J = 7.1 Hz, 3H). |
| | MS [M+H]⁺ 518.1 | |
| 52 | 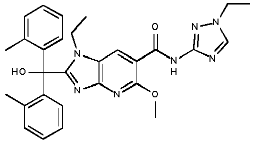 | 1-ethyl-N-(1-ethyl-1H-1,2,4-triazol-3-yl)-2-[hydroxybis(2-methylphenyl)methyl]-5-methoxy-1H-imidazo[4,5-b]pyridine-6-carboxamide |
| | MW 525.6 | 1H NMR (500 MHz, DMSO-d6) δ 10.33 (s, 1H), 8.42 - 8.38 (m, 1H), 8.36 - 8.31 (m, 1H), 7.27 (td, J = 7.3, 1.3 Hz, 2H), 7.23 - 7.20 (m, 2H), 7.17 - 7.13 (m, 2H), 7.00 - 6.97 (m, 2H), 6.87 - 6.82 (m, 1H), 4.24 (q, J = 7.0 Hz, 2H), 4.15 (q, J = 7.3 Hz, 2H), 4.00 - 3.95 (m, 3H), 2.03 (s, 6H), 1.39 (t, J = 7.3 Hz, 3H), 1.01 (t, J = 7.1 Hz, 3H). |
| | MS [M+H]⁺ 526.2 | |
| 53 | 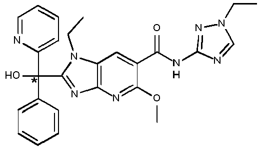 | 1-ethyl-N-(1-ethyl-1H-1,2,4-triazol-3-yl)-2-[hydroxy(phenyl)(pyridin-2-yl)methyl]-5-methoxy-1H-imidazo[4,5-b]pyridine-6-carboxamide |
| | Isolated enantiomer MW 498.5 | 1H NMR (500 MHz, DMSO-d6) δ 10.34 (s, 1H), 8.55 - 8.52 (m, 1H), 8.42 - 8.37 (m, 1H), 8.35 - 8.30 (m, 1H), 7.89 - 7.84 (m, 1H), 7.58 - 7.55 (m, 1H), 7.45 (s, 1H), 7.44 - 7.41 (m, 2H), 7.39 - 7.33 (m, 3H), 7.32 - 7.28 (m, 1H), 4.23 - 4.12 (m, 4H), 3.97 (s, 3H), 1.39 (t, J = 7.3 Hz, 3H), 1.04 (t, J = 7.1 Hz, 3H). |
| | MS [M+H]⁺ 499.2 | |
| | | Chiral SFC: column: Lux Amylose-1; solvents: CO₂, 40% iPrOH + 0.5% diethylamine; rt 5.09 min |
| 54 | 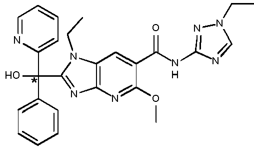 | 1-ethyl-N-(1-ethyl-1H-1,2,4-triazol-3-yl)-2-[hydroxy(phenyl)(pyridin-2-yl)methyl]-5-methoxy-1H-imidazo[4,5-b]pyridine-6-carboxamide |
| | Isolated enantiomer MW 498.5 | 1H NMR (500 MHz, DMSO-d6) δ 10.34 (s, 1H), 8.55 - 8.52 (m, 1H), 8.42 - 8.37 (m, 1H), 8.35 - 8.30 (m, 1H), 7.89 - 7.84 (m, 1H), 7.58 - 7.55 (m, 1H), 7.45 (s, 1H), 7.44 - 7.41 (m, 2H), 7.39 - 7.33 (m, 3H), 7.32 - 7.28 (m, 1H), 4.23 - 4.12 (m, 4H), 3.97 (s, 3H), 1.39 (t, J = 7.2 Hz, 3H), 1.04 (t, J = 7.1 Hz, 3H). |
| | MS [M+H]⁺ 499.2 | |
| | | Chiral SFC: column: Lux Amylose-1; solvents: CO₂, 40% iPrOH + 0.5% diethylamine; rt 2.60 min. |
| 55 | 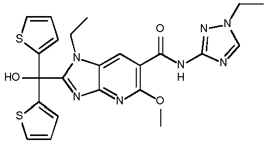 | 1-ethyl-N-(1-ethyl-1H-1,2,4-triazol-3-yl)-2-[hydroxybis(thiophen-2-yl)methyl]-5-methoxy-1H-imidazo[4,5-b]pyridine-6-carboxamide |
| | MW 509.6 | 1H NMR (500 MHz, DMSO-d6) δ 10.36 (s, 1H), 8.42 - 8.37 (m, 1H), 8.36 - 8.32 (m, 1H), 7.89 (s, 1H), 7.52 (dd, J = 5.0, 1.3 Hz, 2H), 6.99 (dd, J = 5.0, 3.6 Hz, 2H), 6.96 (dd, J = 3.6, 1.3 Hz, 2H), 4.43 (q, J = 7.1 Hz, 2H), 4.15 (q, J = 7.2 Hz, 2H), 4.04 - 3.95 (m, 3H), 1.39 (t, J = 7.2 Hz, 3H), 1.07 (t, J = 7.1 Hz, 3H). |
| | MS [M+H]⁺ 510.1 | |
| 56 | 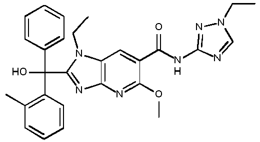 | 1-ethyl-N-(1-ethyl-1H-1,2,4-triazol-3-yl)-2-[hydroxy(2-methylphenyl)phenylmethyl]-5-methoxy-1H-imidazo[4,5-b]pyridine-6-carboxamide |
| | Racemate MW 511.6 | 1H NMR (500 MHz, DMSO-d6) δ 10.34 (s, 1H), 8.43 - 8.39 (m, 1H), 8.35 - 8.29 (m, 1H), 7.41 - 7.30 (m, 5H), 7.27 - 7.20 (m, 2H), 7.24 - 7.06 (m, 1H), 7.12 - 7.07 (m, 1H), 6.73 - 6.70 (m, 1H), 4.32 (q, J = 7.2 Hz, 2H), 4.15 (q, J = 7.3 Hz, 2H), 4.01 - 3.94 (m, 3H), 2.07 (s, 3H), 1.42 - 1.35 (m, 3H), 1.08 (t, J = 7.1 Hz, 3H). |
| | MS [M+H]⁺ 512.3 | |
| 57 | 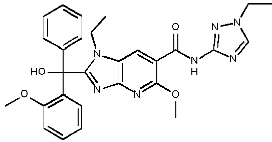 | 1-ethyl-N-(1-ethyl-1H-1,2,4-triazol-3-yl)-2-[hydroxy(2-methoxyphenyl)phenylmethyl]-5-methoxy-1H-imidazo[4,5-b]pyridine-6-carboxamide |
| | Racemate MW 527.6 | 1H NMR (500 MHz, DMSO-d6) δ 10.29 (s, 1H), 8.39 (s, 1H), 8.32 - 8.29 (m, 1H), 7.44 - 7.40 (m, 2H), 7.38 - 7.29 (m, 4H), 7.08 - 7.05 (m, 1H), 6.93 - 6.89 (m, 1H), 6.87 (dd, J = 7.8, 2.0 Hz, 1H), 6.59 (s, 1H), 4.34 - 4.26 (m, 2H), 4.15 (q, J = 7.3 Hz, 2H), 4.02 - 3.95 (m, 3H), 3.50 (s, 3H), 1.39 (t, J = 7.2 Hz, 3H), 1.06 (t, J = 7.1 Hz, 3H). |
| | MS [M+H]⁺ 528.2 | |
| 58 | 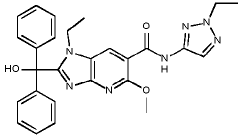 | 1-ethyl-N-(2-ethyl-2H-1,20,3-triazol-4-yl)-2-(hydroxydiphenylmethyl)-5-methoxy-1H-imidazo[4,5-b]pyridine-6-carboxamide |
| | MW 497.6 | 1H NMR (500 MHz, DMSO-d6) δ 10.76 (s, 1H), 8.39 (s, 1H), 7.99 (s, 1H), 7.38 - 7.28 (m, 10H), 7.27 (s, 1H), 4.38 (q, J = 7.3 Hz, 2H), 4.28 (q, J = 7.1 Hz, 2H), 4.01 (s, 3H), 1.45 (t, J = 7.3 Hz, 3H), 1.03 (t, J = 7.1 Hz, 3H). |
| | MS [M+H]⁺ 498.2 | |
| 59 | 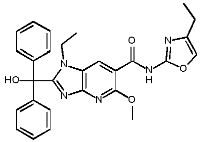 | 1-ethyl-N-(4-ethyl-1,3-oxazol-2-yl)-2-(hydroxydiphenylmethyl)-5-methoxy-1H-imidazo[4,5-b]pyridine-6-carboxamide |
| | MW 497.6 | 1H NMR (500 MHz, DMSO-d6) δ 11.02 (s, 1H), 8.32 (s, 1H), 7.63 - 7.62 (m, 1H), 7.38 - 7.28 (m, 10H), 7.27 (s, 1H), 4.27 (q, J = 7.1 Hz, 2H), 3.95 (s, 3H), 2.48 - 2.42 (m, 2H), 1.15 (t, J = 7.5 Hz, 3H), 1.03 (t, J = 7.1 Hz, 3H). |
| | MS [M+H]⁺ 498.2 | |
| 60 | 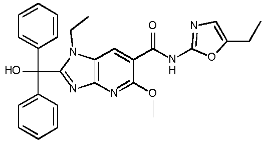 | 1-ethyl-N-(5-ethyl-1,3-oxazol-2-yl)-2-(hydroxydiphenylmethyl)-5-methoxy-1H-imidazo[4,5-b]pyridine-6-carboxamide |
| | MW 497.6 | 1H NMR (500 MHz, DMSO-d6) δ 10.91 (s, 1H), 8.32 (s, 1H), 7.38 - 7.28 (m, 10H), 7.27 (s, 1H), 6.77 (s, 1H), 4.27 (q, J = 7.1 Hz, 2H), 3.95 (s, 3H), 2.67 - 2.61 (m, 2H), 1.18 (t, J = 7.5 Hz, 3H), 1.03 (t, J = 7.1 Hz, 3H). |
| | MS [M+H]⁺ 498.2 | |
| 61 | 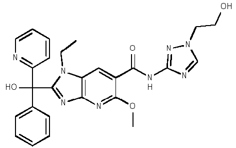 | 1-ethyl-2-[hydroxy(phenyl)(pyridin-2-yl)methyl]-N-[1-(2-hydroxyethyl)-1H-1,2,4-triazol-3-yl]-5-methoxy-1H-imidazo[4,5-b]pyridine-6-carboxamide |
| | racemate MW 514.5 | 1H NMR (500 MHz, DMSO-d6) δ 10.36 (s, 1H), 8.55 - 8.52 (m, 1H), 8.36 - 8.30 (m, 2H), 7.89 - 7.84 (m, 1H), 7.58 - 7.55 (m, 1H), 7.46 - 7.28 (m, 7H), 4.96 (t, J = 5.3 Hz, 1H), 4.22 - 4.13 (m, 4H), 4.01 - 3.92 (m, 3H), 3.74 (q, J = 5.3 Hz, 2H), 1.04 (t, J = 7.1 Hz, 3H). |
| | MS [M+H]⁺ 515.2 | |
| 62 | 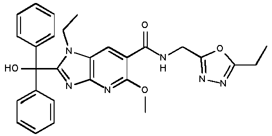 | 1-ethyl-N-[(5-ethyl-1,30,4-oxadiazol-2-yl)methyl]-2-(hydroxydiphenylmethyl)-5-methoxy-1H-imidazo[4,5-b]pyridine-6-carboxamide |
| | MW 512.6 | 1H NMR (400 MHz, DMSO-d6) δ 8.98 (t, J = 5.8 Hz, 1H), 8.42 (s, 1H), 7.38 - 7.26 (m, 11H), 4.72 (d, J = 5.8 Hz, 2H), 4.27 (q, J = 7.1 Hz, 2H), 4.00 (s, 3H), 2.83 (q, J = 7.6 Hz, 2H), 1.24 (t, J = 7.6 Hz, 3H), 1.02 (t, J = 7.1 Hz, 3H). |
| | MS [M+H]⁺ 513.2 | |
| 63 | 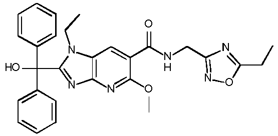 | 1-ethyl-N-[(5-ethyl-1,2,4-oxadiazol-3-yl)methyl]-2-(hydroxydiphenylmethyl)-5-methoxy-1H-imidazo[4,5-b]pyridine-6-carboxamide |
| | MW 512.6 | 1H NMR (400 MHz, DMSO-d6) δ 8.90 (t, J = 5.8 Hz, 1H), 8.43 (s, 1H), 7.38 - 7.26 (m, 11H), 4.63 (d, J = 5.8 Hz, 2H), 4.27 (q, J = 7.1 Hz, 2H), 4.01 (s, 3H), 2.92 (q, J = 7.5 Hz, 2H), 1.27 (t, J = 7.6 Hz, 3H), 1.02 (t, J = 7.1 Hz, 3H). |
| | MS [M+H]⁺ 513.2 | |
| 64 | 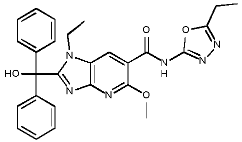 | 1-ethyl-N-(5-ethyl-1,30,4-oxadiazol-2-yl)-2-(hydroxydiphenylmethyl)-5-methoxy-1H-imidazo[4,5-b]pyridine-6-carboxamide |
| | MW 498.5 | 1H NMR (500 MHz, DMSO-d6) δ 11.36 (s, 1H), 8.34 (s, 1H), 7.38 - 7.28 (m, 10H), 7.28 (s, 1H), 4.27 (q, J = 7.1 Hz, 2H), 3.95 (s, 3H), 2.84 (q, J = 7.5 Hz, 2H), 1.26 (t, J = 7.6 Hz, 3H), 1.03 (t, J = 7.1 Hz, 3H). |
| | MS [M+H]⁺ 499.2 | |
| 65 | 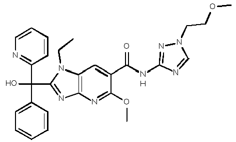 | 1-ethyl-2-[hydroxy(phenyl)(pyridin-2-yl)methyl]-5-methoxy-N-[1-(2-methoxyethyl)-1H-1,2,4-triazol-3-yl]-1H-imidazo[4,5-b]pyridine-6-carboxamide |
| | racemate MW 528.6 | 1H NMR (400 MHz, DMSO-d6) δ 10.37 (s, 1H), 8.55 - 8.52 (m, 1H), 8.38 - 8.34 (m, 1H), 8.34 - 8.30 (m, 1H), 7.89 - 7.84 (m, 1H), 7.58 - 7.55 (m, 1H), 7.45 (s, 1H), 7.44 - 7.28 (m, 6H), 4.29 (t, J = 5.2 Hz, 2H), 4.19 (q, J = 7.2 Hz, 2H), 4.02 - 3.92 (m, 3H), 3.68 (t, J = 5.1 Hz, 2H), 3.25 (s, 3H), 1.04 (t, J = 7.1 Hz, 3H). |
| | MS [M+H]⁺ 529.2 | |
| 66 | 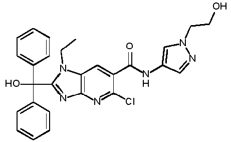 | 5-chloro-1-ethyl-2-(hydroxydiphenylmethyl)-N-[1-(2-hydroxyethyl)-1H-pyrazol-4-yl]-1H-imidazo[4,5-b]pyridine-6-carboxamide |
| | MW 517.0 | 1H NMR (500 MHz, DMSO-d6) δ 10.58 (s, 1H), 8.34 (s, 1H), 8.04 - 8.02 (m, 1H), 7.52 - 7.51 (m, 1H), 7.40 (s, 1H), 7.38 - 7.29 (m, 10H), 4.88 (t, J = 5.3 Hz, 1H), 4.26 (q, J = 7.1 Hz, 2H), 4.13 (t, J = 5.6 Hz, 2H), 3.72 (q, J = 5.5 Hz, 2H), 1.04 (t, J = 7.1 Hz, 3H). |
| | MS [M+H]⁺ 517.2 | |
| 67 | 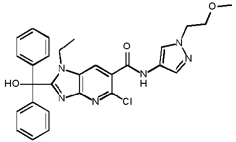 | 5-chloro-1-ethyl-2-(hydroxydiphenylmethyl)-N-[1-(2-methoxyethyl)-1H-pyrazol-4-yl]-1H-imidazo[4,5-b]pyridine-6-carboxamide |
| | MW 531.0 | 1H NMR (500 MHz, DMSO-d6) δ 10.59 (s, 1H), 8.36 (s, 1H), 8.03 - 8.02 (m, 1H), 7.52 - 7.51 (m, 1H), 7.40 (s, 1H), 7.38 - 7.29 (m, 10H), 4.29 - 4.23 (m, 4H), 3.67 (t, J = 5.3 Hz, 2H), 3.24 (s, 3H), 1.04 (t, J = 7.1 Hz, 3H). |
| | MS [M+H]⁺ 531.2 | |
| 68 | 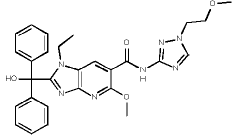 | 1-ethyl-2-(hydroxydiphenylmethyl)-5-methoxy-N-[1-(2-methoxyethyl)-1H-1,2,4-triazol-3-yl]-1H-imidazo[4,5-b]pyridine-6-carboxamide |
| | MW 527.6 | 1H NMR (700 MHz, DMSO-d6) δ 10.38 (s, 1H), 8.39 - 8.35 (m, 1H), 8.35 - 8.29 (m, 1H), 7.37 - 7.28 (m, 11H), 4.31 - 4.26 (m, 2H), 4.27 (q, J = 7.3 Hz, 2H), 4.03 - 3.92 (m, 3H), 3.71 - 3.64 (m, 2H), 3.24 (s, 3H), 1.02 (t, J = 7.1 Hz, 3H). |
| | MS [M+H]⁺ 528.2 | |
| 69 | 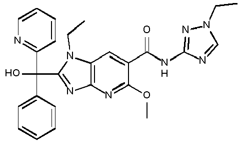 | 1-ethyl-N-(1-ethyl-1H-1,2,4-triazol-3-yl)-2-[hydroxy(phenyl)(pyridin-2-yl)methyl]-5-methoxy-1H-imidazo[4,5-b]pyridine-6-carboxamide |
| | racemate MW 498.5 | 1H NMR (500 MHz, DMSO-d6) δ 10.36 (s, 1H), 8.54 - 8.52 (m, 1H), 8.43 - 8.38 (m, 1H), 8.36 - 8.29 (m, 1H), 7.89 - 7.84 (m, 1H), 7.58 - 7.55 (m, 1H), 7.46 (s, 1H), 7.45 - 7.41 (m, 2H), 7.39 - 7.28 (m, 4H), 4.23 - 4.11 (m, 4H), 4.02 - 3.91 (m, 3H), 1.39 (t, J = 7.1 Hz, 3H), 1.03 (t, J = 7.1 Hz, 3H). |
| | MS [M+H]⁺ 499.2 | |
| 70 | 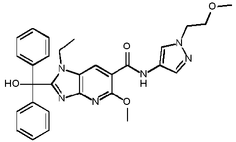 | 1-ethyl-2-(hydroxydiphenylmethyl)-5-methoxy-N-[1-(2-methoxyethyl)-1H-pyrazol-4-yl]-1H-imidazo[4,5-b]pyridine-6-carboxamide |
| | MW 526.6 | 1H NMR (500 MHz, DMSO-d6) δ 10.14 (s, 1H), 8.35 (s, 1H), 8.05 - 8.04 (m, 1H), 7.64 - 7.62 (m, 1H), 7.37 - 7.28 (m, 11H), 4.26 (q, J = 7.1 Hz, 2H), 4.23 (t, J = 5.3 Hz, 2H), 3.99 (s, 3H), 3.66 (t, J = 5.3 Hz, 2H), 3.24 (s, 3H), 1.03 (t, J = 7.1 Hz, 3H). |
| | MS [M+H]⁺ 527.2 | |
| 71 | 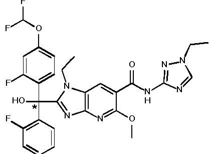 | 2-[4-(difluoromethoxy)-2-fluorophenyl](2-fluorophenyl)hydroxymethyl]-1-ethyl-N-(1-ethyl-1H-1,2,4-triazol-3-yl)-5-methoxy-1H-imidazo[4,5-b]pyridine-6-carboxamide |
| | Isolated enantiomer MW 599.5 | 1H NMR (500 MHz, DMSO-d6) δ 10.37 (s, 1H), 8.44 - 8.38 (m, 1H), 8.38 - 8.32 (m, 1H), 7.49 - 7.47 (m, 1H), 7.47 - 7.41 (m, 1H), 7.40 - 7.34 (m, 2H), 7.33 (t, J = 73.6 Hz, 1H), 7.27 - 7.23 (m, 1H), 7.18 - 7.13 (m, 1H), 7.09 (dd, J = 11.8, 2.5 Hz, 1H), 7.06 (dd, J = 8.6, 2.6 Hz, 1H), 4.34 - 4.22 (m, 2H), 4.15 (q, J = 7.1 Hz, 2H), 4.03 - 3.90 (m, 3H), 1.39 (t, J = 7.3 Hz, 3H), 1.12 (t, J = 7.1 Hz, 3H). |
| | MS [M+H]⁺ 600.2 | |
| | | Chiral SFC: method: SFC; column: Lux Amylose-1; eluent: CO₂:2-Propanol+0.5%DEA (70:30); wave length: 220nm flow: 5mL/min; rt 4.44 min. |
| 72 | 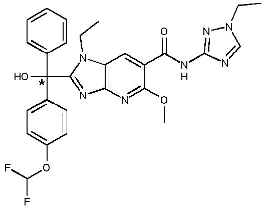 | 2-[4-(difluoromethoxy)phenyl](hydroxy)ph enylmethyl]-1-ethyl-N-(1-ethyl-1H-1,2,4-triazol-3-yl)-5-methoxy-1H-imidazo[4,5-b]pyridine-6-carboxamide |
| | Isolated enantiomer MW 563.6 | 1H NMR (700 MHz, DMSO-d6) δ 10.36 (s, 1H), 8.44 - 8.37 (m, 1H), 8.37 - 8.30 (m, 1H), 7.39 - 7.35 (m, 5H), 7.33 - 7.28 (m, 3H), 7.24 (t, J = 74.1 Hz, 1H), 7.17 - 7.14 (m, 2H), 4.32 - 4.21 (m, 2H), 4.19 - 4.11 (m, 2H), 4.02 - 3.93 (m, 3H), 1.43 - 1.35 (m, 3H), 1.04 (t, J = 7.0 Hz, 3H). |
| | MS [M+H]⁺ 564.2 | |
| | | Chiral SFC: method: SFC; column: Lux Amylose-1; eluent: CO₂:2-Propanol+0.5%DEA (75:25); wave length: 220nm flow: 5mL/min; rt 8.31 min. |
| 73 | 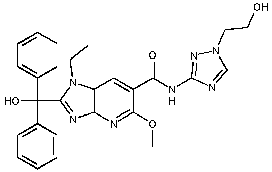 | 1-ethyl-2-(hydroxydiphenylmethyl)-N-[1-(2-hydroxyethyl)-1H-1,2,4-triazol-3-yl]-5-methoxy-1H-imidazo[4,5-b]pyridine-6-carboxamide |
| | MW 513.6 | 1H NMR (500 MHz, DMSO-d6) δ 10.39 (s, 1H), 8.35 - 8.32 (m, 1H), 8.32 - 8.29 (m, 1H), 7.37 - 7.28 (m, 11H), 5.01 (t, J = 5.3 Hz, 1H), 4.27 (q, J = 7.0 Hz, 2H), 4.16 (t, J = 5.2 Hz, 2H), 4.02 - 3.94 (m, 3H), 3.76 - 3.70 (m, 2H), 1.02 (t, J = 7.1 Hz, 3H). |
| | MS [M+H]⁺ 514.2 | |
| 74 | 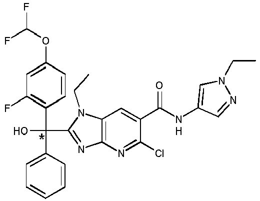 | 5-chloro-2-[4-(difluoromethoxy)-2-fluorophenyl](hydroxy)phenylmethyl]-1-ethyl-N-(1-ethyl-1H-pyrazol-4-yl)-1H-imidazo[4,5-b]pyridine-6-carboxamide |
| | Isolated enantiomer MW 585.0 | 1H NMR (700 MHz, DMSO-d6) δ 10.58 (s, 1H), 8.36 (s, 1H), 8.04 (s, 1H), 7.51 (s, 1H), 7.50 (s, 1H), 7.42 - 7.33 (m, 5H), 7.32 (t, J = 73.6 Hz, 1H), 7.12 (dd, J = 11.8, 2.4 Hz, 1H), 7.06 (t, J = 8.7 Hz, 1H), 7.00 (dd, J = 8.7, 2.5 Hz, 1H), 4.36 - 4.21 (m, 2H), 4.12 (q, J = 7.3 Hz, 2H), 1.36 (t, J = 7.3 Hz, 3H), 1.05 (t, J = 7.1 Hz, 3H). |
| | MS [M+H]⁺ 585.1 | |
| | | Chiral SFC: method: SFC; column: Lux Cellulose-2; eluent: CO₂:Ethanol+0.5%DEA (75:25); wave length: 220nm; flow: 5; rt 4.69 min. |
| 75 | 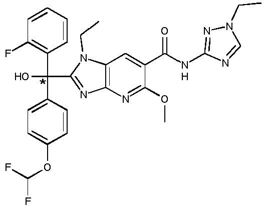 | 2-[4-(difluoromethoxy)phenyl](2-fluorophenyl)hydroxymethyl]-1-ethyl-N-(1-ethyl-1H-1,2,4-triazol-3-yl)-5-methoxy-1H-imidazo[4,5-b]pyridine-6-carboxamide |
| | Isolated enantiomer MW 581.6 | 1H NMR (700 MHz, DMSO-d6) δ 10.36 (s, 1H), 8.43 - 8.38 (m, 1H), 8.37 - 8.31 (m, 1H), 7.45 - 7.40 (m, 4H), 7.26 (t, J = 74.1 Hz, 1H), 7.22 - 7.15 (m, 5H), 4.32 - 4.25 (m, 2H), 4.18 - 4.12 (m, 2H), 4.03 - 3.91 (m, 3H), 1.43 - 1.35 (m, 3H), 1.07 (t, J = 7.2 Hz, 3H). |
| | MS [M+H]⁺ 582.2 | Chiral SFC: method: SFC; column: Lux Amylose-1; eluent: CO₂:2-Propanol+0.5%DEA (75:25); wave length: 220nm; flow: 5mL/min; rt 7.03 min. |
| 76 | 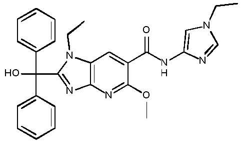 | 1-ethyl-N-(1-ethyl-1H-imidazol-4-yl)-2-(hydroxydiphenylmethyl)-5-methoxy-1H-imidazo[4,5-b]pyridine-6-carboxamide |
| | MW 496.6 | 1H NMR (500 MHz, DMSO-d6) δ 10.26 (s, 1H), 8.42 (s, 1H), 7.52 (d, J = 1.5 Hz, 1H), 7.40 (d, J = 1.6 Hz, 1H), 7.37 - 7.33 (m, 4H), 7.32 - 7.27 (m, 7H), 4.27 (q, J = 6.9 Hz, 2H), 4.04 (s, 3H), 3.99 (q, J = 7.3 Hz, 2H), 1.35 (t, J = 7.3 Hz, 3H), 1.02 (t, J = 7.1 Hz, 3H). |
| | MS [M+H]⁺ 497.2 | |
| 77 | 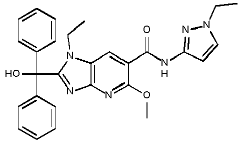 | 1-ethyl-N-(1-ethyl-1H-pyrazol-3-yl)-2-(hydroxydiphenylmethyl)-5-methoxy-1H-imidazo[4,5-b]pyridine-6-carboxamide |
| | MW 496.6 | 1H NMR (500 MHz, DMSO-d6) δ 10.41 (s, 1H), 8.41 (s, 1H), 7.66 (d, J = 2.3 Hz, 1H), 7.38 - 7.27 (m, 11H), 6.61 (d, J = 2.2 Hz, 1H), 4.28 (q, J = 7.1 Hz, 2H), 4.06 (q, J = 7.3 Hz, 2H), 4.02 (s, 3H), 1.36 (t, J = 7.3 Hz, 3H), 1.03 (t, J = 7.1 Hz, 3H). |
| | MS [M+H]⁺ 497.2 | |
| 78 | 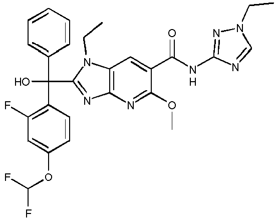 | 2-{[4-(difluoromethoxy)-2-fluorophenyl](hydroxy)phenylmethyl}-1-ethyl-N-(1-ethyl-1H-1,2,4-triazol-3-yl)-5-methoxy-1H-imidazo[4,5-b]pyridine-6-carboxamide |
| | racemate MW 581.6 | 1H NMR (500 MHz, DMSO-d6) δ 10.35 (s, 1H), 8.43 - 8.38 (m, 1H), 8.36 - 8.30 (m, 1H), 7.43 - 7.32 (m, 6H), 7.32 (t, J = 73.6 Hz, 1H), 7.11 (dd, J = 11.8, 2.5 Hz, 1H), 7.05 (t, J = 8.7 Hz, 1H), 6.99 (dd, J = 8.7, 2.5 Hz, 1H), 4.38 - 4.19 (m, 2H), 4.15 (q, J = 7.3 Hz, 2H), 4.04 - 3.92 (m, 3H), 1.39 (t, J = 6.9 Hz, 3H), 1.04 (t, J = 7.1 Hz, 3H). |
| | MS [M+H]⁺ 582.2 | |
| 79 | 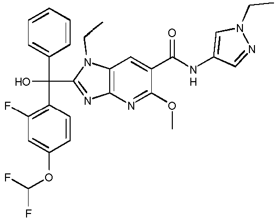 | 2-{[4-(difluoromethoxy)-2-fluorophenyl](hydroxy)phenylmethyl}-1-ethyl-N-(1-ethyl-1H-pyrazol-4-yl)-5-methoxy-1H-imidazo[4,5-b]pyridine-6-carboxamide |
| | racemate MW 580.6 | 1H NMR (500 MHz, DMSO-d6) δ 10.13 (s, 1H), 8.36 (s, 1H), 8.06 - 8.05 (m, 1H), 7.62 - 7.61 (m, 1H), 7.43 - 7.32 (m, 6H), 7.32 (t, J = 73.7 Hz, 1H), 7.11 (dd, J = 11.8, 2.5 Hz, 1H), 7.06 (t, J = 8.7 Hz, 1H), 6.99 (dd, J = 8.7, 2.5 Hz, 1H), 4.37 - 4.19 (m, 2H), 4.11 (q, J = 7.3 Hz, 2H), 4.00 (s, 3H), 1.36 (t, J = 7.3 Hz, 3H), 1.04 (t, J = 7.1 Hz, 3H). |
| | MS [M+H]⁺ 581.2 | |
| 80 | 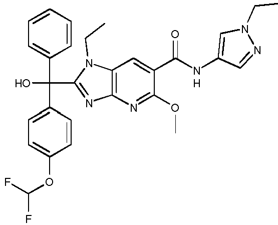 | 2-{[4-(difluoromethoxy)phenyl](hydroxy) ph enylmethyl}-1-ethyl-N-(1-ethyl-1H-pyrazol-4-yl)-5-methoxy-1H-imidazo[4,5-b]pyridine-6-carboxamide |
| | racemate MW 562.6 | 1H NMR (500 MHz, DMSO-d6) δ 10.13 (s, 1H), 8.36 (s, 1H), 8.06 - 8.04 (m, 1H), 7.62 - 7.61 (m, 1H), 7.38 - 7.27 (m, 8H), 7.24 (t, J = 74.1 Hz, 1H), 7.17 - 7.14 (m, 2H), 4.32 - 4.19 (m, 2H), 4.11 (q, J = 7.3 Hz, 2H), 4.00 (s, 3H), 1.36 (t, J = 7.3 Hz, 3H), 1.05 (t, J = 7.1 Hz, 3H). |
| | MS [M+H]⁺ 563.2 | |
| 81 | 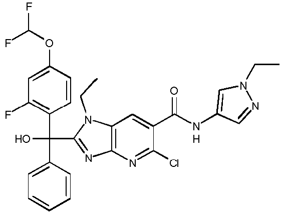 | 5-chloro-2-{[4-(difluoromethoxy)-2-fluorophenyl](hydroxy)phenylmethyl}-1-ethyl-N-(1-ethyl-1H-pyrazol-4-yl)-1H-imidazo[4,5-b]pyridine-6-carboxamide |
| | racemate MW 585.0 | 1H NMR (700 MHz, DMSO-d6) δ 10.58 (s, 1H), 8.36 (s, 1H), 8.04 - 8.03 (m, 1H), 7.51 (s, 1H), 7.50 - 7.49 (m, 1H), 7.43 - 7.21 (m, 6H), 7.12 (dd, J = 11.8, 2.5 Hz, 1H), 7.06 (t, J = 8.7 Hz, 1H), 7.00 (dd, J = 8.7, 2.5 Hz, 1H), 4.36 - 4.21 (m, 2H), 4.12 (q, J = 7.2 Hz, 2H), 1.36 (t, J = 7.3 Hz, 3H), 1.05 (t, J = 7.1 Hz, 3H). |
| | MS [M+H]⁺ 585.1 | |
| 82 | 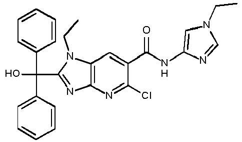 | 5-chloro-1-ethyl-N-(1-ethyl-1H-imidazol-4-yl)-2-(hydroxydiphenylmethyl)-1H-imidazo[4,5-b]pyridine-6-carboxamide |
| | MW 501.0 | 1H NMR (500 MHz, DMSO-d6) δ 10.91 (s, 1H), 8.28 (s, 1H), 7.50 (d, J = 1.6 Hz, 1H), 7.43 (d, J = 1.5 Hz, 1H), 7.38 (s, 1H), 7.38 - 7.29 (m, 10H), 4.27 (q, J = 7.1 Hz, 2H), 4.00 (q, J = 7.3 Hz, 2H), 1.36 (t, J = 7.3 Hz, 3H), 1.04 (t, J = 7.1 Hz, 3H). |
| | MS [M+H]⁺ 501.2 | |
| 83 | 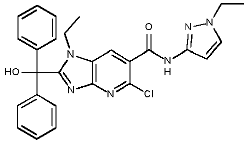 | 5-chloro-1-ethyl-N-(1-ethyl-1 H-pyrazol-3-yl)-2-(hydroxydiphenylmethyl)-1H-imidazo[4,5-b]pyridine-6-carboxamide |
| | MW 501.0 | 1H NMR (700 MHz, DMSO-d6) δ 11.02 (s, 1H), 8.32 (s, 1H), 7.67 (d, J = 2.2 Hz, 1H), 7.39 (s, 1H), 7.38 - 7.30 (m, 10H), 6.60 (d, J = 2.2 Hz, 1H), 4.27 (q, J = 7.1 Hz, 2H), 4.06 (q, J = 7.2 Hz, 2H), 1.35 (t, J = 7.2 Hz, 3H), 1.03 (t, J = 7.1 Hz, 3H). |
| | MS [M+H]⁺ 501.2 | |
| 84 | 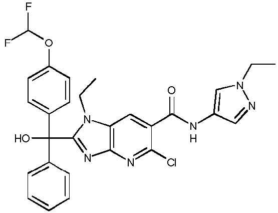 | 5-chloro-2-{[4-(difluoromethoxy)phenyl](hydroxy) ph enylmethyl}-1-ethyl-N-(1-ethyl-1H-pyrazol-4-yl)-1H-imidazo[4,5-b]pyridine-6-carboxamide |
| | racemate MW 567.0 | 1H NMR (500 MHz, DMSO-d6) δ 10.59 (s, 1H), 8.36 (s, 1H), 8.04 - 8.03 (m, 1H), 7.50 - 7.49 (m, 1H), 7.48 (s, 1H), 7.40 - 7.35 (m, 4H), 7.34 - 7.28 (m, 3H), 7.24 (t, J = 74.0 Hz, 1H), 7.18 - 7.15 (m, 2H), 4.32 - 4.19 (m, 2H), 4.12 (q, J = 7.2 Hz, 2H), 1.36 (t, J = 7.2 Hz, 3H), 1.06 (t, J = 7.1 Hz, 3H). |
| | MS [M+H]⁺ 567.2 | |
| 85 | 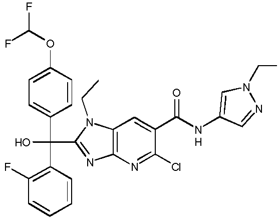 | 5-chloro-2-{[4-(difluoromethoxy)phenyl](2-fluorophenyl)hydroxymethyl}-1-ethyl-N-(1-ethyl-1H-pyrazol-4-yl)-1H-imidazo[4,5-b]pyridine-6-carboxamide |
| | racemate MW 585.0 | 1H NMR (700 MHz, DMSO-d6) δ 10.58 (s, 1H), 8.35 (s, 1H), 8.04 (s, 1H), 7.54 (s, 1H), 7.49 (s, 1H), 7.46 - 7.41 (m, 3H), 7.25 (t, J = 74.0 Hz, 1H), 7.23 - 7.15 (m, 5H), 4.31 - 4.25 (m, 2H), 4.13 (q, J = 7.2 Hz, 2H), 1.36 (t, J = 7.2 Hz, 3H), 1.08 (t, J = 7.1 Hz, 3H). |
| | MS [M+H]⁺ 585.2 | |
| 86 | 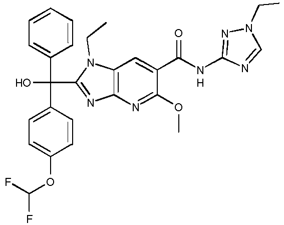 | 2-{[4-(difluoromethoxy)phenyl](hydroxy) ph enylmethyl}-1-ethyl-N-(1-ethyl-1H-1,2,4-triazol-3-yl)-5-methoxy-1H-imidazo[4,5-b]pyridine-6-carboxamide |
| | racemate MW 563.6 | 1H NMR (700 MHz, DMSO-d6) δ 10.37 - 10.33 (m, 1H), 8.43 - 8.37 (m, 1H), 8.37 - 8.31 (m, 1H), 7.38 - 7.35 (m, 5H), 7.33 - 7.29 (m, 3H), 7.24 (t, J = 74.1 Hz, 1H), 7.17 - 7.14 (m, 2H), 4.33 - 4.22 (m, 2H), 4.18 - 4.12 (m, 2H), 4.03 - 3.94 (m, 3H), 1.42 - 1.36 (m, 3H), 1.05 (t, J = 7.1 Hz, 3H). |
| | MS [M+H]⁺ 564.2 | |
| 87 | 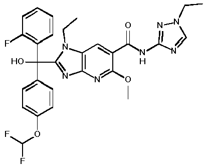 | 2-{[4-(difluoromethoxy)phenyl](2-fluorophenyl)hydroxymethyl}-1-ethyl-N-(1-ethyl-1H-1,2,4-triazol-3-yl)-5-methoxy-1H-imidazo[4,5-b]pyridine-6-carboxamide |
| | racemate MW 581.6 | 1H NMR (500 MHz, DMSO-d6) δ 10.36 (s, 1H), 8.42 - 8.37 (m, 1H), 8.36 - 8.30 (m, 1H), 7.45 - 7.40 (m, 4H), 7.25 (t, J = 74.1 Hz, 1H), 7.22 - 7.14 (m, 5H), 4.32 - 4.24 (m, 2H), 4.15 (q, J = 7.3 Hz, 2H), 4.02 - 3.92 (m, 3H), 1.39 (t, J = 7.3 Hz, 3H), 1.06 (t, J = 7.1 Hz, 3H). |
| | MS [M+H]⁺ 582.2 | |
| 88 | 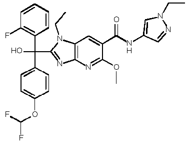 | 2-{[4-(difluoromethoxy)phenyl](2-fluorophenyl)hydroxymethyl}-1-ethyl-N-(1-ethyl-1H-pyrazol-4-yl)-5-methoxy-1H-imidazo[4,5-b]pyridine-6-carboxamide |
| | racemate MW 580.6 | 1H NMR (700 MHz, DMSO-d6) δ 10.13 (s, 1H), 8.36 (s, 1H), 8.06 - 8.05 (m, 1H), 7.62 - 7.61 (m, 1H), 7.44 - 7.40 (m, 4H), 7.26 (t, J = 74.0 Hz, 1H), 7.22 - 7.15 (m, 5H), 4.31 - 4.24 (m, 2H), 4.11 (q, J = 7.2 Hz, 2H), 3.99 (s, 3H), 1.36 (t, J = 7.3 Hz, 3H), 1.07 (t, J = 7.1 Hz, 3H). |
| | MS [M+H]⁺ 581.2 | |
| 89 | 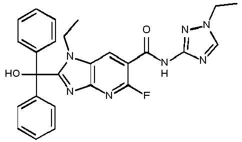 | 1-ethyl-N-(1-ethyl-1H-1,2,4-triazol-3-yl)-5-fluoro-2-(hydroxydiphenylmethyl)-1H-imidazo[4,5-b]pyridine-6-carboxamide |
| | MW 485.5 | 1H NMR (700 MHz, DMSO-d6) δ 10.86 (s, 1H), 8.48 - 8.43 (m, 1H), 8.43 - 8.38 (m, 1H), 7.38 - 7.29 (m, 11H), 4.30 (q, J = 7.1 Hz, 2H), 4.19 - 4.12 (m, 2H), 1.42 - 1.35 (m, 3H), 1.06 (t, J = 7.1 Hz, 3H). |
| | MS [M+H]⁺ 486.1 | |
| 90 | 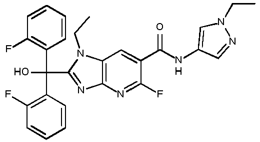 | 2-[bis(2-fluorophenyl)(hydroxy)methyl]-1-ethyl-N-(1-ethyl-1H-pyrazol-4-yl)-5-fluoro-1H-imidazo[4,5-b]pyridine-6-carboxamide |
| | MW 520.5 | 1H NMR (700 MHz, DMSO-d6) δ 10.49 (s, 1H), 8.49 (d, J = 8.6 Hz, 1H), 8.05 (s, 1H), 7.56 (s, 1H), 7.52 (s, 1H), 7.47 - 7.42 (m, 2H), 7.39 - 7.35 (m, 2H), 7.26 - 7.23 (m, 2H), 7.18 - 7.14 (m, 2H), 4.32 (q, J = 7.1 Hz, 2H), 4.13 (q, J = 7.3 Hz, 2H), 1.36 (t, J = 7.2 Hz, 3H), 1.12 (t, J = 7.1 Hz, 3H). |
| | MS [M+H]⁺ 521.2 | |
| 91 | 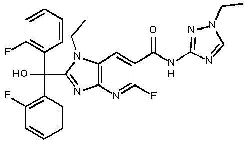 | 2-[bis(2-fluorophenyl)(hydroxy)methyl]-1-ethyl-N-(1-ethyl-1H-1,2,4-triazol-3-yl)-5-fluoro-1H-imidazo[4,5-b]pyridine-6-carboxamide |
| | MW 521.5 | 1H NMR (700 MHz, DMSO-d6) δ 10.87 (s, 1H), 8.49 - 8.45 (m, 1H), 8.43 - 8.39 (m, 1H), 7.51 (s, 1H), 7.46 - 7.42 (m, 2H), 7.38 - 7.34 (m, 2H), 7.24 (td, J = 7.6, 1.2 Hz, 2H), 7.18 - 7.13 (m, 2H), 4.31 (q, J = 7.1 Hz, 2H), 4.19 - 4.13 (m, 2H), 1.43 - 1.36 (m, 3H), 1.11 (t, J = 7.1 Hz, 3H). |
| | MS [M+H]⁺ 522.2 | |
| 92 | 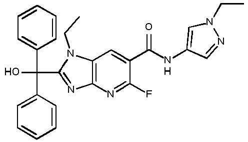 | 1-ethyl-N-(1-ethyl-1H-pyrazol-4-yl)-5-fluoro-2-(hydroxydiphenylmethyl)-1H-imidazo[4,5-b]pyridine-6-carboxamide |
| | MW 484.5 | 1H NMR (700 MHz, DMSO-d6) δ 10.51 (s, 1H), 8.46 (d, J = 8.5 Hz, 1H), 8.04 (s, 1H), 7.56 (s, 1H), 7.40 - 7.29 (m, 11H), 4.29 (q, J = 7.1 Hz, 2H), 4.12 (q, J = 7.2 Hz, 2H), 1.35 (t, J = 7.2 Hz, 3H), 1.06 (t, J = 7.1 Hz, 3H). |
| | MS [M+H]⁺ 485.1 | |
| 93 | 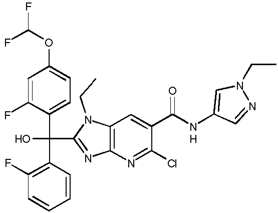 | 5-chloro-2-{[4-(difluoromethoxy)-2-fluorophenyl](2-fluorophenyl)hydroxymethyl}-1-ethyl-N-(1-ethyl-1H-pyrazol-4-yl)-1H-imidazo[4,5-b]pyridine-6-carboxamide |
| | racemate MW 603.0 | 1H NMR (700 MHz, DMSO-d6) δ 10.60 (s, 1H), 8.38 (s, 1H), 8.05 - 8.04 (m, 1H), 7.62 - 7.61 (m, 1H), 7.51 - 7.49 (m, 1H), 7.48 - 7.44 (m, 1H), 7.42 - 7.36 (m, 2H), 7.28 (t, J = 73.6 Hz, 1H), 7.28 - 7.25 (m, 1H), 7.18 - 7.14 (m, 1H), 7.10 (dd, J = 11.8, 2.5 Hz, 1H), 7.07 (dd, J = 8.6, 2.6 Hz, 1H), 4.33 - 4.23 (m, 2H), 4.13 (q, J = 7.2 Hz, 2H), 1.36 (t, J = 7.3 Hz, 3H), 1.13 (t, J = 7.1 Hz, 3H). |
| | MS [M+H]⁺ 603.2 | |
| 94 | 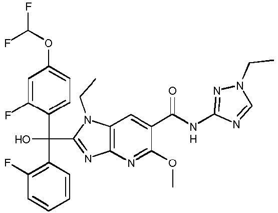 | 2-{[4-(difluoromethoxy)-2-fluorophenyl](2-fluorophenyl)hydroxymethyl}-1-ethyl-N-(1-ethyl-1H-1,2,4-triazol-3-yl)-5-methoxy-1H-imidazo[4,5-b]pyridine-6-carboxamide |
| | racemate MW 599.5 | 1H NMR (700 MHz, DMSO-d6) δ 10.39 (s, 1H), 8.43 - 8.38 (m, 1H), 8.38 - 8.33 (m, 1H), 7.52 - 7.48 (m, 1H), 7.46 - 7.43 (m, 1H), 7.40 - 7.34 (m, 2H), 7.33 (t, J = 73.6 Hz, 1H), 7.27 - 7.24 (m, 1H), 7.18 - 7.14 (m, 1H), 7.10 (dd, J = 11.8, 2.6 Hz, 1H), 7.06 (dd, J = 8.7, 2.6 Hz, 1H), 4.33 - 4.24 (m, 2H), 4.18 - 4.13 (m, 2H), 4.01 - 3.95 (m, 3H), 1.43 - 1.36 (m, 3H), 1.12 (t, J = 7.2 Hz, 3H). |
| | MS [M+H]⁺ 600.2 | |
| 95 | 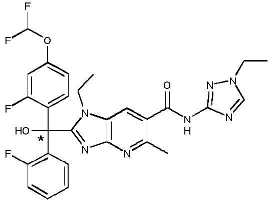 | 2-[4-(difluoromethoxy)-2-fluorophenyl](2-fluorophenyl)hydroxymethyl]-1-ethyl-N-(1-ethyl-1H-1,2,4-triazol-3-yl)-5-methyl-1H-imidazo[4,5-b]pyridine-6-carboxamide |
| | Isolated enantiomer MW 583.5 | 1H NMR (700 MHz, DMSO-d6) δ 10.78 (s, 1H), 8.41 (s, 1H), 8.14 - 8.09 (m, 1H), 7.49 (s, 1H), 7.46 - 7.42 (m, 1H), 7.39 - 7.34 (m, 2H), 7.34 (t, J = 73.6 Hz, 1H), 7.27 - 7.24 (m, 1H), 7.17 - 7.13 (m, 1H), 7.09 (dd, J = 11.8, 2.6 Hz, 1H), 7.06 (dd, J = 8.6, 2.6 Hz, 1H), 4.30 - 4.20 (m, 2H), 4.19 - 4.13 (m, 2H), 2.60 (s, 3H), 1.42 - 1.36 (m, 3H), 1.15 - 1.11 (m, 3H). |
| | MS [M+H]⁺ 584.2 | |
| | | Chiral SFC: column: Chiralpak AZ solvents: CO₂ , 40% isopropanol + 0.5% diethylamine, rt 4.58 min. |
| 96 | 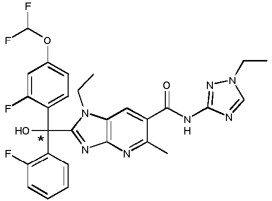 | 2-[4-(difluoromethoxy)-2-fluorophenyl](2-fluorophenyl)hydroxymethyl]-1-ethyl-N-(1-ethyl-1H-1,2,4-triazol-3-yl)-5-methyl-1H-imidazo[4,5-b]pyridine-6-carboxamide |
| | Isolated enantiomer MW 583.5 | 1H NMR (700 MHz, DMSO-d6) δ 10.78 (s, 1H), 8.42 - 8.39 (m, 1H), 8.14 - 8.10 (m, 1H), 7.49 (s, 1H), 7.46 - 7.42 (m, 1H), 7.39 - 7.34 (m, 2H), 7.34 (t, J = 73.6 Hz, 1H), 7.27 - 7.24 (m, 1H), 7.17 - 7.14 (m, 1H), 7.09 (dd, J = 11.7, 2.5 Hz, 1H), 7.06 (dd, J = 8.6, 2.6 Hz, 1H), 4.30 - 4.20 (m, 2H), 4.19 - 4.13 (m, 2H), 2.60 (s, 3H), 1.42 - 1.36 (m, 3H), 1.15 - 1.11 (m, 3H). |
| | MS [M+H]⁺ 584.3 | |
| | | Chiral SFC: column: Chiralpak AZ solvents: CO₂ , 40% isopropanol + 0.5% diethylamine, rt 3.33 min. |
| 97 | 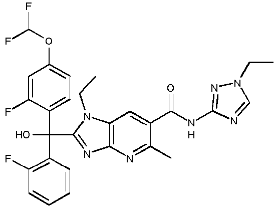 | 2-{[4-(difluoromethoxy)-2-fluorophenyl](2-fluorophenyl)hydroxymethyl}-1-ethyl-N-(1-ethyl-1H-1,2,4-triazol-3-yl)-5-methyl-1H-imidazo[4,5-b]pyridine-6-carboxamide |
| | racemate MW 583.5 | 1H NMR (400 MHz, DMSO-d6) δ 10.76 (s, 1H), 8.41 - 8.39 (m, 1H), 8.14 - 8.12 (m, 1H), 7.49 - 7.46 (m, 1H), 7.46 - 7.41 (m, 1H), 7.40 - 7.33 (m, 2H), 7.33 (t, J = 73.6 Hz, 1H), 7.28 - 7.23 (m, 1H), 7.18 - 7.12 (m, 1H), 7.11 - 7.04 (m, 2H), 4.31 - 4.20 (m, 2H), 4.16 (q, J = 7.3 Hz, 2H), 2.61 (s, 3H), 1.39 (t, J = 7.2 Hz, 3H), 1.14 (t, J = 7.1 Hz, 3H). |
| | MS [M+H]⁺ 584.2 | |
| 98 | 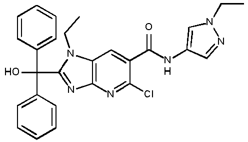 | 5-chloro-1-ethyl-N-(1-ethyl-1H-pyrazol-4-yl)-2-(hydroxydiphenylmethyl)-1H-imidazo[4,5-b]pyridine-6-carboxamide |
| | MW 501.0 | 1H NMR (700 MHz, DMSO-d6) δ 10.59 (s, 1H), 8.35 (s, 1H), 8.04 - 8.03 (m, 1H), 7.50 - 7.49 (m, 1H), 7.41 (s, 1H), 7.37 - 7.34 (m, 4H), 7.33 - 7.29 (m, 6H), 4.26 (q, J = 7.1 Hz, 2H), 4.12 (q, J = 7.2 Hz, 2H), 1.36 (t, J = 7.2 Hz, 3H), 1.04 (t, J = 7.0 Hz, 3H). |
| | MS [M+H]⁺ 501.1 | |
| 99 | 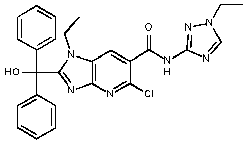 | 5-chloro-1-ethyl-N-(1-ethyl-1H-1,2,4-triazol-3-yl)-2-(hydroxydiphenylmethyl)-1H-imidazo[4,5-b]pyridine-6-carboxamide |
| | MW 502.0 | 1H NMR (700 MHz, DMSO-d6) δ 10.97 (s, 1H), 8.44 - 8.39 (m, 1H), 8.38 - 8.32 (m, 1H), 7.40 (s, 1H), 7.38 - 7.29 (m, 10H), 4.31 - 4.25 (m, 2H), 4.20 - 4.13 (m, 2H), 1.43 - 1.37 (m, 3H), 1.06 - 1.00 (m, 3H). |
| | MS [M+H]⁺ 502.1 | |
| 100 | 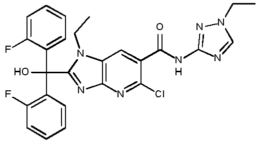 | 2-[bis(2-fluorophenyl)(hydroxy)methyl]-5-chloro-1-ethyl-N-(1-ethyl-1H-1,2,4-triazol-3-yl)-1H-imidazo[4,5-b]pyridine-6-carboxamide |
| | MW 538.0 | 1H NMR (700 MHz, DMSO-d6) δ 10.98 (s, 1H), 8.44 - 8.39 (m, 1H), 8.38 - 8.34 (m, 1H), 7.57 - 7.52 (m, 1H), 7.47 - 7.42 (m, 2H), 7.38 - 7.34 (m, 2H), 7.27 - 7.23 (m, 2H), 7.19 - 7.14 (m, 2H), 4.33 - 4.25 (m, 2H), 4.22 - 4.11 (m, 2H), 1.45 - 1.35 (m, 3H), 1.13 - 1.04 (m, 3H). |
| | MS [M+H]⁺ 538.2 | |
| 101 | 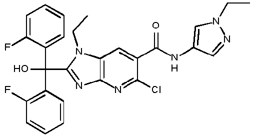 | 2-[bis(2-fluorophenyl)(hydroxy)methyl]-5-chloro-1-ethyl-N-(1-ethyl-1H-pyrazol-4-yl)-1H-imidazo[4,5-b]pyridine-6-carboxamide |
| | MW 537.0 | 1H NMR (700 MHz, DMSO-d6) δ 10.60 (s, 1H), 8.37 (s, 1H), 8.05 - 8.04 (m, 1H), 7.55 (s, 1H), 7.51 - 7.49 (m, 1H), 7.47 - 7.43 (m, 2H), 7.38 - 7.35 (m, 2H), 7.26 - 7.23 (m, 2H), 7.18 - 7.14 (m, 2H), 4.28 (q, J = 7.1 Hz, 2H), 4.13 (q, J = 7.3 Hz, 2H), 1.36 (t, J = 7.2 Hz, 3H), 1.09 (t, J = 7.1 Hz, 3H). |
| | MS [M+H]⁺ 537.2 | |
| 102 | 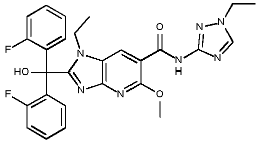 | 2-[bis(2-fluorophenyl)(hydroxy)methyl]-1-ethyl-N-(1-ethyl-1H-1,2,4-triazol-3-yl)-5-methoxy-1H-imidazo[4,5-b]pyridine-6-carboxamide |
| | MW 533.5 | 1H NMR (700 MHz, DMSO-d6) δ 10.37 (s, 1H), 8.43 - 8.38 (m, 1H), 8.37 - 8.32 (m, 1H), 7.45 - 7.41 (m, 2H), 7.41 (s, 1H), 7.36 - 7.33 (m, 2H), 7.25 - 7.22 (m, 2H), 7.18 - 7.13 (m, 2H), 4.28 (q, J = 7.1 Hz, 2H), 4.18 - 4.12 (m, 2H), 4.02 - 3.92 (m, 3H), 1.43 - 1.35 (m, 3H), 1.08 (t, J = 7.2 Hz, 3H). |
| | MS [M+H]⁺ 534.2 | |
| 103 | 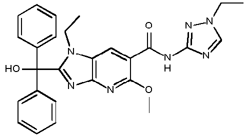 | 1-ethyl-N-(1-ethyl-1H-1,2,4-triazol-3-yl)-2-(hydroxydiphenylmethyl)-5-methoxy-1H-imidazo[4,5-b]pyridine-6-carboxamide |
| | MW 497.6 | 1H NMR (700 MHz, DMSO-d6) δ 10.36 (s, 1H), 8.43 - 8.37 (m, 1H), 8.35 - 8.29 (m, 1H), 7.37 - 7.28 (m, 11H), 4.27 (q, J = 7.2 Hz, 2H), 4.18 - 4.12 (m, 2H), 4.02 - 3.92 (m, 3H), 1.43 - 1.35 (m, 3H), 1.02 (t, J = 7.1 Hz, 3H). |
| | MS [M+H]⁺ 498.2 | |
| 104 | 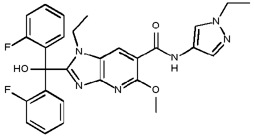 | 2-[bis(2-fluorophenyl)(hydroxy)methyl]-1-ethyl-N-(1-ethyl-1H-pyrazol-4-yl)-5-methoxy-1H-imidazo[4,5-b]pyridine-6-carboxamide |
| | MW 532.6 | 1H NMR (700 MHz, DMSO-d6) δ 10.14 (s, 1H), 8.37 (s, 1H), 8.06 - 8.05 (m, 1H), 7.62 - 7.61 (m, 1H), 7.45 - 7.41 (m, 2H), 7.40 (s, 1H), 7.36 - 7.32 (m, 2H), 7.25 - 7.22 (m, 2H), 7.18 - 7.13 (m, 2H), 4.28 (q, J = 7.1 Hz, 2H), 4.12 (q, J = 7.3 Hz, 2H), 3.99 (s, 3H), 1.36 (t, J = 7.3 Hz, 3H), 1.09 (t, J = 7.1 Hz, 3H). |
| | MS [M+H]⁺ 533.2 | |
| 105 | 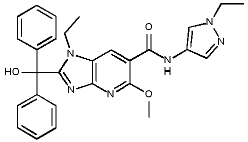 | 1-ethyl-N-(1-ethyl-1H-pyrazol-4-yl)-2-(hydroxydiphenylmethyl)-5-methoxy-1H-imidazo[4,5-b]pyridine-6-carboxamide |
| | MW 496.6 | 1H NMR (700 MHz, DMSO-d6) δ 10.13 (s, 1H), 8.35 (s, 1H), 8.06 - 8.04 (m, 1H), 7.61 - 7.60 (m, 1H), 7.37 - 7.34 (m, 4H), 7.32 - 7.28 (m, 6H), 7.28 (s, 1H), 4.27 (q, J = 7.2 Hz, 2H), 4.11 (q, J = 7.2 Hz, 2H), 4.00 (s, 3H), 1.36 (t, J = 7.3 Hz, 3H), 1.03 (t, J = 7.1 Hz, 3H). |
| | MS [M+H]⁺ 497.2 | |
| 106 | 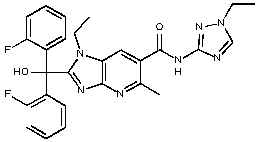 | 2-[bis(2-fluorophenyl)(hydroxy)methyl]-1-ethyl-N-(1-ethyl-1H-1,2,4-triazol-3-yl)-5-methyl-1H-imidazo[4,5-b]pyridine-6-carboxamide |
| | MW 517.5 | 1H NMR (500 MHz, DMSO-d6) δ 10.75 - 10.72 (m, 1H), 8.41 - 8.38 (m, 1H), 8.11 - 8.07 (m, 1H), 7.46 - 7.41 (m, 2H), 7.38 (s, 1H), 7.37 - 7.32 (m, 2H), 7.25 - 7.21 (m, 2H), 7.17 - 7.12 (m, 2H), 4.25 (q, J = 7.1 Hz, 2H), 4.16 (q, J = 7.3 Hz, 2H), 2.61 (s, 3H), 1.39 (t, J = 7.2 Hz, 3H), 1.11 (t, J = 7.1 Hz, 3H). |
| | MS [M+H]⁺ 518.2 | |
| 107 | 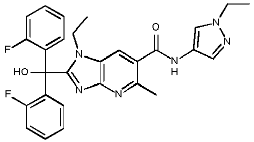 | 2-[bis(2-fluorophenyl)(hydroxy)methyl]-1-ethyl-N-(1-ethyl-1H-pyrazol-4-yl)-5-methyl-1H-imidazo[4,5-b]pyridine-6-carboxamide |
| | MW 516.6 | 1H NMR (500 MHz, DMSO-d6) δ 10.48 - 10.46 (m, 1H), 8.10 - 8.09 (m, 1H), 8.07 - 8.06 (m, 1H), 7.51 - 7.50 (m, 1H), 7.48 - 7.47 (m, 1H), 7.46 - 7.41 (m, 2H), 7.37 - 7.32 (m, 2H), 7.26 - 7.22 (m, 2H), 7.18 - 7.13 (m, 2H), 4.24 (q, J = 7.1 Hz, 2H), 4.12 (q, J = 7.3 Hz, 2H), 2.58 (s, 3H), 1.35 (t, J = 7.3 Hz, 3H), 1.10 (t, J = 7.1 Hz, 3H). |
| | MS [M+H]⁺ 517.2 | |
| 108 | 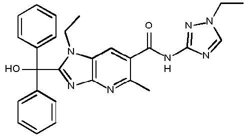 | 1-ethyl-N-(1-ethyl-1H-1,2,4-triazol-3-yl)-2-(hydroxydiphenylmethyl)-5-methyl-1H-imidazo[4,5-b]pyridine-6-carboxamide |
| | MW 481.6 | 1H NMR (500 MHz, DMSO-d6) δ 10.81 (s, 1H), 8.44 - 8.40 (m, 1H), 8.13 - 8.07 (m, 1H), 7.38 - 7.28 (m, 11H), 4.23 (q, J = 7.0 Hz, 2H), 4.15 (q, J = 7.3 Hz, 2H), 2.60 (s, 3H), 1.42 - 1.35 (m, 3H), 1.03 (t, J = 7.1 Hz, 3H). |
| | MS [M+H]⁺ 482.2 | |
| 109 | 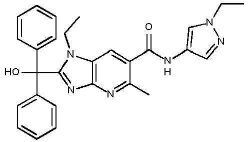 | 1-ethyl-N-(1-ethyl-1H-pyrazol-4-yl)-2-(hydroxydiphenylmethyl)-5-methyl-1H-imidazo[4,5-b]pyridine-6-carboxamide |
| | MW 480.6 | 1H NMR (500 MHz, DMSO-d6) δ 10.38 (s, 1H), 8.05 (s, 1H), 8.04 - 8.03 (m, 1H), 7.50 - 7.49 (m, 1H), 7.37 - 7.26 (m, 11H), 4.23 (q, J = 7.1 Hz, 2H), 4.12 (q, J = 7.2 Hz, 2H), 2.60 (s, 3H), 1.36 (t, J = 7.3 Hz, 3H), 1.06 (t, J = 7.0 Hz, 3H). |
| | MS [M+H]⁺ 481.2 | |
| 110 | 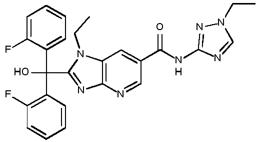 | 2-[bis(2-fluorophenyl)(hydroxy)methyl]-1-ethyl-N-(1-ethyl-1H-1,2,4-triazol-3-yl)-1H-imidazo[4,5-b]pyridine-6-carboxamide |
| | MW 503.5 | 1H NMR (500 MHz, DMSO-d6) δ 10.86 (s, 1H), 8.94 (d, J = 2.0 Hz, 1H), 8.59 (d, J = 2.1 Hz, 1H), 8.44 (s, 1H), 7.46 (s, 1H), 7.46 - 7.41 (m, 2H), 7.40 - 7.35 (m, 2H), 7.26 - 7.22 (m, 2H), 7.18 - 7.13 (m, 2H), 4.32 (q, J = 7.1 Hz, 2H), 4.18 (q, J = 7.3 Hz, 2H), 1.42 (t, J = 7.2 Hz, 3H), 1.16 (t, J = 7.1 Hz, 3H). |
| | MS [M+H]⁺ 504.2 | |
| 111 | 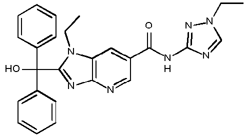 | 1-ethyl-N-(1-ethyl-1H-1,2,4-triazol-3-yl)-2-(hydroxydiphenylmethyl)-1H-imidazo[4,5-b]pyridine-6-carboxamide |
| | MW 467.5 | 1H NMR (500 MHz, DMSO-d6) δ 10.85 (s, 1H), 8.94 (d, J = 2.1 Hz, 1H), 8.57 (d, J = 2.1 Hz, 1H), 8.43 (s, 1H), 7.38 - 7.28 (m, 11H), 4.30 (q, J = 7.1 Hz, 2H), 4.18 (q, J = 7.3 Hz, 2H), 1.41 (t, J = 7.3 Hz, 3H), 1.09 (t, J = 7.1 Hz, 3H). |
| | MS [M+H]⁺ 468.1 | |
| 112 | 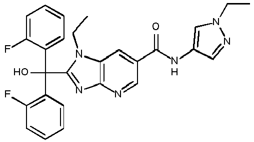 | 2-[bis(2-fluorophenyl)(hydroxy)methyl]-1-ethyl-N-(1-ethyl-1H-pyrazol-4-yl)-1H-imidazo[4,5-b]pyridine-6-carboxamide |
| | MW 502.5 | 1H NMR (500 MHz, DMSO-d6) δ 10.51 (s, 1H), 8.97 (d, J = 2.0 Hz, 1H), 8.50 (d, J = 2.1 Hz, 1H), 8.07 - 8.06 (m, 1H), 7.61 - 7.60 (m, 1H), 7.46 (s, 1H), 7.46 - 7.41 (m, 2H), 7.40 - 7.35 (m, 2H), 7.26 - 7.22 (m, 2H), 7.18 - 7.12 (m, 2H), 4.33 (q, J = 7.1 Hz, 2H), 4.13 (q, J = 7.3 Hz, 2H), 1.37 (t, J = 7.3 Hz, 3H), 1.16 (t, J = 7.1 Hz, 3H). |
| | MS [M+H]⁺ 503.2 | |
| 113 | 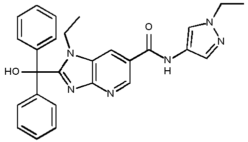 | 1-ethyl-N-(1-ethyl-1H-pyrazol-4-yl)-2-(hydroxydiphenylmethyl)-1H-imidazo[4,5-b]pyridine-6-carboxamide |
| | MW | 1H NMR (400 MHz, DMSO-d6) δ 10.51 (s, 1H), 8.97 (d, J = 2.0 Hz, 1H), 8.48 (d, J = 2.1 Hz, 1H), 8.07 - 8.06 (m, 1H), 7.61 - 7.59 (m, 1H), 7.39 - 7.27 (m, 11H), 4.30 (q, J = 7.1 Hz, 2H), 4.13 (q, J = 7.2 Hz, 2H), 1.37 (t, J = 7.2 Hz, 3H), 1.10 (t, J = 7.1 Hz, 3H). |
| | MS [M+H]⁺ | |
| 114 | 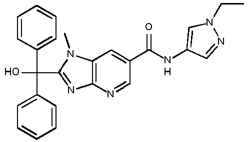 | N-(1-ethyl-1 H-pyrazol-4-yl)-2-(hydroxydiphenylmethyl)-1-methyl-1 H-imidazo[4,5-b]pyridine-6-carboxamide |
| | MW 452.5 | 1H NMR (500 MHz, DMSO-d6) δ 10.52 (s, 1H), 8.98 (d, J = 2.1 Hz, 1H), 8.50 (d, J = 2.1 Hz, 1H), 8.07 - 8.06 (m, 1H), 7.60 - 7.59 (m, 1H), 7.38 - 7.28 (m, 11H), 4.13 (q, J = 7.3 Hz, 2H), 3.67 (s, 3H), 1.37 (t, J = 7.3 Hz, 3H). |
| | MS [M+H]⁺ 453.1 | |

### Biological Activity

### Biochemical activity ACSS2 assay (IC₅₀ ACSS2 biochemical)

The biochemical activity assay for ACSS2 is based on the detection of released AMP by AMP-Glo^{™} Assay kit (Promega, Madison). The assay is run in three steps: the enzymatic reaction in which human rec ACSS2 activates acetate with ATP and Coenzyme A as cosubstrates to acetyl-CoA thereby releasing AMP and the detection reaction of AMP by AMP Glo assay in which after the destruction of the residual ATP with AMP Glo reagent 1 produced AMP is converted to ATP that is measured in a luciferase assay system (detection reagent). The ACSS2 activity correlates with the detected luminescence signal.

The assay was performed in Perkin Elmer 384well white Proxiplates in a total volume of 8 µl.

1 nM (fc) C-term myc tagged ACSS2 (human, recombinant, Origene, Rockville, US) and a mixture of 100 µM (fc) ATP, 100 µM (fc) Coenzyme A and 500 µM (fc) sodium acetate were incubated in a total volume of 5 µl (50 mM Hepes, 1 mM Mg-chloride, 150 mM NaCl, 1 mM DTT, 0.01 % (w/v) BSA, 0.3 % DMSO, pH 7.5) in the absence or presence of the test compound (10 dilution concentrations, start conc 30 µM) for 180 min at 37°C. The reaction was stopped and residual ATP destroyed by the addition of 1 µl AMP Glo reagent solution (Promega, Madison, US). After 1h incubation at room temperature 2 µl of AMP Glo detection reagent was added and the assay was incubated for 0.75 hr at room temperature. The luminescence signal was measured with an Envision multimode reader (Perkin Elmer LAS Germany GmbH) at 700 nm in luminescence mode. The full value used was the inhibitor-free reaction. The pharmacological zero value was generated by addition of ACSS2 inhibitor (Ac-CoA Synthase Inhibitor - CAS 508186-14-9 - Calbiochem) in a final concentration of 5 µM. The inhibitory values (IC50) were determined using the program Assay Analyzer^{®} from GeneData.

Experimental data of the compounds shown in Table 1 in the IC50 ACSS2 biochemical assay are shown in Table 2 below and classified in the following groups:

| | |
|---|---|
| Group A | IC₅₀ is in the range of 0.01 nM to < 1nM |
| Group B | IC₅₀ is in the range of 1 nM to < 10nM |
| Group C | IC₅₀ is in the range of 10 nM to < 100 nM |
| Group D | IC₅₀ is in the range of 100 nM to < 10000 nM |

### Cellular assay of ¹⁴C acetate incorporation into fatty acids (IC₅₀ ACSS2 cellular lipids)

This protocol describes a cellular assay capable to quantify endogenous fatty acid synthesis activity in the human HCT-15 cancer cell line following the incorporation of extracellularly administered radiolabeled ¹⁴C acetate with a scintillation proximity based read out on extracted fatty acids.

HCT-15 cells were grown in RPMI 1640 (Gibco) supplemented with 2mM glutamine, 1 mM sodium pyruvate 10 mM HEPES and 10 % FCS (heat-inactivated) passaged every 2-3 days to remain a subconfluent, vital culture. A working cell bank was prepared with each aliquot containing 1x10⁷ viable cells. After thawing, these cells are dilute immediately 1:20 in pre-warmed (37°C) culture medium. Cells are collected by centrifugation at 200 x g for 5 min, and the supernatant is replaced by 30 mL of fresh culture medium per aliquot to obtain a cell suspension containing 350000 cells/ml. From this cell suspension 100 µL are dispersed into each well of a collagen coated 96 well plate, (black, clear bottom, PS, F-bottom, Greiner) and culture for 24 h (37°C, 5% CO₂). After incubation the culture medium is removed and the wells are washed once with 100 µL PBS++ (supplemented with Mg²⁺/ Ca²⁺, Gibco) and 50 µL assay medium (RPMI 1640 + 10 mM HEPES) are added to each well. Compounds serial dilutions are prepared from 10mM stock solutions in DMSO using a fixed dilution factor (usually 1:3). After preparation of the dilution series in DMSO, the individual dilutions are further diluted in the assay medium. This working dilution is prepared such, that the concentration is 7x of the final concentration in the assay. The maximal DMSO concentration in the final assay is 0.1%.

To each well 10 µL compound working dilution or DMSO blank are added and the cells are cultured for 2 h (37°C, 5% CO₂). Then 10 µL AlamarBlue reagent (Invitrogen) containing 14 µCi/mL ¹⁴C-acetate (Perkin Elmer) are added to each well (70µl total volume) and cells are again cultured for 2 h. Cell viability is verified by measuring the AlamarBlue fluorescence (Tecan Safire, ex: 544nm / em: 590nm). After incubation the culture medium is removed and the wells are carefully washed once with cold 100 µL PBS++. For cell lysis and fatty acid recovery 50 µl 0.1 M NaOH and 0.1% Triton-X100 are added per well. The plate is sealed with PlateLoc (clear peelable heat seal, Agilent) and incubate at 70 °C for 16-24 h. After cooling to room temperature, the plates are centrifuged for 1 min at 1000rpm and the plate seals are removed. For acidification 150µL 0.1 M HCl are added to each well, mixed and 150µL of the mixture are transferred into a 96 well FlashPlate (Perkin Elmer). The plates are sealed with TopSeal-A (Perkin Elmer). To allow for the fatty acid binding to the well surfaces the plates are incubate for 4 h at 70°C. After cooling to room temperature, the plates are centrifuged for 1 min at 1000rpm and stored for 0.5-2h at room temperature in the dark. Radioactivity from ¹⁴C incorporation into fatty acids in the wells is measured as CPM count in a MicroBeta scintillation counter (Perkin Elmer).

Experimental data of the compounds shown in Table 1 in the IC₅₀ ACSS2 cellular lipids assay are shown in Table 2 below and classified in the following groups:

| | |
|---|---|
| Group A | IC₅₀ is in the range of 0.01 nM to < 1nM |
| Group B | IC₅₀ is in the range of 1 nM to < 10nM |
| Group C | IC₅₀ is in the range of 10 nM to < 100 nM |
| Group D | IC₅₀ is in the range of 100 nM to < 10000 nM |

### Cellular assay of ¹⁴C acetate incorporation into histones (IC₅₀ ACSS2 cellular Histone)

This protocol describes a cellular assay capable to quantify endogenous histone acetylation activity in the human HCT-15 cancer cell line following the incorporation of extracellularly administered radiolabeled ¹⁴C acetate with a scintillation based read out on acid extracted histones.

HCT-15 cells are trypsinized, washed and suspended in DMEM culture medium supplemented with penicillin/streptomycin (100U/mL), sodium pyruvate (1mM), and 10% FBS
Compounds serial dilutions are prepared in 96-well V-bottom microplate from 10mM stock solutions in DMSO. From these dilutions 0.5µl are transferred to a fresh plate including pure DMSO as negative control. The dilutions are prepared such, that the final concentration in the assay is 1/400 of the concentration in the serial dilution. The final DMSO concentration in the assay is 0.25 %.

The cell suspension is seeded at densities of 2x10⁵ cells per 170µl into individual wells of a 96 well plate each containing 0.5µl aliquots of compound serial dilutions and are kept for 1h in a cell incubator. To each well 30 µL of a mixture containing 0,5mCi/mL of ¹⁴C-acetate with full medium are added. Then, the wells are incubated for 3 hrs in the cell incubator.

Further steps of the procedure should be conducted on ice or instrumentations precooled to 4°C. Cells are sedimented in the 96-well V-bottom microplate by centrifugation (Eppendorf 5804 R) at 1200 rpm for 5 min. The supernatant is removed and the cells are washed twice with 200µL of PBS-NaB (5 mM sodium butyrate) buffer by iterative resuspension and centrifugation. Finally, the cells are resuspended in 50µL of TEB (PBS-NaB + 0.5 % Triton X-100) and left on ice for 10 minutes. After centrifugation at 2300rpm for 10 minutes at 4°C the supernatant is removed, the remaining pellet is suspended in 50 µL of 0,2M HCl and incubated over night at 4°C. After incubation the well plate is shaken for 2 minutes in a MTP plate shaker set to 1200 rpm and then centrifuged at 3700 rpm for 10 mins. The lysate is carefully aspirated (~43µL) and transferred into a white MTP plate (Greiner bio-one 65509). 90µL of Ultima Gold XR scintillation cocktail are added and the plates are sealed with a transparent cover tape before mixing vigorously using plate shaker set to 1200 rpm. Radioactivity from ¹⁴C incorporation in the wells is measured as CPM count in a MicroBeta Trilux luminescence counter.

Experimental data of the compounds shown in Table 1 in the IC₅₀ ACSS2 cellular Histone assay are shown in Table 2 below and classified in the following groups:

| | |
|---|---|
| Group A | IC₅₀ is in the range of 0.01 nM to < 1nM |
| Group B | IC₅₀ is in the range of 1 nM to < 10nM |
| Group C | IC₅₀ is in the range of 10 nM to < 100 nM |
| Group D | IC₅₀ is in the range of 100 nM to < 10000 nM |

### Micronucleus assay (MNT)

The in vitro MNT assay is performed in CHO-K1 cells due to their stable and well-characterized karyotype, high sensitivity and suitability for a high content imaging approach. They have a basal spontaneous micronucleus frequency of 3-4%.

24 hrs after plating CHO-K1 cells are treated with test compounds for 24 hrs (in duplicates; fixed concentration ranges from 0.2 µM to 100 µM in 2-fold dilution steps). After a medium change cells are incubated for 24 hrs with Cytochalasin B to block cytokinesis and are then fixed and nuclei / micronuclei visualized with a DNA stain. Images are acquired with the Molecular Devices high content imagers IXU or IXM and are analyzed with the dedicated MetaXpress software micronucleus module. Criteria for scoring of micronuclei (MN) are the following:
- diameter of the MN should be less than 1/3 of the main nucleus
- MN should be separated from or marginally overlap with main nucleus (= no blebs)
- MN should have similar staining as the main nucleus At least 1000 binucleated cells are evaluated per treatment replicate. Mitomycin C is used as reference stimulator of micronucleus formation. Cytotoxicity is evaluated in parallel and defined by comparing total nuclei count in compound-treated samples to neutral control samples treated with vehicle only (1% DMSO) (100% cytotoxicity means all cells are dead or lost).

A test compound is regarded as POSITIVE if generation of micronuclei and exhibition of less than 60% cytotoxicity at the same concentration can be observed. (A data value is considered as positive if it contains more micronuclei than the mean of the neutral controls plus 3x standard deviation.) A compound is reported as NEGATIVE if no generation of micronuclei at concentrations exhibiting less than 60% cytotoxicity can be observed and at least one tested concentration gives more than 60% cytotoxicity.

A compound is reported as PN (putative negative) if not generating micronuclei and exhibiting less than 60% cytotoxicity at any tested concentration.

A compound is reported as ND (not determinable) at a certain concentration - independent of generating micronuclei or not - if it exhibits more than 60% cytotoxicity at this concentration.

Compounds are scored only in the soluble concentration range. Experimental data of the compounds shown in Table 1 in the MNT assay are shown in Table 2 below and classified in the following groups:

| | |
|---|---|
| Group A | negative, putative negative (micronuclei detected beyond threshold of 60% cyctotoxicity) |
| Group B | positive |

**Table 2**

| **Compound No.** | **IC50 biochemical** | **IC50 cellular lipids** | **IC50 cellular Histone** | **MNT** |
|---|---|---|---|---|
| 1 | A | A | A | A |
| 2 | B | A | A | A |
| 3 | B | C | B | A |
| 4 | C | C | B | A |
| 5 | B | A | B | A |
| 6 | C | B | B | A |
| 7 | B | | B | A |
| 8 | C | C | B | A |
| 9 | B | A | A | B |
| 10 | C | C | C | A |
| 11 | B | B | A | A |
| 12 | B | B | B | |
| 13 | C | D | C | A |
| 14 | B | B | B | A |
| 15 | C | | C | A |
| 16 | C | C | B | A |
| 17 | C | | C | |
| 18 | D | | D | A |
| 19 | C | C | C | A |
| 20 | B | B | A | A |
| 21 | D | | | A |
| 22 | C | | C | A |
| 23 | B | C | B | A |
| 24 | B | B | A | A |
| 25 | B | B | A | A |
| 26 | D | | | |
| 27 | B | | | |
| 28 | B | B | A | A |
| 29 | C | | C | A |
| 30 | B | A | B | A |
| 31 | B | B | A | A |
| 32 | C | | | |
| 33 | C | | | |
| 34 | B | | | |
| 35 | B | | | |
| 36 | A | | | |
| 37 | A | | | |
| 38 | B | | | |
| 39 | B | | | |
| 40 | C | | | |
| 41 | B | | B | |
| 42 | C | | B | |
| 43 | C | | C | |
| 44 | B | | B | |
| 45 | B | | B | |
| 46 | C | D | D | |
| 47 | B | C | A | |
| 48 | D | | D | |
| 49 | C | | B | |
| 50 | B | D | B | |
| 51 | B | B | A | |
| 52 | C | | C | |
| 53 | B | A | A | inconclusive |
| 54 | C | | C | |
| 55 | B | A | A | |
| 56 | B | | | |
| 57 | D | | C | |
| 58 | C | | C | |
| 59 | B | A | A | |
| 60 | A | B | A | |
| 61 | C | A | C | |
| 62 | C | B | C | |
| 63 | D | | C | |
| 64 | A | A | C | |
| 65 | D | C | B | |
| 66 | D | B | C | |
| 67 | C | | C | |
| 68 | C | D | C | |
| 69 | B | B | A | |
| 70 | C | | C | |
| 71 | A | | A | |
| 72 | B | | A | |
| 73 | B | C | C | |
| 74 | B | | B | |
| 75 | A | A | B | |
| 76 | B | B | B | |
| 77 | B | | B | |
| 78 | B | | A | |
| 79 | C | | B | |
| 80 | C | | C | |
| 81 | B | | C | |
| 82 | B | | A | |
| 83 | B | B | A | |
| 84 | C | | C | |
| 85 | C | | C | |
| 86 | B | | B | |
| 87 | B | | B | |
| 88 | C | | C | |
| 89 | B | B | B | A |
| 90 | B | B | B | A |
| 91 | B | B | A | A |
| 92 | C | B | B | |
| 93 | B | | C | |
| 94 | B | B | B | A |
| 95 | C | | C | |
| 96 | B | | C | |
| 97 | B | | C | |
| 98 | B | C | B | A |
| 99 | B | | A | |
| 100 | A | | B | |
| 101 | B | | A | |
| 102 | A | B | A | A |
| 103 | B | B | A | A |
| 104 | B | B | B | |
| 105 | B | | B | |
| 106 | B | | B | |
| 107 | B | C | A | |
| 108 | B | C | B | |
| 109 | B | | B | |
| 110 | B | | B | |
| 111 | B | D | D | |
| 112 | C | | C | |
| 113 | C | | C | |
| 114 | C | | C | |
| Reference Compound | D | B | A | B |

The following examples relate to medicaments:

### Example A: Injection vials

A solution of 100 g of an active ingredient of the formula I-a, I-b or I-c and 5 g of disodium hydrogenphosphate in 3 l of bidistilled water is adjusted to pH 6.5 using 2 N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient of the formula I-a, I-b or I-c with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient of the formula I-a, I-b or I-c, 9.38 g of NaH₂PO₄ · 2 H₂O, 28.48 g of Na₂HPO₄ · 12 H₂O and 0.1 g of benzalkonium chloride in 940 mL of bidistilled water. The pH is adjusted to 6.8, and the solution is made up to 1 l and sterilised by irradiation. This solution can be used in the form of eye drops.

### Example D: Ointment

500 mg of an active ingredient of the formula I-a, I-b or I-c are mixed with 99.5 g of Vaseline under aseptic conditions.

### Example E: Tablets

A mixture of 1 kg of active ingredient of the formula I-a, I-b or I-c, 4 kg of lactose, 1.2 kg of potato starch, 0.2 kg of talc and 0.1 kg of magnesium stearate is pressed in a conventional manner to give tablets in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient of the formula I-a, I-b or I-c are introduced into hard gelatine capsules in a conventional manner in such a way that each capsule contains 20 mg of the active ingredient.

### Example H: Ampoules

A solution of 1 kg of active ingredient of the formula I-a, I-b or I-c in 60 l of bidistilled water is sterile filtered, transferred into ampoules, lyophilised under sterile conditions and sealed under sterile conditions. Each ampoule contains 10 mg of active ingredient.

## Claims

1. An amide derivative of formula I-a, I-b or I-c 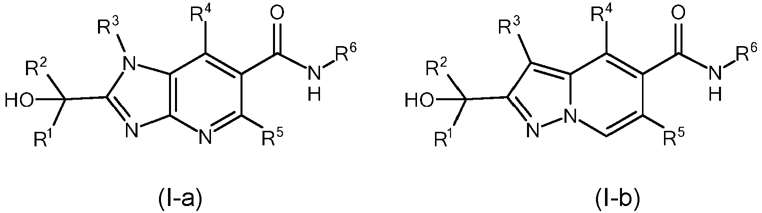 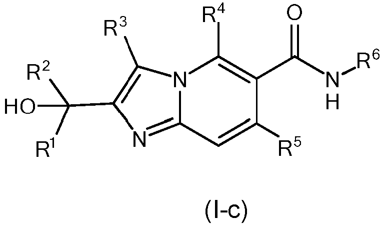
wherein independently from each other
R¹ denotes Ar^{A} or Hetar^{A};
R² denotes Ar^{B} or Hetar^{B};
R³ denotes C₁₋₆-aliphatic or -O-C₁₋₆-aliphatic;
R⁴ denotes H, D, C₁₋₆-aliphatic or -O-C₁₋₆-aliphatic;
R⁶ denotes H, D, C₁₋₆-aliphatic, -O-C₁₋₆-aliphatic or halogen;
R⁶ denotes Hetar^{C} or -CH₂-Hetar^{C};
Ar^{A} is a mono- or biaryl with 5, 6, 7, 8, 9, 10, 11 ring carbon atoms, wherein that aryl may be unsubstituted or substituted with substituents R^{A1}, R^{A2}, R^{A3}, R^{A4} and/or R^{A5} which may be the same or different;
Ar^{B} is a mono- or biaryl with 5, 6, 7, 8, 9, 10, 11 ring carbon atoms, wherein that aryl may be unsubstituted or substituted with substituents R^{B1}, R^{B2}, R^{B3}, R^{B4} and/or R^{B5} which may be the same or different;
Hetar^{A} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{A1}, R^{A2}, R^{A3}, R^{A4} and/or R^{A5} which may be the same or different;
Hetar^{B} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{B1}, R^{B2}, R^{B3}, R^{B4} and/or R^{B5} which may be the same or different;
Hetar^{C} is a mono- or bicyclic heteroaryl with 5, 6, 7, 8, 9, 10, 11 ring atoms wherein 1, 2, 3, 4, 5 of said ring atoms is/are a hetero atom(s) selected from N, O and/or S and the remaining are carbon atoms, wherein that heteroaryl may be unsubstituted or substituted with substituents R^{C1}, R^{C2}, R^{C3}, R^{C4} and/or R^{C5} which may be the same or different;
R^{A1}, R^{A2}, R^{A3}, R^{A4}, R^{A5}, R^{B1}, R^{B2}, R^{B3}, R^{B4}, R^{B5}, R^{C1}, R^{C2}, R^{C3}, R^{C4}, R^{C5} are independently from each other H, D, halogen, C₁₋₆-aliphatic, -O-C₁₋₆-aliphatic;
halogen denotes F, Cl, Br or I; or any N-oxide, solvate, tautomer or stereoisomer thereof and/or any pharmaceutically acceptable salt of each of the foregoing, including mixtures thereof in all ratios.

2. The amide derivative according to claim 1, or any N-oxide solvate, tautomer or stereoisomer thereof and/or any pharmaceutically acceptable salt of each of the foregoing, including mixtures thereof in all ratios, wherein
R¹ and R² have the same meaning.

3. The amide derivative according to claim 1, or any N-oxide solvate, tautomer or stereoisomer thereof and/or any pharmaceutically acceptable salt of each of the foregoing, including mixtures thereof in all ratios, wherein
R¹ and R² have a different meaning.

4. The amide derivative according to any preceding claim, or any N-oxide, solvate, tautomer or stereoisomer thereof and/or any pharmaceutically acceptable salt of each of the foregoing, including mixtures thereof in all ratios,
wherein
Ar^{A} is phenyl which may be unsubstituted or mono- or di-substituted with R^{A1} and/or R^{A2} wherein R^{A1} and/or R^{A2} are as defined in any preceding claim; or trideuterophenyl, tetradeuterophenyl or pentadeuterophenyl;
Ar^{B} is phenyl which may be unsubstituted or mono- or di-substituted with R^{B1} and/or R^{B2} wherein R^{B1} and/or R^{B2} are as defined in any preceding claim; or trideuterophenyl, tetradeuterophenyl or pentadeuterophenyl;
Hetar^{A} is a monocyclic heteroaryl with 5 or 6 ring atoms or a bicyclic heteroaryl with 10 ring atoms wherein 1 or 2 of said ring atoms is/are a hetero atom(s) selected from N, O and/or S and the remaining are carbon atoms, wherein that heteroaryl may be unsubstituted or substituted with substituents R^{A1} and/or R^{A2} which may be the same or different;
Hetar^{B} is a monocyclic heteroaryl with 5 or 6 ring atoms wherein 1 or 2 of said ring atoms is/are a hetero atom(s) selected from N, O and/or S and the remaining are carbon atoms, wherein that heteroaryl may be unsubstituted or substituted with substituents R^{B1} and/or R^{B2} which may be the same or different.

5. The amide derivative according to claim 4, or any N-oxide solvate, tautomer or stereoisomer thereof and/or any pharmaceutically acceptable salt of each of the foregoing, including mixtures thereof in all ratios, wherein
R^{A1}, R^{A2}, R^{B1}, R^{B2} are independently from each other H, D, F, Cl, C₁₋₄-aliphatic, -O-C₁₋₄-aliphatic.

6. The amide derivative according to any preceding claim, or any N-oxide, solvate, tautomer or stereoisomer thereof and/or any pharmaceutically acceptable salt of each of the foregoing, including mixtures thereof in all ratios,
wherein independently from each other
R³ denotes substituted or unsubstituted C₁₋₄-alkyl;
R⁴ denotes H;
R⁵ denotes H, C₁₋₄-alkyl, -O-C₁₋₄-alkyl, F or Cl.

7. The amide derivative according to any preceding claim, or any N-oxide, solvate, tautomer or stereoisomer thereof and/or any pharmaceutically acceptable salt of each of the foregoing, including mixtures thereof in all ratios,
wherein
R⁶ denotes -CH₂-Hetar^{C} or Hetar^{C}
Hetar^{C} is a monocyclic heteroaryl with 5 or 6 ring atoms wherein 1, 2 or 3 of said ring atoms is/are a hetero atom(s) selected from N, O and/or S and the remaining are carbon atoms, wherein that heteroaryl may be unsubstituted or substituted with substituents R^{C1} and/or R^{C2} which may be the same or different;
R^{C1}, R^{C2} denote H or substituted or unsubstituted C₁₋₄-alkyl wherein at least one of R^{C1}, and R^{C2} is not H.

8. The amide derivative according to any preceding claim, or any N-oxide, solvate, tautomer or stereoisomer thereof and/or any pharmaceutically acceptable salt of each of the foregoing, including mixtures thereof in all ratios,
wherein
R⁶ denotes Hetar^{C};
Hetar^{C} is a monocyclic heteroaryl with 5 ring atoms wherein 2 or 3 of said ring atoms is/are a hetero atom(s) selected from N and/or O and the remaining are carbon atoms, wherein that heteroaryl may be unsubstituted or substituted with one substituent R^{C1},
R^{C1} denotes ethyl, 2-aminoethyl, 2-hydroxyethyl, 2-methoxyethyl.

9. The amide derivative according to any preceding claim, or any N-oxide, solvate, tautomer or stereoisomer thereof and/or any pharmaceutically acceptable salt of each of the foregoing, including mixtures thereof in all ratios,
wherein independently from each other
R³ denotes methyl, ethyl, 2-dimethylaminoethyl, 2-hydroxyethyl, 2-methoxyethyl;
R⁴ denotes H;
R⁵ denotes H, methyl, ethyl, methoxy, F or Cl;
R⁶ denotes Hetar^{C};
Ar^{A} is phenyl; mono-, di-, tri-, tetra- or pentadeuterophenyl, preferably pentadeuterophenyl; fluorophenyl, preferably 2-fluorophenyl; methylphenyl (tolyl), preferably 2-methylphenyl; methoxyphenyl, preferably 2-methoxyphenyl; difluoromethoxy, preferably 4-difluoromethoxy; 4-difluoromethoxy-2-fluorophenyl;
Ar^{B} phenyl; mono-, di-, tri-, tetra- or pentadeuterophenyl, preferably pentadeuterophenyl; fluorophenyl, preferably 2-fluorophenyl; methylphenyl (tolyl), preferably 2-methylphenyl; difluoromethoxy, preferably 4-difluoromethoxy;
Hetar^{A} methylpyrazolyl, preferably 1-methlypyrazol-3-yl, 1-methylpyrazol-4-yl; thien-2-yl, thien-3-yl; methylthienyl, preferably 5-methylthien-2-yl; thiazolyl, preferably 1,3-thiazol-2-yl; pyridinyl, preferably pyridin-2-yl; pyrimidinyl, preferably pyrimidin-4-yl; pyridazinyl, preferably pyridazin-3-yl; quinolinyl, preferably quinolin-2-yl;
Hetar^{B} thien-2-yl, thien-3-yl; methylthienyl, preferably 5-methylthien-2-yl; pyridinyl, preferably pyridin-2-yl;
Hetar^{C} ethylpyrazolyl, preferably 1-ethylpyrazol-3-yl, 1-ethylpyrazol-4-yl; hydroxyethylpyrazolyl, preferably 1-(2-hydroxyethyl)pyrazol-4-yl; methoxyethylpyrazolyl, preferably 1-(2-methyoxyethyl)pyrazol-4-yl; ethylimidazolyl, preferably 1-ethyl-1H-imidazol-4-yl; ethyloxazolyl, preferably 4-ethyl-1,3-oxazol-2-yl, 5-ethyl-1,3-oxazol-2-yl; ethyltriazolyl, preferably 1-ethyl-1H-1,2,4-triazol-3-yl, 2-ethyl-2H-1,2,3-triazol-4-yl; aminoethyltriazolyl, preferably 1-(2-aminoethyl)-1H-1,2,4-triazol-3-yl; hydroxyethyltriazolyl, preferably 1-(2hydroxyethyl)-1H-1,2,4-triazol-3-yl; methoxyethyltriazolyl, preferably 1-(2-methoxyethyl)-1H-1,2,4-triazol-3-yl; ethyloxadiazolyl, preferably 5-ethyl-1,3,4-oxadiazol-2-yl, 5-ethyl-1,2,4-oxadiazol-3-yl.

10. Amide derivative according to any preceding claim, or any N-oxide, solvate, tautomer or stereoisomer thereof and/or any pharmaceutically acceptable salt of each of the foregoing, including mixtures thereof in all ratios,
wherein the amide derivative is selected from the group consisting of the compounds shown in Table 1 (which is divided in Tables 1a, 1b, 1c and 1d).

11. Amide derivative according to any of claims 1 to 10, or any N-oxide, solvate, tautomer or stereoisomer thereof and/or any pharmaceutically acceptable salt of each of the foregoing, including mixtures thereof in all ratios, for use as a medicament.

12. Compound according to any of claims 1 to 10, or any N-oxide, solvate, tautomer or stereoisomer thereof and/or any pharmaceutically acceptable salt of each of the foregoing, including mixtures thereof in all ratios, for use in the prevention and/or treatment of a medical condition or disease that is affected by inhibiting ACSS2 wherein that medical condition or disease is selected from the group consisting of: cancer, in particular tumors including solid tumors; an inflammatory disorder or disease, in particular Crohn's disease, ulcerative colitis, idiopathic pulmonary fibrosis, muscular dystrophy, rheumatoid arthritis, and systemic sclerosis (scleroderma); a neurogenerative disorder or disease, in particular Huntington's disease; Lipid metabolism disorders, e.g. NASH (non-alcoholic steatohepatitis), NAFLD (non-alcoholic fatty liver disease) , fatty liver disease; viral infections, e.g. with cytomegalovirus; post-traumatic stress disorder (PTSD); bipolar disorder, depression, Tourettes's Syndrome, schizophrenia, obsessive-compulsive disorder, anxiety disorder, panic disorders, phobias, addiction to e.g. alcohol, tobacco, opioids, sedatives, hypnotics, anxiolytics, cocaine, cannabis, amphetamines, hallucinogens, inhalants, phencyclidine, impulse control disorders, behavioral addictions.

13. A pharmaceutical composition comprising at least one amide derivative according to any one of claims 1 to 10, or any N-oxide, solvate, tautomer or stereoisomer thereof and/or any pharmaceutically acceptable salt of each of the foregoing, including mixtures thereof in all ratios, as active ingredient, together with a pharmaceutically acceptable carrier wherein that pharmaceutical composition further comprises a second active ingredient or any of its N-oxides, solvates, tautomers or stereoisomers thereof as well as the pharmaceutically acceptable salts of each of the foregoing, including mixtures thereof in all ratios, wherein that second active ingredient is other than an amide derivative of formula I-a, I-b or I-c as defined in any one of claims 1 to 10.

14. Set (kit) comprising separate packs of
a) an effective amount of an amide derivative of formula I-a, I-b or I-c according to any one of claims 1 to 10, or any N-oxide, solvate, tautomer or stereoisomer thereof and/or any pharmaceutically acceptable salt of each of the foregoing, including mixtures thereof in all ratios; and
b) an effective amount of a further active ingredient that further active ingredient not being an amide derivative of formula I-a, I-b or I-c as defined in any one of claims 1 to 10.

15. Process for manufacturing an amide derivative of formula I-a, I-b or I-c according to any of claims 1 to 10, or any N-oxide, solvate, tautomer or stereoisomer thereof and/or any pharmaceutically acceptable salt of each of the foregoing, the process being **characterized in that**
(a) a carboxylic acid of general formula II-a 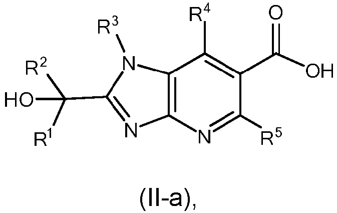
wherein R¹, R², R³, R⁴ and R⁵ are as defined in any of claims 1 to 10, is subjected to an amidation reaction with a compound of formula III:
R⁶-NH₂ (III)
wherein R⁶ is as defined in any of claims 1 to 10, optionally in the presence of a suitable catalyst, to yield the amide derivative of formula I-a: 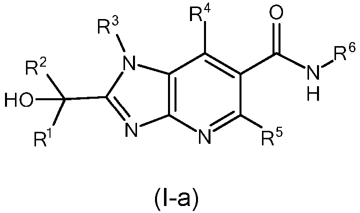
wherein R¹, R², R³, R⁴, R⁵ and R⁶ are as defined in any of claims 1 to 10; or
(b) a carboxylic acid of general formula II-b 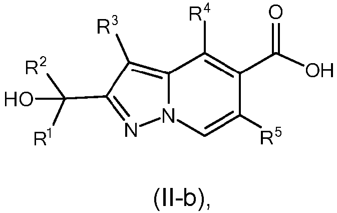
wherein R¹, R², R³, R⁴ and R⁵ are as defined in any of claims 1 to 10, is subjected to an amidation reaction with a compound of formula III:
R⁶-NH₂ (III)
wherein R⁶ is as defined in any of claims 1 to 10, optionally in the presence of a suitable catalyst, to yield the amide derivative of formula I-b: 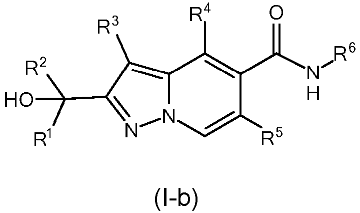
wherein R¹, R², R³, R⁴, R⁵ and R⁶ are as defined in any of claims 1 to 10;
or
c) a carboxylic acid of general formula II-c 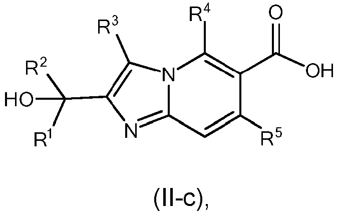
wherein R¹, R², R³, R⁴ and R⁵ are as defined in any of claims 1 to 10, is subjected to an amidation reaction with a compound of formula III:
R⁶-NH₂ (III)
wherein R⁶ is as defined in any of claims 1 to 10, optionally in the presence of a suitable catalyst, to yield the amide derivative of formula I-c: 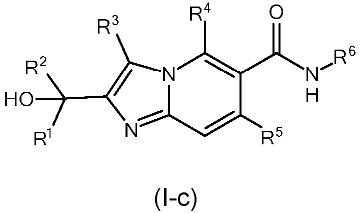
wherein R¹, R², R³, R⁴, R⁵ and R⁶ are as defined in any of claims 1 to 10.

16. Carboxylic acid of formula II-a, II-b or II-c 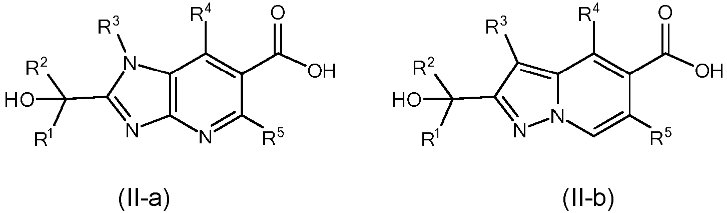 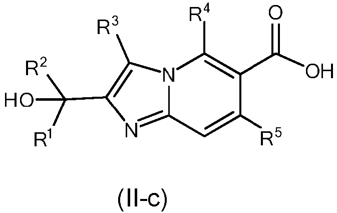 wherein R¹, R², R³, R⁴, R⁵ and R⁶ are as defined in any of claims 1 to 10.

## Patentansprüche

1. Amidderivat der Formel I-a, I-b oder I-c 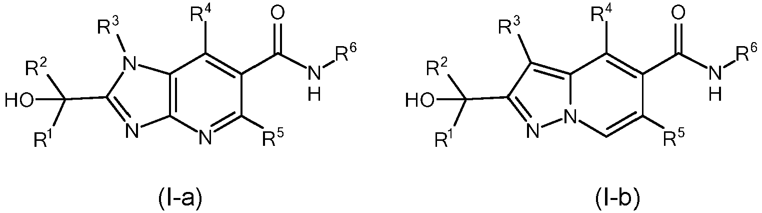 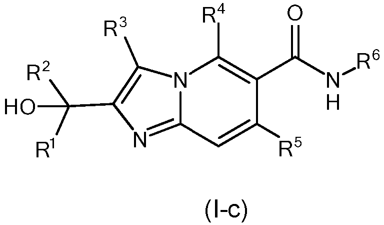
wobei unabhängig voneinander
R¹ Ar^{A} oder Hetar^{A} bedeutet;
R² Ar^{B} oder Hetar^{B} bedeutet;
R³ C₁₋₆-Aliphat oder -O-C₁₋₆-Aliphat bedeutet;
R⁴ H, D, C₁₋₆-Aliphat oder -O-C₁₋₆-Aliphat bedeutet;
R⁵ H, D, C₁₋₆-Aliphat, -O-C₁₋₆-Aliphat oder Halogen bedeutet;
R⁶ Hetar^{C} oder -CH₂-Hetar^{C} bedeutet;
Ar^{A} ein Mono- oder Biaryl mit 5, 6, 7, 8, 9, 10, 11 Ringkohlenstoffatomen ist, wobei dieses Aryl unsubstituiert oder mit Substituenten R^{A1}, R^{A2}, R^{A3}, R^{A4} und/oder **R^{A5},** die gleich oder verschieden sein können, substituiert sein kann;
Ar^{B} ein Mono- oder Biaryl mit 5, 6, 7, 8, 9, 10, 11 Ringkohlenstoffatomen ist, wobei dieses Aryl unsubstituiert oder mit Substituenten R^{B1}, R^{B2}, R^{B3}, R^{B4} und/oder R^{B5}, die gleich oder verschieden sein können, substituiert sein kann;
Hetar^{A} ein mono- oder bicyclisches Heteroaryl mit 5, 6, 7, 8, 9, 10, 11 Ringatomen ist, wobei 1, 2, 3, 4, 5 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{A1}, R^{A2}, R^{A3}, R^{A4} und/oder R^{A5}, die gleich oder verschieden sein können, substituiert sein kann;
Hetar^{B} ein mono- oder bicyclisches Heteroaryl mit 5, 6, 7, 8, 9, 10, 11 Ringatomen ist, wobei 1, 2, 3, 4, 5 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{B1}, R^{B2}, R^{B3}, R^{B4} und/oder R^{B5}, die gleich oder verschieden sein können, substituiert sein kann;
Hetar^{C} ein mono- oder bicyclisches Heteroaryl mit 5, 6, 7, 8, 9, 10, 11 Ringatomen ist, wobei 1, 2, 3, 4, 5 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{C1}, R^{C2}, R^{C3}, R^{C4} und/oder R^{C5}, die gleich oder verschieden sein können, substituiert sein kann;
R^{A1}, R^{A2}, R^{A3}, R^{A4}, R^{A5}, R^{B1}, R^{B2}, R^{B3}, R^{B4}, R^{B5}, R^{C1}, R^{C2}, R^{C3}, R^{C4}, R^{C5}unabhängig voneinander H, D, Halogen, C₁₋₆-Aliphat, -O-C₁₋₆-Aliphat sind;
Halogen F, Cl, Br oder I bedeutet;
oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen.

2. Amidderivat nach Anspruch 1, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
R¹ und R² dieselbe Bedeutung besitzen.

3. Amidderivat nach Anspruch 1, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
R¹ und R² eine unterschiedliche Bedeutung besitzen.

4. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
Ar^{A} Phenyl, das unsubstituiert oder ein- oder zweifach mit R^{A1} und/oder R^{A2} substituiert sein kann, wobei R^{A1} und/oder R^{A2} wie in einem beliebigen vorhergehenden Anspruch definiert sind; oder Trideuterophenyl, Tetradeuterophenyl oder Pentadeuterophenyl ist;
Ar^{B} Phenyl, das unsubstituiert oder ein- oder zweifach mit R^{B1} und/oder R^{B2} substituiert sein kann, wobei R^{B1} und/oder R^{B2} wie in einem beliebigen vorhergehenden Anspruch definiert sind; oder Trideuterophenyl, Tetradeuterophenyl oder Pentadeuterophenyl ist;
Hetar^{A} ein monocyclisches Heteroaryl mit 5 oder 6 Ringatomen oder ein bicyclisches Heteroaryl mit 10 Ringatomen ist, wobei 1 oder 2 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{A1} und/oder R^{A2}, die gleich oder verschieden sein können, substituiert sein kann;
Hetar^{B} ein monocyclisches Heteroaryl mit 5 oder 6 Ringatomen ist, wobei 1 oder 2 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{B1} und/oder R^{B2}, die gleich oder verschieden sein können, substituiert sein kann.

5. Amidderivat nach Anspruch 4, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
R^{A1}, R^{A2}, R^{B1}, R^{B2} unabhängig voneinander H, D, F, Cl, C₁₋₄-Aliphat, -O-C₁₋₄-Aliphat sind.

6. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei unabhängig voneinander
R³ substituiertes oder unsubstituiertes C₁₋₄-Alkyl bedeutet;
R⁴ H bedeutet;
R⁵ H, C₁₋₄-Alkyl, -O-C₁₋₄-Alkyl, F oder Cl bedeutet.

7. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
R⁶ -CH₂-Hetar^{C} oder Hetar^{C} bedeutet;
Hetar^{C} ein monocyclisches Heteroaryl mit 5 oder 6 Ringatomen ist, wobei 1, 2 oder 3 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{C1} und/oder R^{C2}, die gleich oder verschieden sein können, substituiert sein kann;
R^{C1}, R^{C2} H oder substituiertes oder unsubstituiertes C₁₋₄-Alkyl bedeuten, wobei mindestens einer von R^{C1} und R^{C2} nicht H ist.

8. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei
R⁶ Hetar^{C} bedeutet;
Hetar^{C} ein monocyclisches Heteroaryl mit 5 Ringatomen ist, wobei 2 oder 3 der Ringatome ein Heteroatom(e) ist/sind, das/die aus N und/oder O ausgewählt ist/sind, und die restlichen Kohlenstoffatome sind, wobei dieses Heteroaryl unsubstituiert oder mit einem Substituenten R^{C1} substituiert sein kann;
R^{C1} Ethyl, 2-Aminoethyl, 2-Hydroxyethyl, 2-Methoxyethyl bedeutet.

9. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei unabhängig voneinander
R³ Methyl, Ethyl, 2-Dimethylaminoethyl, 2-Hydroxyethyl, 2-Methoxyethyl bedeutet;
R⁴ H bedeutet;
R⁵ H, Methyl, Ethyl, Methoxy, F oder Cl bedeutet;
R⁶ Hetar^{C} bedeutet;
Ar^{A} Phenyl; Mono-, Di-, Tri-, Tetra- oder Pentadeuterophenyl, vorzugsweise Pentadeuterophenyl; Fluorphenyl, vorzugsweise 2-Fluorphenyl; Methylphenyl (Tolyl), vorzugsweise 2-Methylphenyl; Methoxyphenyl, vorzugsweise 2-Methoxyphenyl; Difluormethoxy, vorzugsweise 4-Difluormethoxy; 4-Difluormethoxy-2-fluorphenyl ist;
Ar^{B} Phenyl; Mono-, Di-, Tri-, Tetra- oder Pentadeuterophenyl, vorzugsweise Pentadeuterophenyl; Fluorphenyl, vorzugsweise 2-Fluorphenyl; Methylphenyl (Tolyl), vorzugsweise 2-Methylphenyl; Difluormethoxy, vorzugsweise 4-Difluormethoxy ist;
Hetar^{A} Methylpyrazolyl, vorzugsweise 1-Methlypyrazol-3-yl, 1-Methyl-pyrazol-4-yl; Thien-2-yl, Thien-3-yl; Methylthienyl, vorzugsweise 5-Methylthien-2-yl; Thiazolyl, vorzugsweise 1,3-Thiazol-2-yl; Pyridinyl, vorzugsweise Pyridin-2-yl; Pyrimidinyl, vorzugsweise Pyrimidin-4-yl; Pyridazinyl, vorzugsweise Pyridazin-3-yl; Chinolinyl, vorzugsweise Chinolin-2-yl ist;
Hetar^{B} Thien-2-yl, Thien-3-yl; Methylthienyl, vorzugsweise 5-Methylthien-2-yl; Pyridinyl, vorzugsweise Pyridin-2-yl ist;
Hetar^{C} Ethylpyrazolyl, vorzugsweise 1-Ethylpyrazol-3-yl, 1-Ethylpyrazol-4-yl; Hydroxyethylpyrazolyl, vorzugsweise 1-(2-Hydroxyethyl)pyrazol-4-yl; Methoxyethylpyrazolyl, vorzugsweise 1-(2-Methoxyethyl)pyrazol-4-yl; Ethylimidazolyl, vorzugsweise 1-Ethyl-1H-imidazol-4-yl; Ethyloxazolyl, vorzugsweise 4-Ethyl-1,3-oxazol-2-yl, 5-Ethyl-1,3-oxazol-2-yl; Ethyltriazolyl, vorzugsweise 1-Ethyl-1H-1,2,4-triazol-3-yl, 2-Ethyl-2H-1,2,3-triazol-4-yl; Aminoethyltriazolyl, vorzugsweise 1-(2-Aminoethyl)-1H-1,2,4-Triazol-3-yl; Hydroxyethyltriazolyl, vorzugsweise 1-(2-Hydroxyethyl)-1H-1,2,4-triazol-3-yl; Methoxyethyltriazolyl, vorzugsweise 1-(2-Methoxyethyl)-1H-1,2,4-triazol-3-yl; Ethyloxadiazolyl, vorzugsweise 5-Ethyl-1,3,4-oxadiazol-2-yl, 5-Ethyl-1,2,4-oxadiazol-3-yl ist.

10. Amidderivat nach einem beliebigem vorhergehenden Anspruch, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen,
wobei das Amidderivat aus der Gruppe bestehend aus den Verbindungen, die in Tabelle 1 (die in die Tabellen 1a, 1b, 1c und 1d unterteilt ist) gezeigt sind, ausgewählt ist.

11. Amidderivat nach beliebigen der Ansprüche 1 bis 10, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen, zur Verwendung als Arzneimittel.

12. Verbindung nach beliebigen der Ansprüche 1 bis 10, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen, zur Verwendung bei der Prävention und/oder Behandlung eines gesundheitlichen Leidens oder einer Erkrankung, das/die durch die Hemmung von ACSS2 beeinflusst wird, wobei dieses gesundheitliche Leiden oder diese Erkrankung ausgewählt ist aus der Gruppe bestehend aus: Krebs, insbesondere Tumoren, darunter festen Tumoren; einer Entzündungsstörung oder -krankheit, insbesondere Morbus Crohn, Colitis ulcerosa, idiopathischer Lungenfibrose, Muskeldystrophie, rheumatoider Arthritis und systemischer Sklerose (Sklerodermie); einer neurogenerativen Störung oder Erkrankung, insbesondere Huntington-Krankheit; Störungen des Fettstoffwechsels, z.B. NASH (nichtalkoholische Steatohepatitis), NAFLD (nichtalkoholische Fettleberkrankheit), Fettleberkrankheit; Virusinfektionen, z.B. mit Cytomegalovirus; posttraumatischer Belastungsstörung (PTSD); bipolarer Störung, Depression, Tourette-Syndrom, Schizophrenie, Zwangsstörung, Angststörung, Panikstörungen, Phobien, Abhängigkeit von z.B. Alkohol, Tabak, Opiaten, Sedativa, Hypnotika, Anxiolytika, Kokain, Cannabis, Amphetaminen, Halluzinogenen, Inhalaten, Phencyclidin, Störungen der Impulskontrolle, Verhaltenssuchtkrankheiten.

13. Pharmazeutische Zusammensetzung enthaltend mindestens ein Amidderivat nach irgendeinem der Ansprüche 1 bis 10, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen, als Wirkstoff, zusammen mit einem pharmazeutisch unbedenklichen Träger, wobei die pharmazeutische Zusammensetzung ferner einen zweiten Wirkstoff oder ein beliebiges seiner N-Oxide, Solvate, Tautomere oder Stereoisomere davon sowie der pharmazeutisch unbedenklichen Salze der jeweiligen Vorgenannten, einschließlich deren Mischungen in allen Verhältnissen, enthält, wobei der zweite Wirkstoff von einem Amidderivat der Formel I-a, I-b oder I-c wie in irgendeinem der Ansprüche 1 bis 10 definiert verschieden ist.

14. Set( Kit ) umfassend getrennte Packungen von
a) einer wirksamen Menge eines Amidderivats der Formel I-a, I-b oder I-c nach irgendeinem der Ansprüche 1 bis 10, oder ein beliebiges N-Oxid, Solvat, Tautomer oder Stereoisomer davon und/oder ein beliebiges pharmazeutisch unbedenkliches Salz der jeweiligen Vorgenannten, einschließlich deren Mischungen in allen Verhältnissen; und
b) einer wirksamen Menge eines weiteren Wirkstoffs, wobei es sich bei dem weiteren Wirkstoff nicht um ein Amidderivat der Formel I-a, I-b oder I-c wie in irgendeinem der Ansprüche 1 bis 10 definiert handelt.

15. Verfahren zur Herstellung eines Amidderivats der Formel I-a, I-b oder I-c nach beliebigen der Ansprüche 1 bis 10, oder ein beliebiges N-Oxid, Solvat, Tautomer oder Stereoisomer davon und/oder ein beliebiges pharmazeutisch unbedenkliches Salz der jeweiligen Vorgenannten, wo das Verfahren **dadurch gekennzeichnet ist, dass** man
(a) eine Carbonsäure der allgemeinen Formel II-a 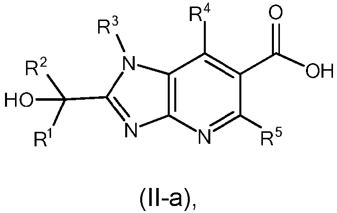
wobei R¹, R², R³, R⁴ und R⁵ wie in beliebigen der Ansprüche 1 bis 10 definiert sind,
einer Amidierungsreaktion mit einer Verbindung der Formel III unterwirft:
R⁶-NH₂ (III)
wobei R⁶ wie in beliebigen der Ansprüche 1 bis 10 definiert ist, gegebenenfalls in Anwesenheit eines geeigneten Katalysators, was das Amidderivat der Formel I-a ergibt: 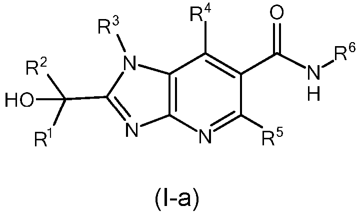
wobei R¹, R², R³, R⁴, R⁵ und R⁶ wie in beliebigen der Ansprüche 1 bis 10 definiert sind;
oder
(b) eine Carbonsäure der allgemeinen Formel II-b 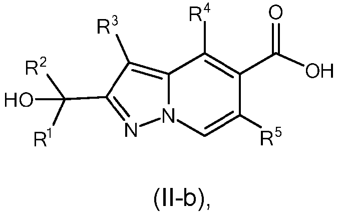
wobei R¹, R², R³, R⁴ und R⁵ wie in beliebigen der Ansprüche 1 bis 10 definiert sind,
einer Amidierungsreaktion mit einer Verbindung der Formel III unterwirft:
R⁶-NH₂ (III)
wobei R⁶ wie in beliebigen der Ansprüche 1 bis 10 definiert ist, gegebenenfalls in Anwesenheit eines geeigneten Katalysators, was das Amidderivat der Formel I-b ergibt: 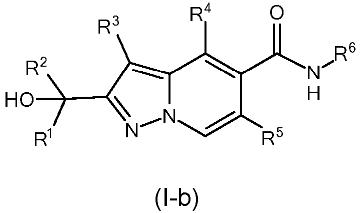
wobei R¹, R², R³, R⁴, R⁵ und R⁶ wie in beliebigen der Ansprüche 1 bis 10 definiert sind;
oder
(c) eine Carbonsäure der allgemeinen Formel II-c 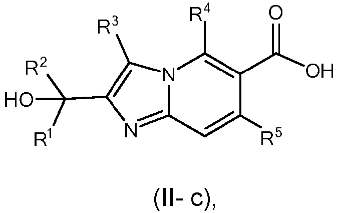
wobei R¹, R², R³, R⁴ und R⁵ wie in beliebigen der Ansprüche 1 bis 10 definiert sind,
einer Amidierungsreaktion mit einer Verbindung der Formel III unterwirft:
R⁶-NH₂ (III)
wobei R⁶ wie in beliebigen der Ansprüche 1 bis 10 definiert ist, gegebenenfalls in Anwesenheit eines geeigneten Katalysators, was das Amidderivat der Formel I-c ergibt: 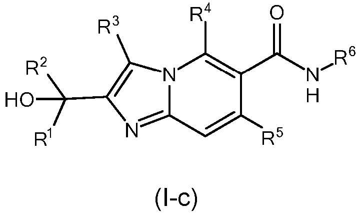
wobei R¹, R², R³, R⁴, R⁵ und R⁶ wie in beliebigen der Ansprüche 1 bis 10 definiert sind.

16. Carbonsäure der Formel II-a, II-b oder II-c 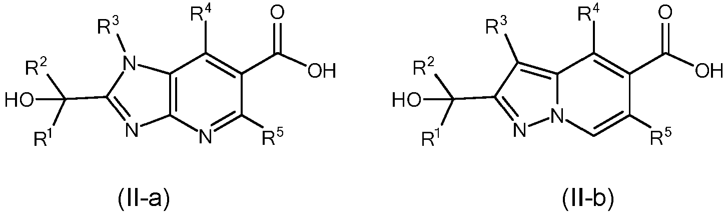 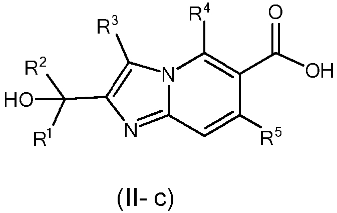 wobei R¹, R², R³, R⁴, R⁵ und R⁶ wie in beliebigen der Ansprüche 1 bis 10 definiert sind.

## Revendications

1. Dérivé d'amide de formule I-a, I-b ou I-c 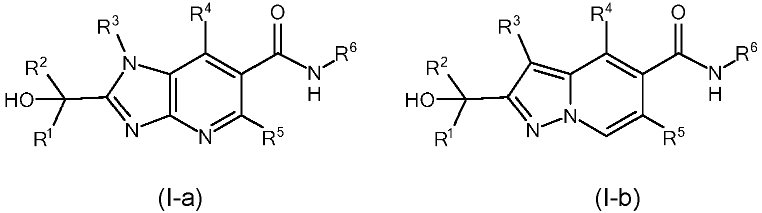 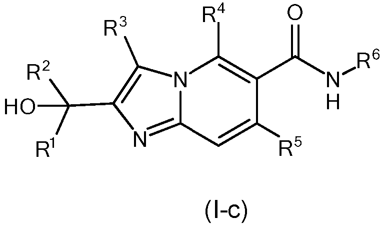
dans laquelle, indépendamment les uns des autres,
R¹ désigne Ar^{A} ou Hetar^{A} ;
R² désigne Ar^{B} ou Hetar^{B} ;
R³ désigne C₁₋₆-aliphatique ou -O-C₁₋₆-aliphatique ;
R⁴ désigne H, D, C₁₋₆-aliphatique ou -O-C₁₋₆-aliphatique ;
R⁵ désigne H, D, C₁₋₆-aliphatique, -O-C₁₋₆-aliphatique ou halogène ;
R⁶ désigne Hetar^{C} ou -CH₂-Hetar^{C} ;
Ar^{A} est un mono- ou biaryle ayant 5, 6, 7, 8, 9, 10, 11 atomes de carbone de cycle, où cet aryle peut être non substitué ou substitué par des substituants R^{A1}, R^{A2}, R^{A3}, R^{A4} et/ou R^{A5} qui peuvent être identiques ou différents ;
Ar^{B} est un mono- ou biaryle ayant 5, 6, 7, 8, 9, 10, 11 atomes de carbone de cycle, où cet aryle peut être non substitué ou substitué par des substituants R^{B1}, R^{B2}, R^{B3}, R^{B4} et/ou R^{B5} qui peuvent être identiques ou différents ;
Hetar^{A} est un hétéroaryle mono- ou bicyclique ayant 5, 6, 7, 8, 9, 10, 11 atomes de cycle où 1, 2, 3, 4, 5 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{A1}, R^{A2}, R^{A3}, R^{A4} et/ou R^{A5} qui peuvent être identiques ou différents ;
Hetar^{B} est un hétéroaryle mono- ou bicyclique ayant 5, 6, 7, 8, 9, 10, 11 atomes de cycle où 1, 2, 3, 4, 5 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{B1}, R^{B2}, R^{B3}, R^{B4} et/ou R^{B5} qui peuvent être identiques ou différents ;
Hetar^{C} est un hétéroaryle mono- ou bicyclique ayant 5, 6, 7, 8, 9, 10, 11 atomes de cycle où 1, 2, 3, 4, 5 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{C1}, R^{C2}, R^{C3}, R^{C4} et/ou R^{C5} qui peuvent être identiques ou différents ;
R^{A1}, R^{A2}, R^{A3}, R^{A4}, R^{A5}, R^{B1}, R^{B2}, R^{B3}, R^{B4}, R^{B5}, R^{C1}, R^{C2}, R^{C3}, R^{C4}, R^{C5} sont indépendamment les uns des autres H, D, halogène, C₁₋₆-aliphatique, - O-C₁₋₆-aliphatique ;
halogène désigne F, Cl, Br ou I ;
ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions.

2. Dérivé d'amide selon la revendication 1, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
R¹ et R² revêtent la même signification.

3. Dérivé d'amide selon la revendication 1, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
R¹ et R² revêtent une signification différente.

4. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
Ar^{A} est phényle qui peut être non substitué ou mono- ou disubstitué par R^{A1} et/ou R^{A2} où R^{A1} et/ou R^{A2} sont tels que définis selon l'une quelconque des revendications précédentes ; ou trideutérophényle, tétradeutéro-phényle ou pentadeutérophényle ;
Ar^{B} est phényle qui peut être non substitué ou mono- ou disubstitué par R^{B1} et/ou R^{B2} où R^{B1} et/ou R^{B2} sont tels que définis selon l'une quelconque des revendications précédentes ; ou trideutérophényle, tétradeutéro-phényle ou pentadeutérophényle ;
Hetar^{A} est un hétéroaryle monocyclique ayant 5 ou 6 atomes de cycle ou un hétéroaryle bicyclique ayant 10 atomes de cycle où 1 ou 2 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{A1} et/ou R^{A2} qui peuvent être identiques ou différents ;
Hetar^{B} est un hétéroaryle monocyclique ayant 5 ou 6 atomes de cycle où 1 ou 2 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{B1} et/ou R^{B2} qui peuvent être identiques ou différents.

5. Dérivé d'amide selon la revendication 4, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
R^{A1}, R^{A2}, R^{B1}, R^{B2} sont indépendamment les uns des autres H, D, F, Cl, C₁₋₄-aliphatique, -O-C₁₋₄-aliphatique.

6. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel, indépendamment les uns des autres,
R³ désigne C₁₋₄-alkyle substitué ou non substitué ;
R⁴ désigne H ;
R⁵ désigne H, C₁₋₄-alkyle, -O-C₁₋₄-alkyle, F ou Cl.

7. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
R⁶ désigne -CH₂-Hetar^{C} ou Hetar^{C} ;
Hetar^{C} est un hétéroaryle monocyclique ayant 5 ou 6 atomes de cycle où 1, 2 ou 3 parmi lesdits atomes de cycle est/sont un ou plusieurs hétéroatomes choisis parmi N, O et/ou S et le reste est constitué d'atomes de carbone, où cet hétéroaryle peut être non substitué ou substitué par des substituants R^{C1} et/ou R^{C2} qui peuvent être identiques ou différents ;
R^{C1}, R^{C2} désignent H ou C₁₋₄-alkyle substitué ou non substitué où au moins l'un parmi R^{C1} et R^{C2} n'est pas H.

8. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel
R⁶ désigne Hetar^{C} ;
Hetar^{C} est un hétéroaryle monocyclique ayant 5 atomes de cycle où 2 ou 3 parmi lesdits atomes de cycle est/sont un ou plusieurs hétéroatomes choisis parmi N et/ou O et le reste est constitué d'atomes de carbone, où cet hétéroaryle peut être non substitué ou substitué par un substituant R^{C1} ;
R^{C1} désigne éthyle, 2-aminoéthyle, 2-hydroxyéthyle, 2-méthoxyéthyle.

9. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
dans lequel, indépendamment les uns des autres,
R³ désigne méthyle, éthyle, 2-diméthylaminoéthyle, 2-hydroxyéthyle, 2-méthoxyéthyle ;
R⁴ désigne H ;
R⁵ désigne H, méthyle, éthyle, méthoxy, F ou Cl ;
R⁶ désigne Hetar^{C} ;
Ar^{A} est phényle ; mono-, di-, tri-, tétra- ou pentadeutérophényle, préférablement pentadeutérophényle ; fluorophényle, préférablement 2-fluorophényle ; méthylphényle (tolyle), préférablement 2-méthylphényle ; méthoxyphényle, préférablement 2-méthoxyphényle ; difluorométhoxy, préférablement 4-difluorométhoxy ; 4-difluorométhoxy-2-fluorophényle ;
Ar^{B} est phényle ; mono-, di-, tri-, tétra- ou pentadeutérophényle, préférablement pentadeutérophényle ; fluorophényle, préférablement 2-fluorophényle ; méthylphényle (tolyle), préférablement 2-méthylphényle ; difluorométhoxy, préférablement 4-difluorométhoxy ;
Hetar^{A} est méthylpyrazolyle, préférablement 1-méthylpyrazol-3-yle, 1-méthylpyrazol-4-yle ; thién-2-yle, thién-3-yle ; méthylthiényle, préférablement 5-méthylthién-2-yle ; thiazolyle, préférablement 1,3-thiazol-2-yle ; pyridinyle, préférablement pyridin-2-yle ; pyrimidinyle, préférablement pyrimidin-4-yle ; pyridazinyle, préférablement pyridazin-3-yle ; quino-léinyle, préférablement quinoléin-2-yle ;
Hetar^{B} est thién-2-yle, thién-3-yle ; méthylthiényle, préférablement 5-méthylthién-2-yle ; pyridinyle, préférablement pyridin-2-yle ;
Hetar^{C} est éthylpyrazolyle, préférablement 1-éthylpyrazol-3-yle, 1-éthyl-pyrazol-4-yle ; hydroxyéthylpyrazolyle, préférablement 1-(2-hydroxy-éthyl)pyrazol-4-yle ; méthoxyéthylpyrazolyle, préférablement 1-(2-méthoxyéthyl)pyrazol-4-yle ; éthylimidazolyle, préférablement 1-éthyl-1H-imidazol-4-yle ; éthyloxazolyle, préférablement 4-éthyl-1,3-oxazol-2-yle, 5-éthyl-1,3-oxazol-2-yle ; éthyltriazolyle, préférablement 1-éthyl-1H-1,2,4-triazol-3-yle, 2-éthyl-2H-1,2,3-triazol-4-yle ; aminoéthyltriazolyle, préférablement 1-(2-aminoéthyl)-1H-1,2,4-triazol-3-yle ; hydroxyéthyl-triazolyle, préférablement 1-(2-hydroxyéthyl)-1H-1,2,4-triazol-3-yle ; méthoxyéthyltriazolyle, préférablement 1-(2-méthoxyéthyl)-1H-1,2,4-triazol-3-yle ; éthyloxadiazolyle, préférablement 5-éthyl-1,3,4-oxadiazol-2-yle, 5-éthyl-1,2,4-oxadiazol-3-yle.

10. Dérivé d'amide selon l'une quelconque des revendications précédentes, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions,
le dérivé d'amide étant choisi dans le groupe constitué par les composés illustrés dans le Tableau 1 (qui est divisé en Tableaux 1a, 1b, 1c et 1d).

11. Dérivé d'amide selon l'une quelconque des revendications 1 à 10, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions, pour une utilisation comme médicament.

12. Composé selon l'une quelconque des revendications 1 à 10, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions, pour une utilisation dans la prévention et/ou le traitement d'une condition médicale ou maladie qui est affectée par l'inhibition d'ACSS2, cette condition médicale ou maladie étant choisie dans le groupe constitué par : le cancer, en particulier les tumeurs, y compris les tumeurs solides ; une affection ou une maladie inflammatoire, en particulier la maladie de Crohn, la rectocolite hémorragique, la fibrose pulmonaire idiopathique, la dystrophie musculaire, la polyarthrite rhumatoïde et la sclérodermie systémique (sclérodermie) ; une affection ou une maladie neurodégénérative, en particulier la maladie de Huntington ; des troubles du métabolisme lipidique, par exemple la stéatohépatite non alcoolique (NASH), la maladie du foie gras non alcoolique (NAFLD), la stéatose hépatique ; les infections virales, par exemple par cytomégalovirus ; le syndrome de stress post-traumatique (SSPT) ; le trouble bipolaire, la dépression, le syndrome de Gilles de La Tourette, la schizophrénie, le trouble obsessionnel compulsif, le trouble anxieux, les troubles paniques, les phobies, la dépendance par exemple à l'alcool, au tabac, aux opiacés, aux sédatifs, aux hypnotiques, aux anxiolytiques, à la cocaïne, au cannabis, aux amphétamines, aux hallucinogènes, aux inhalants, à la phencyclidine, les troubles du contrôle des impulsions, les addictions comportementales.

13. Composition pharmaceutique comprenant au moins un dérivé d'amide selon l'une quelconque des revendications 1 à 10, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions, comme ingrédient actif, conjointement avec un véhicule pharmaceutiquement acceptable, cette composition pharmaceutique comprenant en outre un deuxième ingrédient actif ou l'un quelconque parmi les N-oxydes, solvates, tautomères ou stéréo-isomères de celui-ci ainsi que les sels pharmaceutiquement acceptables de chacun parmi ce qui précède, y compris des mélanges de ceux-ci en toutes proportions, où ce deuxième ingrédient actif est autre qu'un dérivé d'amide de formule I-a, I-b ou I-c tel que défini selon l'une quelconque des revendications 1 à 10.

14. Ensemble (kit) comprenant des conditionnements séparés
a) d'une quantité efficace d'un dérivé d'amide de formule I-a, I-b ou I-c selon l'une quelconque des revendications 1 à 10, ou un N-oxyde, solvate, tautomère ou stéréoisomère quelconque de celui-ci et/ou un sel pharmaceutiquement acceptable quelconque de chacun parmi ce qui précède, y compris des mélanges de ceux-ci en toutes proportions ; and
b) d'une quantité efficace d'un autre ingrédient actif, cet autre ingrédient actif n'étant pas un dérivé d'amide de formule I-a, I-b ou I-c tel que défini selon l'une quelconque des revendications 1 à 10.

15. Procédé de préparation d'un dérivé d'amide de formule I-a, I-b ou I-c selon l'une quelconque des revendications 1 à 10, ou d'un N-oxyde, solvate, tautomère ou stéréoisomère quelconque de celui-ci et/ou d'un sel pharmaceutiquement acceptable quelconque de chacun parmi ce qui précède, le procédé étant **caractérisé en ce que**
(a) un acide carboxylique de formule générale II-a 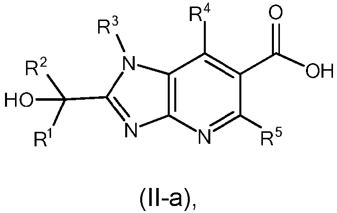
dans laquelle R¹, R², R³, R⁴ et R⁵ sont tels que définis selon l'une quelconque des revendications 1 à 10,
est soumis à une réaction d'amidation avec un composé de formule III :
R⁶-NH₂ (III)
dans laquelle R⁶ est tel que défini selon l'une quelconque des revendications 1 à 10, éventuellement en présence d'un catalyseur convenable, pour conduire au dérivé d'amide de formule I-a : 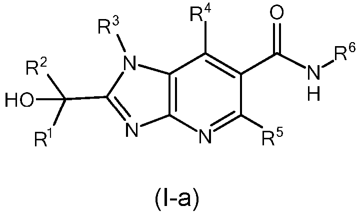
dans laquelle R¹, R², R³, R⁴, R⁵ et R⁶ sont tels que définis selon l'une quelconque des revendications 1 à 10 ;
ou
(b) un acide carboxylique de formule générale II-b 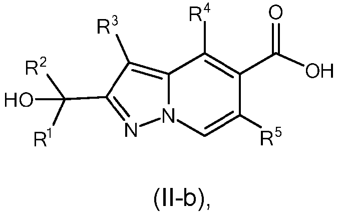
dans laquelle R¹, R², R³, R⁴ et R⁵ sont tels que définis selon l'une quelconque des revendications 1 à 10,
est soumis à une réaction d'amidation avec un composé de formule III :
R⁶-NH₂ (III)
dans laquelle R⁶ est tel que défini selon l'une quelconque des revendications 1 à 10, éventuellement en présence d'un catalyseur convenable, pour conduire au dérivé d'amide de formule I-b : 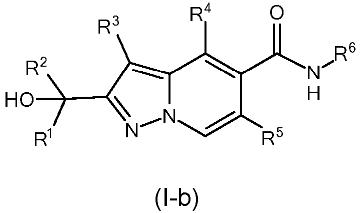
dans laquelle R¹, R², R³, R⁴, R⁵ et R⁶ sont tels que définis selon l'une quelconque des revendications 1 à 10 ;
ou
(c) un acide carboxylique de formule générale II-c 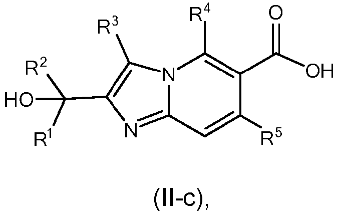
dans laquelle R¹, R², R³, R⁴ et R⁵ sont tels que définis selon l'une quelconque des revendications 1 à 10,
est soumis à une réaction d'amidation avec un composé de formule III :
R⁶-NH₂ (III)
dans laquelle R⁶ est tel que défini selon l'une quelconque des revendications 1 à 10, éventuellement en présence d'un catalyseur convenable, pour conduire au dérivé d'amide de formule I-c : 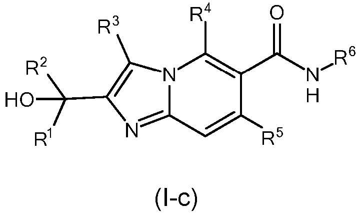
dans laquelle R¹, R², R³, R⁴, R⁵ et R⁶ sont tels que définis selon l'une quelconque des revendications 1 à 10.

16. Acide carboxylique de formule II-a, II-b ou II-c 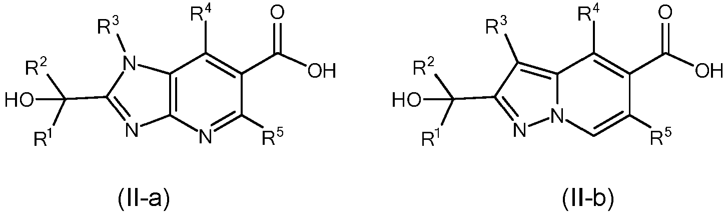 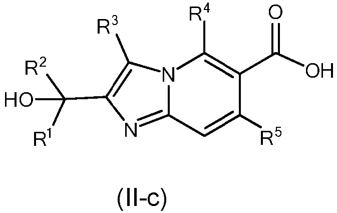 dans laquelle R¹, R², R³, R⁴, R⁵ et R⁶ sont tels que définis selon l'une quelconque des revendications 1 à 10.
